# EUROPEAN PATENT APPLICATION

(11) **EP 4 840 960 A2**
(43) Date of publication of application: **18.11.2026**
(21) Application number: 26201026.7
(22) Date of filing: 03.10.2015
(51) Int. Cl.: C12N 15/113

(54) **TARGETED AUGMENTATION OF NUCLEAR GENE OUTPUT**

(30) Priority: 03.10.2014 US 201462059847 P
(62) Document number(s) of the earlier application(s) in accordance with Art. 76 EPC: 15846242.4 / 3 201 339
(71) Applicant: Cold Spring Harbor Laboratory, Cold Spring Harbor, NY 11724 (US)
(72) Inventor: KRAINER, Adrian, Huntington Station, 11746 (US); AZNAREZ, Isabel, Cambridge, 02139 (US)
(74) Representative: EIP

(57) **Abstract**

Provided herein are methods and compositions for increasing production of a target protein or functional RNA by a cell.

## Description

### CROSS-REFERENCE

This application claims the benefit of U.S. Provisional Application No. 62/059,847, filed October 3, 2014, which application is incorporated herein by reference.

### BACKGROUND

Some genetic diseases are caused by haploinsufficiency, in which there is only one functional copy of a gene and that single copy does not produce enough of the gene product. For example, this can be caused by hemizygous deletions, in which one copy of the gene is lost. Other genetic diseases are caused by mutations which alter the gene product, so that it possesses only partial function.

### SUMMARY

As described herein, antisense oligomers (ASOs) can be used to increase production of proteins, or functional RNAs in the case of non-protein coding genes, by promoting constitutive splicing (employing the wild-type sequence) at an intron splice site of an intron-containing gene to increase expression of the gene product. The ASOs described for use in these methods promote constitutive splicing and do not correct aberrant splicing resulting from mutation, or promote constitutive splicing and do not modulate alternative splicing. The methods described herein may therefore be used to treat a condition resulting from reduced expression or insufficient activity of a gene product.

Described here are methods of increasing expression in cells of a target protein encoded by a pre-mRNA that comprises at least one retained intron (an RIC pre-mRNA); a retained intron is one that remains present when one or more of the other introns have been spliced out (removed). Expression of the target protein depends on complete splicing (removal) of all introns in the pre-mRNA in the nucleus to generate mature mRNA that is subsequently exported to the cytoplasm and translated into the target protein. Inefficient splicing (removal) of an intron results in a retained intron-containing (RIC) pre-mRNA that accumulates primarily in the nucleus, and if exported to the cytoplasm is degraded, such that RIC pre-mRNA is not translated into the target protein. Treatment with an antisense oligomer (ASO) described by the method herein can promote the splicing of a retained intron from pre-mRNA transcripts (pre-mRNA species comprising one or more introns) and result in an increase in mRNA, which is translated to provide higher levels of target protein.

In embodiments, the method is a method of increasing expression of a target protein or functional RNA by cells having a retained-intron-containing pre-mRNA (RIC pre-mRNA), the RIC pre-mRNA comprising a retained intron, an exon flanking the 5' splice site of the retained intron, an exon flanking the 3' splice site of the retained intron, and wherein the RIC pre-mRNA encodes the target protein or functional RNA. In embodiments, the method comprises contacting the cells with an ASO complementary to a targeted portion of the RIC pre-mRNA encoding the target protein or functional RNA, whereby the retained intron is constitutively spliced from the RIC pre-mRNA encoding the target protein or functional RNA, thereby increasing the level of mRNA encoding the target protein or functional RNA, and increasing the expression of target protein or functional RNA in the cells. In embodiments, the cells are in or are from a subject, and the method is a method of treating the subject to increase expression of the target protein or functional RNA in the subject's cells. In embodiments, the cells are in or are from a subject having a condition caused by a deficient amount or activity of the target protein or a deficient amount or activity of the functional RNA. In embodiments, the target protein or the functional RNA is a compensating protein or a compensating functional RNA that functionally augments or replaces a target protein or functional RNA that is deficient in amount or activity in the subject.

In embodiments, the condition caused by a deficient amount or activity of the target protein or a deficient amount or activity of the functional RNA is not a condition caused by alternative or aberrant splicing of the retained intron to which the ASO is targeted. In embodiments, the condition caused by a deficient amount or activity of the target protein or a deficient amount or activity of the functional RNA is not a condition caused by alternative or aberrant splicing of any retained intron in a RIC pre-mRNA encoding the target protein or functional RNA.

In embodiments, the deficient amount of the target protein is caused by haploinsufficiency of the target protein, wherein the subject has a first allele encoding a functional target protein, and a second allele from which the target protein is not produced, or a second allele encoding a nonfunctional target protein, and wherein the antisense oligomer binds to a targeted portion of a RIC pre-mRNA transcribed from the first allele.

In other embodiments, the subject has a condition caused by an autosomal recessive disorder resulting from a deficiency in the amount or function of the target protein, wherein the subject has a) a first mutant allele from which i) the target protein is produced at a reduced level compared to production from a wild-type allele, ii) the target protein is produced in a form having reduced function compared to an equivalent wild-type protein, or iii) the target protein is not produced, and b) a second mutant allele from which i) the target protein is produced at a reduced level compared to production from a wild-type allele, ii) the target protein is produced in a form having reduced function compared to an equivalent wild-type protein, or iii) the target protein is not produced, and wherein the RIC pre-mRNA is transcribed from the first allele and/or the second allele. In embodiments, the target protein is produced both at a reduced level and in a form having reduced function compared to an equivalent wild-type protein.

In embodiments, the target protein is produced in a form having reduced function compared to the equivalent wild-type protein. In other embodiments, the target protein is produced in a form that is fully-functional compared to the equivalent wild-type protein.

In embodiments, the deficient amount of the functional RNA is caused by haploinsufficiency of the functional RNA, wherein the subject has a first allele encoding a functional RNA that is functional, and a second allele from which the functional RNA is not produced, or a second allele encoding a functional RNA that is nonfunctional, and wherein the antisense oligomer binds to a targeted portion of a RIC pre-mRNA transcribed from the first allele.

In other embodiments, the subject has a condition caused by an autosomal recessive disorder resulting from a deficiency in the amount or function of the functional RNA, wherein the subject has a) a first mutant allele from which i) the functional RNA is produced at a reduced level compared to production from a wild-type allele, ii) the functional RNA is produced in a form having reduced function compared to an equivalent wild-type protein, or iii) the functional RNA is not produced, and b) a second mutant allele from which i) the functional RNA is produced at a reduced level compared to production from a wild-type allele, ii) the functional RNA is produced in a form having reduced function compared to an equivalent wild-type protein, or iii) the functional RNA is not produced, and wherein the RIC pre-mRNA is transcribed from the first allele and/or the second allele. In embodiments, the functional RNA is produced both at a reduced level and in a form having reduced function compared to an equivalent wild-type functional RNA.

In embodiments, the functional RNA is produced in a form having reduced function compared to the equivalent wild-type protein. In other embodiments, the functional RNA is produced in a form that is fully-functional compared to the equivalent wild-type protein.

In embodiments, the targeted portion of the RIC pre-mRNA is in the retained intron within the region +6 relative to the 5' splice site of the retained intron to the region -16 relative to the 3' splice site of the retained intron. In embodiments, the targeted portion of the RIC pre-mRNA is in the retained intron within the region +6 to +100 relative to the 5' splice site of the retained intron; or the region -16 to -100 relative to the 3' splice site of the retained intron. In embodiments, the targeted portion of the RIC pre-mRNA is within the region +2e to -4e in the exon flanking the 5' splice site of the retained intron; or the region +2e to -4e in the exon flanking the 3' splice site of the retained intron.

In embodiments, the antisense oligomer does not increase the amount of the target protein or the functional RNA by modulating alternative splicing of pre-mRNA transcribed from a gene encoding the functional RNA or target protein. In embodiments, the antisense oligomer does not increase the amount of the target protein or the functional RNA by modulating aberrant splicing resulting from mutation of the gene encoding the target protein or the functional RNA.

In embodiments, the RIC pre-mRNA was produced by partial splicing of a full-length pre-mRNA or partial splicing of a wild-type pre-mRNA. In embodiments, the mRNA encoding the target protein or functional RNA is a full-length mature mRNA, or a wild-type mature mRNA. In embodiments, the target protein produced is full-length protein, or wild-type protein. In embodiments, the functional RNA produced is full-length functional RNA, or wild-type functional RNA.

In embodiments, the total amount of the mRNA, or the total amount of mature mRNA, encoding the target protein or functional RNA produced in the cell contacted with the antisense oligomer is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to the total amount of the mRNA, or the total amount of mature mRNA, encoding the target protein or functional RNA produced in a control cell.

In embodiments, the total amount of the mRNA encoding the target protein or functional RNA produced in the cell contacted with the antisense oligomer is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to the total amount of the mRNA encoding the target protein or functional RNA produced in a control cell.

In embodiments, the total amount of mature mRNA encoding the target protein or functional RNA produced in the cell contacted with the antisense oligomer is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to the total amount of the mature mRNA, encoding the target protein or functional RNA produced in a control cell.

In embodiments, the total amount of the target protein or functional RNA produced by the cell contacted with the antisense oligomer is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to the amount of the target protein or functional RNA produced by a control cell.

In embodiments, the methods comprise contacting the cells having the RIC pre-mRNA with an antisense oligomer comprising a backbone modification comprising a phosphorothioate linkage or a phosphorodiamidate linkage. In embodiments, the antisense oligomer comprises a phosphorodiamidate morpholino (PMO), a locked nucleic acid (LNA), a peptide nucleic acid (PNA), a 2'-O-methyl, a 2'-Fluoro, or a 2'-O-methoxyethyl moiety. In embodiments, the antisense oligomer comprises at least one modified sugar moiety. In related embodiments, each sugar moiety is a modified sugar moiety.

In embodiments, the antisense oligomer consists of from 8 to 50 nucleobases. In embodiments, the antisense oligomer consists of from 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, or 12 to 15 nucleobases. In embodiments, the antisense oligomer is at least 80%, at least 85%, at least 90%, at least 95%, at least 98%, at least 99%, or 100%, complementary to the targeted portion of the RIC pre-mRNA encoding the protein.

In any of the preceding methods, the cell can comprise a population of RIC pre-mRNAs transcribed from the gene encoding the target protein or functional RNA, wherein the population of RIC pre-mRNAs comprises two or more retained introns, and wherein the antisense oligomer binds to the most abundant retained intron in the population of RIC pre-mRNAs. In these embodiments, the binding of the antisense oligomer to the most abundant retained intron can induce splicing out of the two or more retained introns from the population of RIC pre-mRNAs to produce mRNA encoding the target protein or functional RNA.

In other embodiments, the cell comprises a population of RIC pre-mRNAs transcribed from the gene encoding the target protein or functional RNA, wherein the population of RIC pre-mRNAs comprises two or more retained introns, and wherein the antisense oligomer binds to the second most abundant retained intron in the population of RIC pre-mRNAs. In these embodiments, the binding of the antisense oligomer to the second most abundant retained intron can induce splicing out of the two or more retained introns from the population of RIC pre-mRNAs to produce mRNA encoding the target protein or functional RNA.

In the preceding methods, the condition can be a disease or disorder. In these embodiments, the disease or disorder can be selected from: thrombotic thrombocytopenic purpura, tuberous sclerosis complex, polycystic kidney disease, familial dysautonomia, retinitis pigmentosa type 10, retinitis pigmentosa type 11, cystic fibrosis, retinoblastoma, familial adenomatous polyposis, protein S deficiency, beta thalassemia, and sickle cell disease. In related embodiments, the target protein and the RIC pre-mRNA are encoded by a gene selected from: *ADAMTS13, TSC1, PKD1, IKBKAP, IMPDH1, PRPF31, CFTR, RB1, APC, PROS1, NEDD4L, HBG1, HBG2, and HBB.* In embodiments, the antisense oligomer can bind to a portion of an RIC pre-mRNA selected from SEQ ID NOS: 1-102.

In embodiments, any of the preceding methods further comprises assessing protein expression.

In some embodiments, the subject is a human. In other embodiments, the subject is a non-human animal. In embodiments, the antisense oligomer is administered by intravitreal injection, intrathecal injection, intraperitoneal injection, subcutaneous injection, or intravenous injection of the subject. In embodiments, the cells are *ex vivo.*

In embodiments, the 9 nucleotides at -3e to -1e of the exon flanking the 5' splice site and +1 to +6 of the retained intron, are identical to the corresponding wild-type sequence. In embodiments, the 16 nucleotides at -15 to -1 of the retained intron and +1e of the exon flanking the 3' splice site are identical to the corresponding wild-type sequence.

Described herein are compositions comprising an antisense oligomer for use in a method as described herein. Also described is a pharmaceutical composition comprising the antisense oligomer, and an excipient. In embodiments, the composition comprising the antisense oligomer is intended for use in a method of increasing expression of a target protein or a functional RNA by cells to treat a condition in a subject associated with a deficient protein or deficient functional RNA, wherein the deficient protein or deficient functional RNA is deficient in amount or activity in the subject, wherein the antisense oligomer enhances constitutive splicing of a retained intron-containing pre-mRNA (RIC pre-mRNA) encoding the target protein or the functional RNA, wherein the target protein is: (a) the deficient protein; or (b) a compensating protein which functionally augments or replaces the deficient protein or in the subject; and wherein the functional RNA is: (a) the deficient RNA; or (b) a compensating functional RNA which functionally augments or replaces the deficient functional RNA in the subject; wherein the RIC pre-mRNA comprises a retained intron, an exon flanking the 5' splice site and an exon flanking the 3' splice site, and wherein the retained intron is spliced from the RIC pre-mRNA encoding the target protein or the functional RNA, thereby increasing production or activity of the target protein or the functional RNA in the subject.

In embodiments, the composition comprising the antisense oligomer is intended for use in a method of treating a disease or disorder associated with a target protein or functional RNA in a subject, the method comprising the step of increasing expression of the target protein or functional RNA by cells of the subject, wherein the cells have a retained-intron-containing pre-mRNA (RIC pre-mRNA) comprising a retained intron, an exon flanking the 5' splice site of the retained intron, an exon flanking the 3' splice site of the retained intron, and wherein the RIC pre-mRNA encodes the target protein or functional RNA, the method comprising contacting the cells with the antisense oligomer, whereby the retained intron is constitutively spliced from the RIC pre-mRNA transcripts encoding the target protein or functional RNA, thereby increasing the level of mRNA encoding the target protein or functional RNA, and increasing the expression of the target protein or functional RNA, in the cells of the subject.

In embodiments, the composition comprising the antisense oligomer is intended for use in a method of treating a condition in the subject resulting from a deficiency in the amount or activity of the target protein or the functional RNA. In embodiments, the condition is a disease or disorder. In embodiments, the disease or disorder is selected from: thrombotic thrombocytopenic purpura, tuberous sclerosis complex, polycystic kidney disease, familial dysautonomia, retinitis pigmentosa type 10, retinitis pigmentosa type 11, cystic fibrosis, retinoblastoma, familial adenomatous polyposis, protein S deficiency, beta thalassemia, and sickle cell disease. In embodiments, the composition is intended for use in a method wherein the target protein and RIC pre-mRNA are encoded by a gene selected from: *ADAMTS13, TSC1, PKD1, IKBKAP, IMPDH1, PRPF31, CFTR, RB1, APC, PROS1, NEDD4L, HBG1, HBG2,* and *HBB.*

In embodiments, the antisense oligomer of the composition targets a portion of the RIC pre-mRNA that is in the retained intron within the region +6 relative to the 5' splice site of the retained intron to the region -16 relative to the 3' splice site of the retained intron. In embodiments, the antisense oligomer of the composition targets a portion of the RIC pre-mRNA that is in the retained intron within the region +6 to +100 relative to the 5' splice site of the retained intron; or the region -16 to -100 relative to the 3' splice site of the retained intron. In embodiments, the antisense oligomer targets a portion of the RIC pre-mRNA that is within the region about 100 nucleotides downstream of the 5' splice site of the at least one retained intron, to about 100 nucleotides upstream of the 3' splice site of the at least one retained intron. In embodiments, the targeted portion of the RIC pre-mRNA is within: the region +2e to -4e in the exon flanking the 5' splice site of the retained intron; or the region +2e to -4e in the exon flanking the 3' splice site of the retained intron.

In embodiments, the antisense oligomer of the composition or as used in the methods described herein does not increase the amount of target protein or functional RNA by modulating alternative splicing of the pre-mRNA transcribed from a gene encoding the target protein or functional RNA. In embodiments, the antisense oligomer of the composition or as used in the methods described herein does not increase the amount of target protein or functional RNA by modulating aberrant splicing resulting from mutation of the gene encoding the target protein or functional RNA.

In embodiments, the RIC pre-mRNA was produced by partial splicing from a full-length pre-mRNA or a wild-type pre-mRNA. In embodiments, the mRNA encoding the target protein or functional RNA is a full-length mature mRNA, or a wild-type mature mRNA. In embodiments, the target protein produced is full-length protein, or wild-type protein. In embodiments, the functional RNA produced is full-length functional RNA, or wild-type functional RNA.

In embodiments, the retained intron is a rate-limiting intron. In embodiments, the retained intron is the most abundant intron in said RIC pre-mRNA. In embodiments, the retained intron is the second most abundant intron in said RIC pre-mRNA.

In embodiments, the antisense oligomer of the composition or as used in the methods described herein, comprises a backbone modification comprising a phosphorothioate linkage or a phosphorodiamidate linkage. In embodiments, the antisense oligomer is an antisense oligonucleotide.

In embodiments, the antisense oligomer comprises a phosphorodiamidate morpholino, a locked nucleic acid, a peptide nucleic acid, a 2'-O-methyl, a 2'-Fluoro, or a 2'-O-methoxyethyl moiety. In embodiments, the antisense oligomer comprises at least one modified sugar moiety. In related embodiments, each sugar moiety is a modified sugar moiety.

The antisense oligomer can consist of from 8 to 50 nucleobases. In embodiments, antisense oligomer consists of 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, or 12 to 15 nucleobases.

In embodiments, the antisense oligomer is at least 80%, at least 85%, at least 90%, at least 95%, at least 98%, at least 99%, or is 100% complementary to the targeted portion of the RIC pre-mRNA encoding the protein. In embodiments, the antisense oligomer binds to a portion of an RIC pre-mRNA selected from SEQ **ID** NOS: 1-102.

In embodiments, the antisense oligomer is comprised in a pharmaceutical composition comprising an excipient.

Described herein are methods for identifying an antisense oligomer that increases the amount of mRNA encoding a target protein or functional RNA by inducing constitutive splicing of a retained intron from a RIC pre-mRNA encoding the target protein or functional RNA, from among a set of antisense oligomers that each hybridize to a target region of the RIC pre-mRNA, wherein the RIC pre-mRNA comprises at least one retained intron, wherein the antisense oligomers in the set are tiled every 1 to 5 nucleotides, and wherein the antisense oligomers in the set hybridize to the RIC pre-mRNA within the sequence that is: about 100 nucleotides upstream of the 5' splice site of the at least one retained intron, to about 100 nucleotides downstream of the 5' splice site of the at least one retained intron; or about 100 nucleotides upstream of the 3' splice site of the at least one retained intron, to about 100 nucleotides downstream of the 3' splice site of the at least one retained intron; the method comprising: a) delivering a first antisense oligomer in the set to a cell comprising the RIC pre-mRNA; b) measuring the amount of the RIC pre-mRNA and measuring the amount of mRNA encoding the target protein or functional RNA in the cell to which the first antisense oligomer was delivered; c) measuring the amount of the RIC pre-mRNA and measuring the amount of mRNA encoding a target protein or functional RNA in a control cell; and d) comparing the amounts of RIC pre-mRNA and mRNA encoding a target protein or functional RNA measured in b and c; wherein the first antisense oligomer is identified as an antisense oligomer that increases the amount of mRNA encoding the target protein or functional RNA by inducing constitutive splicing of the at least one retained intron from the RIC pre-mRNA based on an observed decrease in the amount of the RIC pre-mRNA and an observed increase in the amount of mRNA encoding the target protein or functional RNA in the cell to which the first antisense oligomer was delivered compared to a control cell; and repeating steps a through d with additional antisense oligomers in the set of antisense oligomers as needed to identify an antisense oligomer that increases the amount of mRNA from a gene in a cell by inducing constitutive splicing of a retained intron from the RIC pre-mRNA.

Also described herein are methods for identifying an antisense oligomer (ASO) for treating a condition, wherein the condition results from insufficient production of a gene product, the method comprising: identifying the presence of at least one RIC pre-mRNA in the nucleus of a cell from a subject having the condition, wherein the RIC pre-mRNA comprises at least one retained intron and is transcribed from a gene encoding the gene product, and wherein the identified RIC pre-mRNA when fully spliced to mature mRNA encodes the gene product in a form that is fully-functional or partially-functional; a) preparing a set of ASOs that each hybridize to a target region of the at least one RIC pre-mRNA, wherein the antisense oligomers in the set are tiled every 1 to 5 nucleotides, and wherein the antisense oligomers in the set hybridize to the at least one RIC pre-mRNA within the sequence that is: about 100 nucleotides upstream of the 5' splice site of the at least one retained intron, to about 100 nucleotides downstream of the 5' splice site of the at least one retained intron; or about 100 nucleotides upstream of the 3' splice site of the at least one retained intron, to about 100 nucleotides downstream of the 3' splice site of the at least one retained intron; b) delivering a first ASO in the set of ASOs to a cell comprising the at least one RIC pre-mRNA; c) measuring the amount of RIC pre-mRNA and measuring the amount of mRNA encoding the gene product in the cell to which the first antisense oligomer was delivered; d) measuring the amount of RIC pre-mRNA and measuring the amount of mRNA encoding the gene product in a control cell; and e) comparing the values obtained in steps c and d; wherein the first antisense oligomer is identified as an antisense oligomer that increases the amount of mRNA encoding the gene product by inducing constitutive splicing of the at least one retained intron from the RIC pre-mRNA based on an observed decrease in the amount of RIC pre-mRNA and an observed increase in the amount of mRNA encoding the gene product in the cell to which the first antisense oligomer was delivered compared to a control cell; and repeating steps a through e with additional antisense oligomers in the set of antisense oligomers as needed to identify an antisense oligomer that increases the amount of a mRNA encoding the gene product from a gene in a cell by inducing constitutive splicing of a retained intron from a RIC pre-mRNA; and further testing such antisense oligomers that increase the amount of a mRNA encoding the gene product in a cell by inducing constitutive splicing of a retained intron from a RIC pre-mRNA for the ability to increase the amount of the gene product produced by a cell.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The accompanying drawings are not intended to be drawn to scale. The figures are illustrative only and are not required for enablement of the disclosure. For purposes of clarity, not every component may be labeled in every drawing. In the drawings:

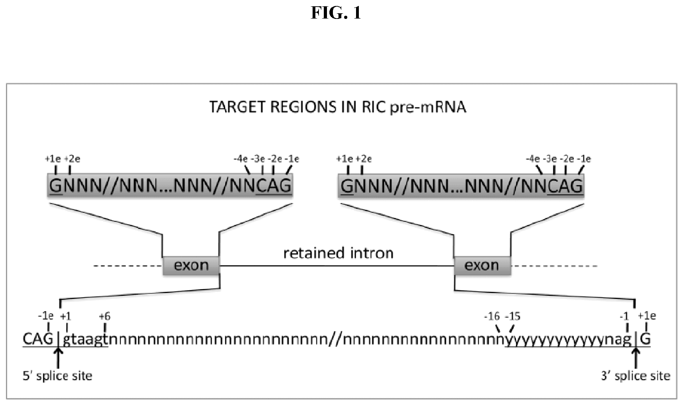

Figure 1 shows a schematic representation of an exemplary retained-intron-containing (RIC) pre-mRNA transcript. The 5' splice site consensus sequence is indicated with underlined letters (letters are nucleotides; upper case: exonic portion and lower case: intronic portion) from -3e to -1e and +1 to +6 (numbers labeled "e" are exonic and unlabeled numbers are intronic). The 3' splice site consensus sequence is indicated with underlined letters (letters are nucleotides; upper case: exonic portion and lower case: intronic portion) from -15 to -1 and +1e (numbers labeled "e" are exonic and unlabeled numbers are intronic). Intronic target regions for ASO screening comprise nucleotides +6 relative to the 5' splice site of the retained intron (arrow at left) to -16 relative to the 3' splice site of the retained intron (arrow at right). In embodiments, intronic target regions for ASO screening comprise nucleotides +6 to +100 relative to the 5' splice site of the retained intron and -16 to -100 relative to the 3' splice site of the retained intron. Exonic target regions comprise nucleotides +2e to -4e in the exon flanking the 5' splice site of the retained intron and +2e to -4e in the exon flanking the 3' splice site of the retained intron. "n" or "N" denote any nucleotide, "y" denotes pyrimidine. The sequences shown represent consensus sequences for mammalian splice sites and individual introns and exons need not match the consensus sequences at every position.

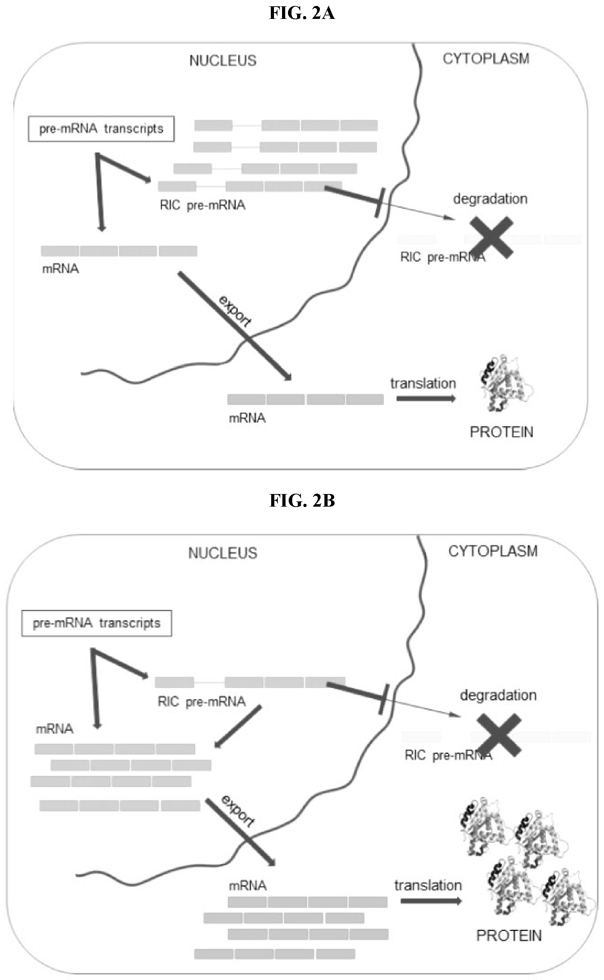

Figure 2A-2B show schematic representations of the Targeted Augmentation of Nuclear Gene Output (TANGO) approach. Figure 2A shows a cell divided into nuclear and cytoplasmic compartments. In the nucleus, a pre-mRNA transcript of a target gene consisting of exons (rectangles) and introns (connecting lines) undergoes splicing to generate an mRNA, and this mRNA is exported to the cytoplasm and translated into target protein. For this target gene, the splicing of intron 1 is inefficient and a retained intron-containing (RIC) pre-mRNA accumulates primarily in the nucleus, and if exported to the cytoplasm, is degraded, leading to no target protein production. Figure 2B shows an example of the same cell divided into nuclear and cytoplasmic compartments. Treatment with an antisense oligomer (ASO) promotes the splicing of intron 1 and results in an increase in mRNA, which is in turn translated into higher levels of target protein.

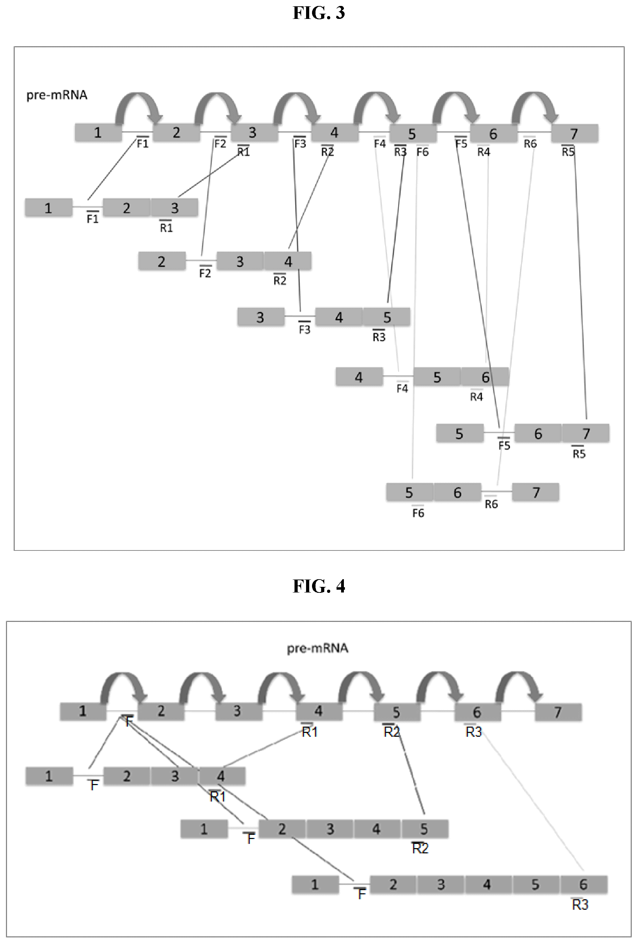

Figure 3 shows a schematic representation of an example of screening for intron retention using RT-PCR, as described in Example 1, of a 7-exon/6-intron gene. Numbered rectangles denote exons connected by lines denoting introns. Arched arrows indicate splicing events. Short horizontal bars denote primer pairs used to assess intron retention. Forward primer are indicated with "F" and reverse primers are indicated with "R," i.e., pairs F1 and R1, F2 and R2, F3 and R3, F4 and R4, F5 and R5, and F6 and R6. An intron is identified as a retained intron when such an intron is present and an adjacent intron is observed to be spliced out (removed).
Figure 4 shows a schematic representation of an example of screening to confirm intron retention using RT-PCR, as described in Example 2, of a 7-exon/6-intron gene. Numbered rectangles denote exons connected by lines denoting introns. Arched arrows indicate splicing events. Short horizontal bars denote primer pairs used to assess intron retention. The forward primer is labeled with an "F" and reverse primers are labeled with "R1," "R2," or "R3." Introns are confirmed as retained introns when such intron is present and one or more adjacent introns is observed to be spliced out (removed).

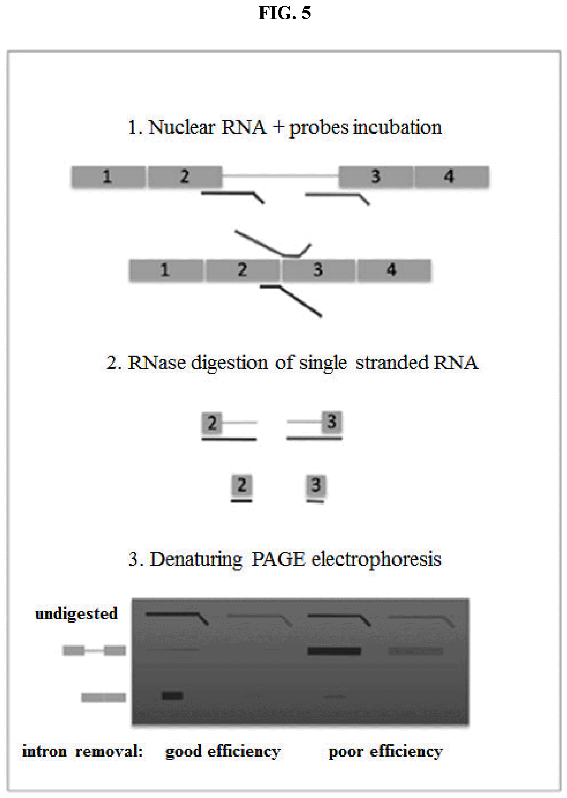

Figure 5 shows a schematic representation of an exemplary RNase protection assay (RPA) to determine intron-removal efficiency.

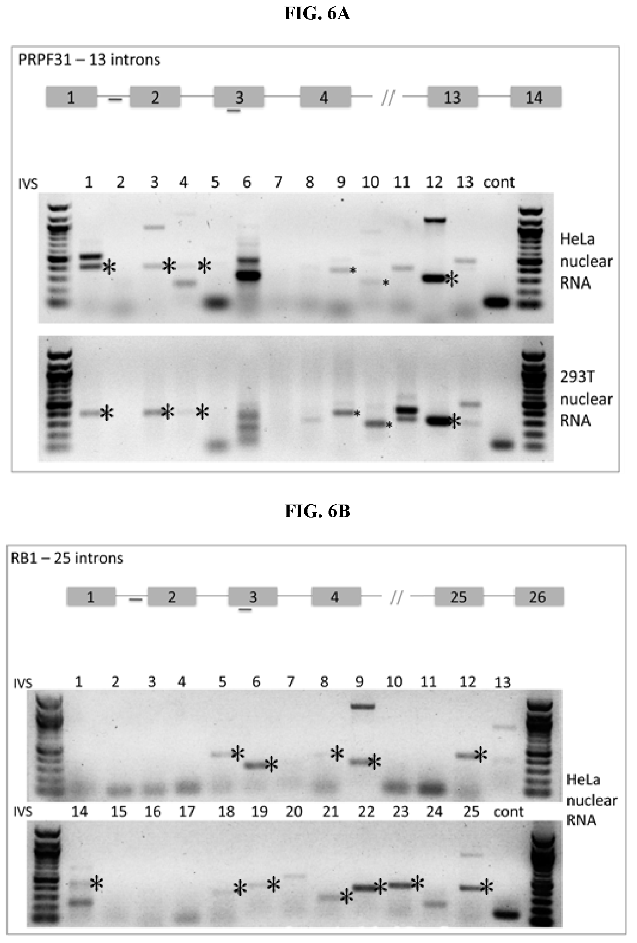

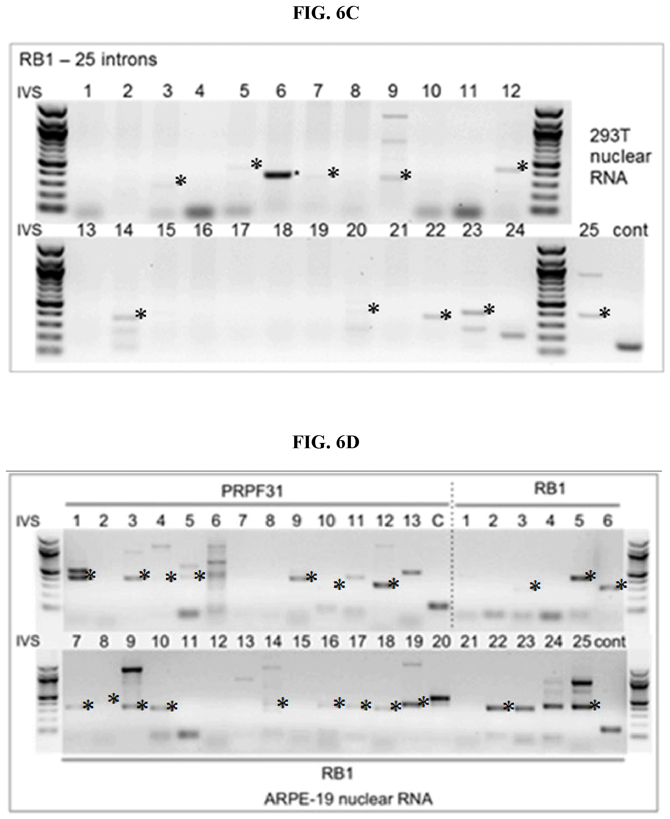

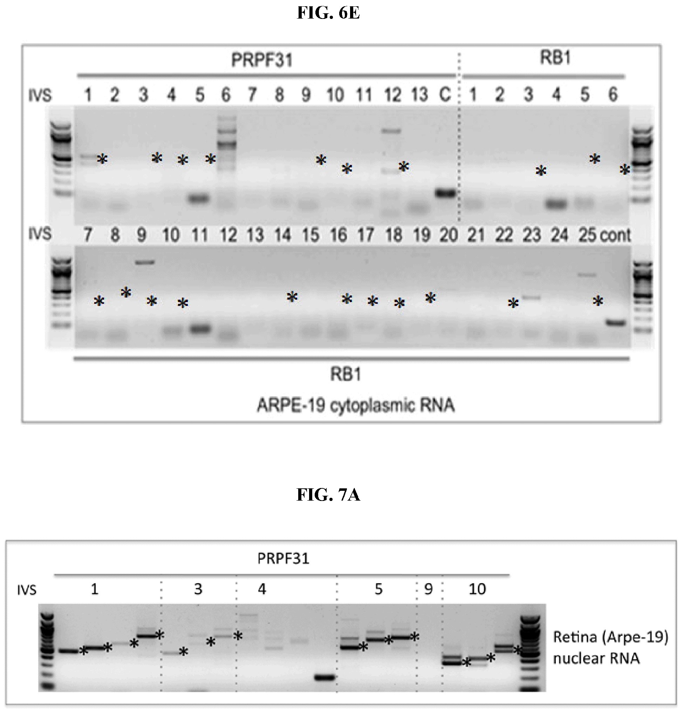

Figures 6A-6E show the identification of intron-retention events in the PRPF31 and RB1 genes, as described in Example 1. Figure 6A shows a schematic representation of the PRPF31 gene with numbered rectangles denoting exons and intervening lines denoting introns. Forward ("F") and reverse ("R") primers are indicated by short lines. Below are representative gels showing RT-PCR products corresponding to intron-retention events in PRPF31. The products were separated in a 1.5% ethidium-bromide-stained agarose gel. The top gel corresponds to products from nuclear fraction of HeLa cells, and the bottom gel corresponds to products from nuclear fractions from 293T cells. Asterisks indicate correct products (by size) for intron-retention events. Figure 6B shows a schematic representation of the RB1 gene with numbered rectangles denoting exons and intervening lines denoting introns. Below are representative gels showing RT-PCR products from HeLa nuclear extracts corresponding to intron-retention events in RB1. The RT-PCR products were separated in a 1.5% ethidium-bromide-stained agarose gel. Figure 6C shows representative gels of RT-PCR products from 293T cell nuclear extracts corresponding to intron-retention events in RB1. Figure 6D shows representative gels of RT-PCR products from ARPE-19 cell nuclear extracts corresponding to intron-retention events in PRPF31 and RB1. RT-PCR products were separated in a 1.5% ethidium-bromide-stained agarose gel. Figure 6E shows representative gels of RT-PCR products from ARPE-19 cell cytoplasmic extracts corresponding to intron-retention events in PRPF31 and RB1. IVS: intervening sequence (intron).

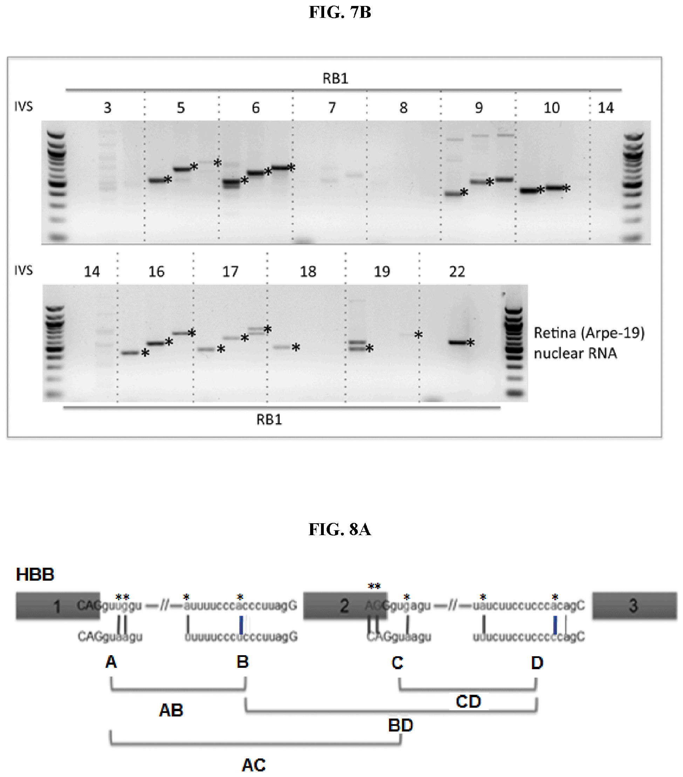

Figures 7A-7B show the identification of intron-retention events in the PRPF31 and RB1 genes, as described in Example 2. Figure 7A shows representative gels of RT-PCR products corresponding to intron-retention events in PRPF31. The RT-PCR products from Arpe-19 cell nuclear extracts were separated in a 1.5% ethidium-bromide-stained agarose gel. Figure 7B shows representative gels of RT-PCR products corresponding to intron-retention events in RB1. The RT-PCR products from Arpe-19 cell nuclear extracts were separated in a 1.5% ethidium-bromide-stained agarose gel. Asterisks indicate correct products (by size) for intron-retention events using the indicated primer pairs. IVS: intervening sequence (intron).

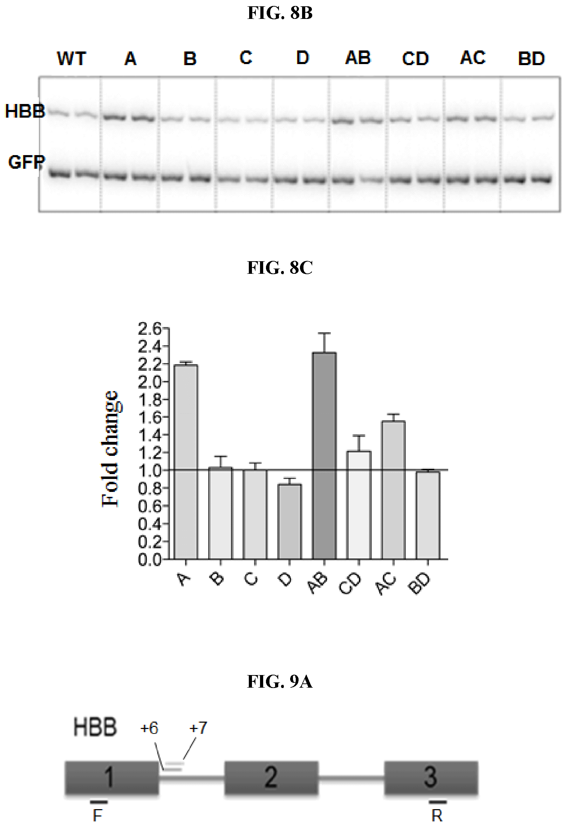

Figures 8A-8C show increased gene expression by promoting splicing efficiency via mutagenesis of splice sites, as described in Example 3. Figure 8A shows a schematic representation of the HBB reporter gene including numbered rectangles denoting exons. Actual HBB splice site sequences are drawn marking the intron-exon boundaries. The nucleotides within the splice site sequences that are indicated with asterisks show the locations of nucleotide substitutions introduced by site directed mutagenesis to bring the splice site sequences to the consensus sequence (sequences directly below the HBB splice sites). A: IVS1 5' splice site mutant, B: IVS1 3' splice site mutant, C: IVS2 5' splice site mutant, D: IVS2 3' splice site mutant. AB, CD, AC and BD: combination mutants. Figure 8B shows a representative gel of radioactive RT-PCR products of wild-type (WT) and mutant HBB reporters. The RT-PCR products were separated in a 5% polyacrylamide gel. Figure 8C shows a bar graph of the intensities of bands corresponding to HBB transcripts normalized to GFP. Fold change was plotted relative to the WT HBB product. The black line indicates a ratio of 1, no change.

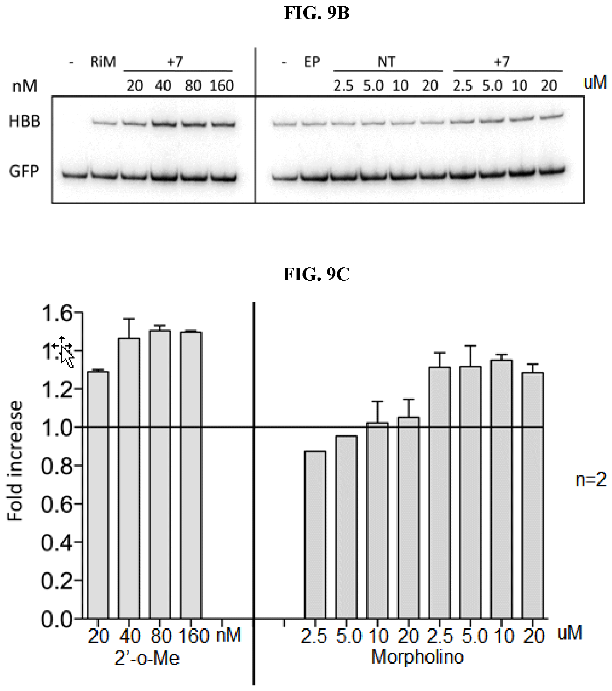

Figures 9A-9C show that ASOs targeting sequences immediately downstream of HBB IVS1 5' splice site increase HBB mRNA, as described in Example 3. Figure 9A shows a schematic representation of the HBB reporter gene. The numbered rectangles denote exons, and intervening lines denote introns. Orange line indicates the IVS1+6 ASO ("+6"), grey line indicates IVS1+7 ASO ("+7"). Black lines indicate forward ("F") and reverse ("R") primers used in PCR amplification of the HBB transcript. Figure 9B presents a representative gel of radioactive RT-PCR products of wild-type HBB reporters untreated (-), mock-treated (RiM, RNAiMAX or EP, EndoPorter) or treated with non-targeting (NT), or IVS1+7 2'-O-Me (left portion of the gel) or PMO (right portion of the gel) ASOs at the indicated concentrations. The RT-PCR products were separated in a 5% polyacrylamide gel. Figure 9C shows a bar graph of the intensities of bands corresponding to HBB transcripts normalized to GFP. Fold change was plotted relative to the product from mock-treated cells. Green bars correspond to treatment with the IVS+7 2'-O-Me ASO and orange bars correspond to treatment with the IVS+7 PMO ASO. The black line indicates a ratio of 1, no change.

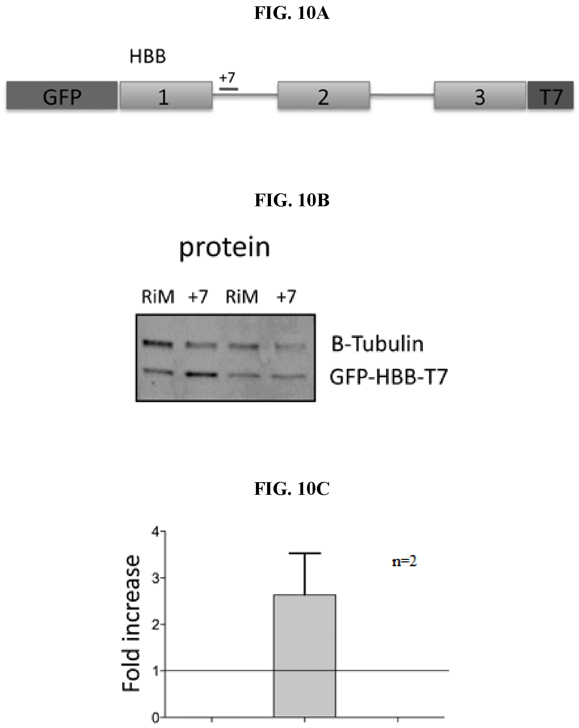

Figures 10A-10C show that IVS1+7 2'-O-Me ASO targeting sequences immediately downstream of the HBB IVS1 5' splice site increase GFP-HBB-T7 protein levels, as described in Example 4. Figure 10A shows a schematic representation of the GFP-HBB-T7 reporter gene that has been integrated in the genome of U2OS cells. The rectangle labeled "GFP" denotes the open reading frame of GFP, numbered rectangles denote HBB exons, intervening lines denote introns and the rectangle labeled "T7" denotes the sequence coding for the T7 tag. The line labeled "+7" indicates the IVS1+7 ASO. Figure 10B presents a representative gel of protein products of wild-type GFP-HBB-T7 reporters mock-treated (RiM, RNAiMAX) or treated with IVS1+7 2'-O-Me ASO at a concentration of 50 nM. The protein products were separated on a 4-20% SDS-polyacrylamide gel. Antibodies against GFP and Beta tubulin were used to detect the protein products. Figure 10C shows a bar graph of the intensity of bands corresponding to GFP-HBB-T7 protein normalized to Beta tubulin from two biological replicates. Fold change was plotted relative to the product from mock-treated cells. The black line indicates a ratio of 1, no change.

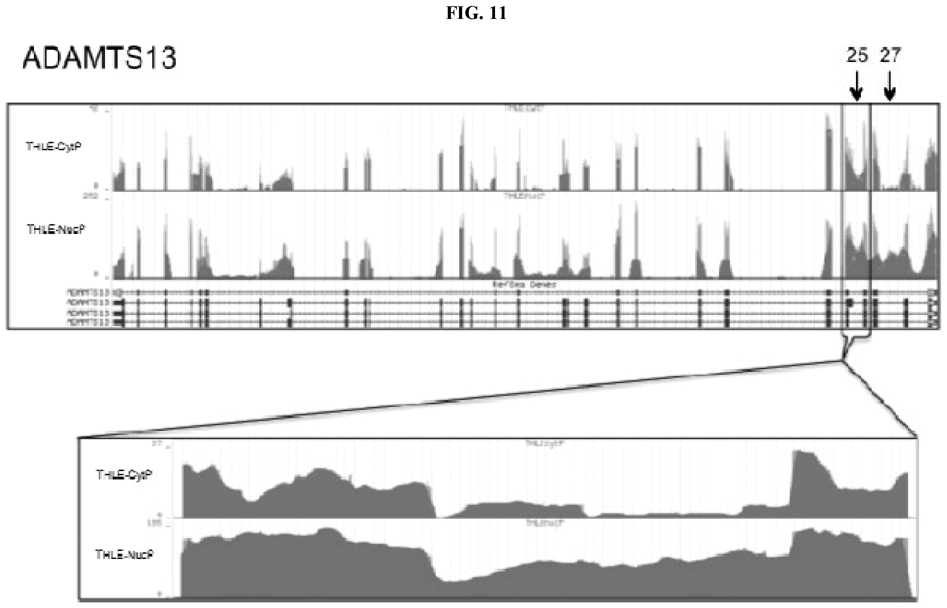

Figure 11 shows the identification of intron-retention events in the ADAMTS13 gene using RNA sequencing (RNAseq), visualized in the UCSC Genome Browser, as described in Example 5. The top panel shows the read density corresponding to the ADAMTS13 transcript expressed in THLE-3 (human liver epithelial) cells and localized in either the cytoplasmic (top) or nuclear fraction (bottom). At the bottom of this panel, a graphic representation of all the refseq. isoforms of the ADAMTS13 gene is shown to scale. The read density is shown as peaks. The highest read density corresponds to exons (black boxes), while no reads are observed for the majority of the introns (lines with arrow heads) in neither cellular fraction. Higher read density is detected for introns 25 and 27 (pointed by the arrows) in the nuclear fraction compared to the cytoplasmic fraction indicating that splicing efficiency of introns 25 and 27 is low, resulting in intron retention. The retained-intron containing pre-mRNA transcripts are retained in the nucleus and are not exported out to the cytoplasm. The read density for intron 25 in THLE-3 cells is shown in detail in the bottom picture.

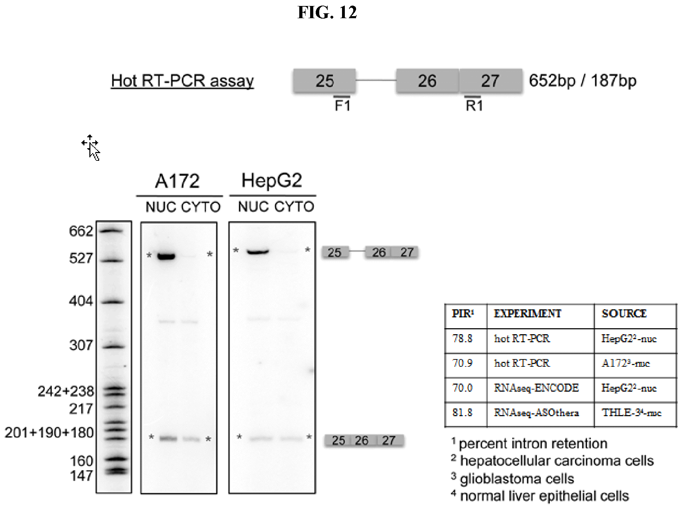

Figure 12 shows the validation of the bioinformatic analysis via radioactive-RT-PCR as described in Example 6. A schematic representation of the radioactive RT-PCR assay to validate the bioinformatic prediction shown in Figure 11 is depicted in Figure 12. The numbered rectangles denote exons, and intervening lines denote introns. Black lines indicate forward ("F1") and reverse ("R1") primers used in the PCR amplification of the ADAMTS-13 transcript resulting in two products, the intron-25-retained (652 bp) and the correctly spliced (187 bp) products. Below are representative gels showing radioactive RT-PCR products from nuclear and cytoplasmic fractions of A172 (glioblastoma, left) and HepG2 (hepatocellular carcinoma, right) cells separated in a 5% polyacrylamide gel. Asterisks indicate correct products (by size). Results show a band corresponding to the intron-25 retained product in the nuclear fractions of both cell lines that is absent from both cytoplasmic fractions. A summary of the quantification on ADAMTS13 intron-25 retention calculated as percent intron retention (PIR) from radioactive RT-PCR and RNAseq experiments is shown on the table on the right.

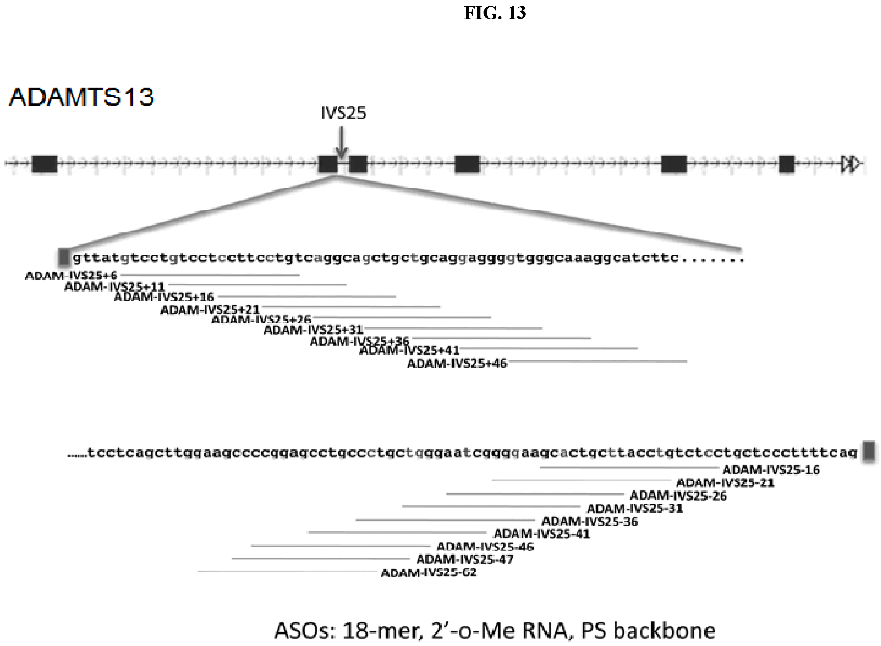

Figure 13 shows a graphic representation of the ASO walk performed for ADAMTS13 IVS 25 targeting sequences immediately downstream of the 5' splice site or upstream of the 3' splice site using 2'-O-Me ASOs, PS backbone, as described in Example 7. ASOs were designed to cover these regions by shifting 5 nucleotides at a time. Exons 24 to 29 and the intronic sequences to are drawn to scale.

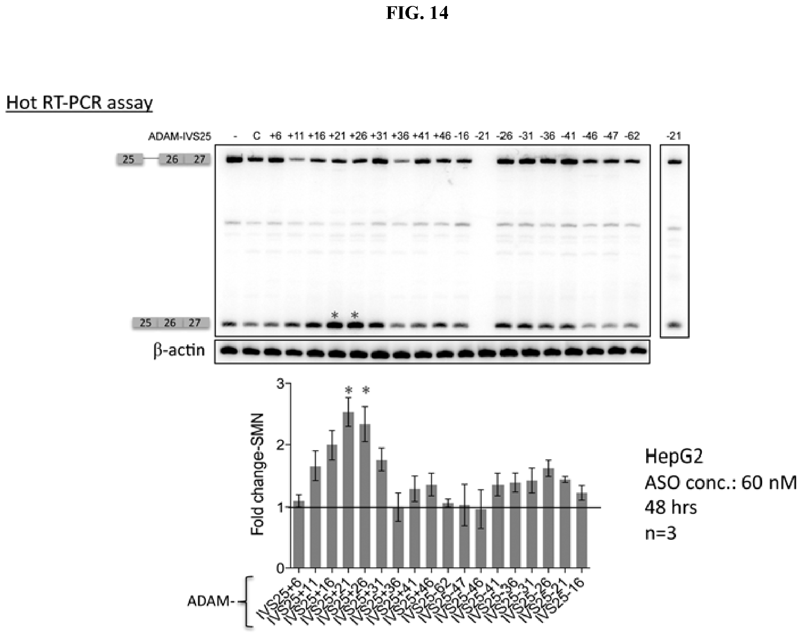

Figure 14 depicts the results of the ASO-walk targeting intron 25 as described in Example 8. At the top, a representative gel shows radioactive RT-PCR products of ADAMTS13 mock-treated (-, RNAiMAX only), SMN-control ASO treated, or treated with a 2'-O-Me ASO targeting intron 25 as described in Figure 13, at 60nM concentration in HepG2 cells. Quantification of the bands corresponding to ADAMTS13 products normalized to Beta actin from 3 independent experiments is plotted in the bar graph below as fold change with respect to the SMN-control-ASO treated products. The black line indicates a ratio of 1, no change. Asterisks indicate ASOs that lead to the highest increase in mRNA levels.

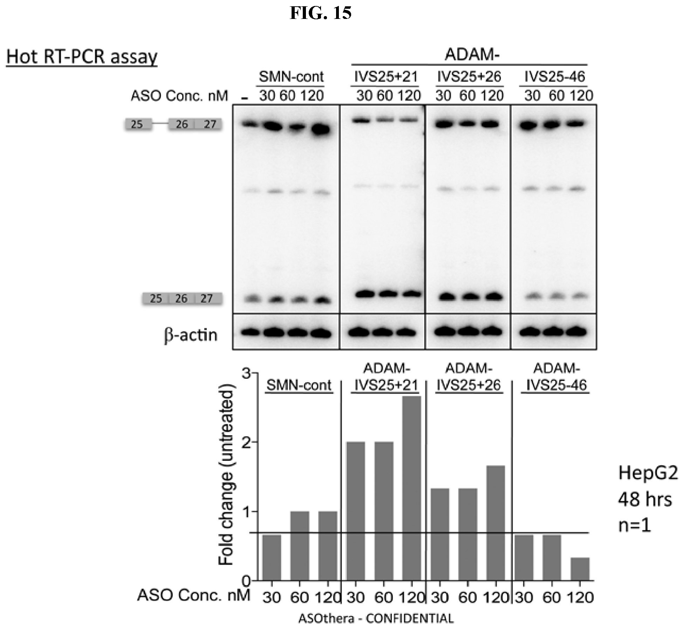

Figure 15 shows dose-response curves for ADAM-IVS25+21, ADAM-IVS25+26, the two best targeting ASOs, and ADAM-IVS-46, an ASO that resulted in a reduction of ADAMTS13 transcript, as described in Example 9. In the top panel a representative gel shows radioactive RT-PCR ADAMTS13 products from HepG2 cells mock-, SMN-control-, ADAM-IVS25+21-, ADAM-IVS25+26-, or ADAM-IVS-46-treated at the indicated concentrations. The RT-PCR products were separated in a 5% polyacrylamide gel. Quantification of the bands corresponding to ADAMTS13 products normalized to Beta actin is plotted in the bar graph below as fold change relative to the mock-treated products. The black line indicates a ratio of 1, no change.

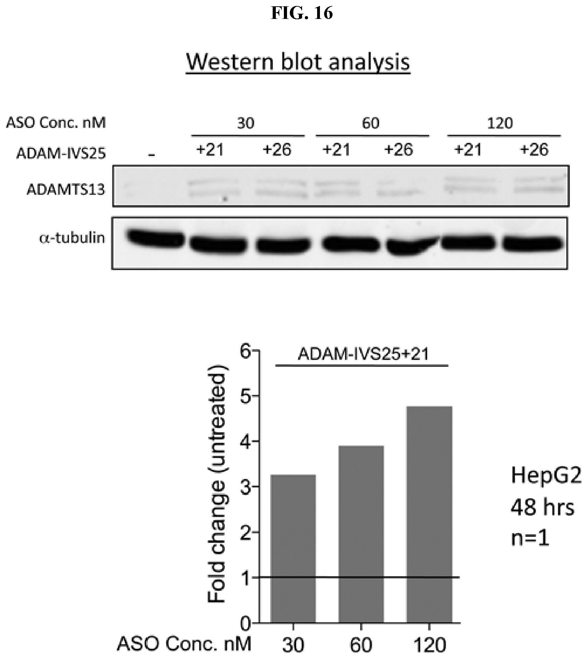

Figure 16 illustrates an increase in ADAMTS13 protein resulting from the treatment of HepG2 cells with ADAM-IVS25+21 and ADAM-IVS25+26 ASOs, as described in Example 10. In the top panel a representative gel shows ADAMTS13 protein products from HepG2 cells mock-, ADAM-IVS25+21-, or ADAM-IVS25+26-treated at the indicated concentrations. The protein products were separated on an 8% SDS-polyacrylamide gel. Antibodies against ADAMTS-13 and alpha tubulin were used to detect the protein products. The bar graph below shows the quantifications of the intensity of bands corresponding to ADAMTS-13 protein levels from ADAM-IVS25+21-treated cells, normalized to alpha tubulin. Fold change was plotted relative to the product from mock-treated cells. The black line indicates a ratio of 1, no change. ADAM-IVS25+21 increases ADAMTS13 protein product in a dose-dependent manner.

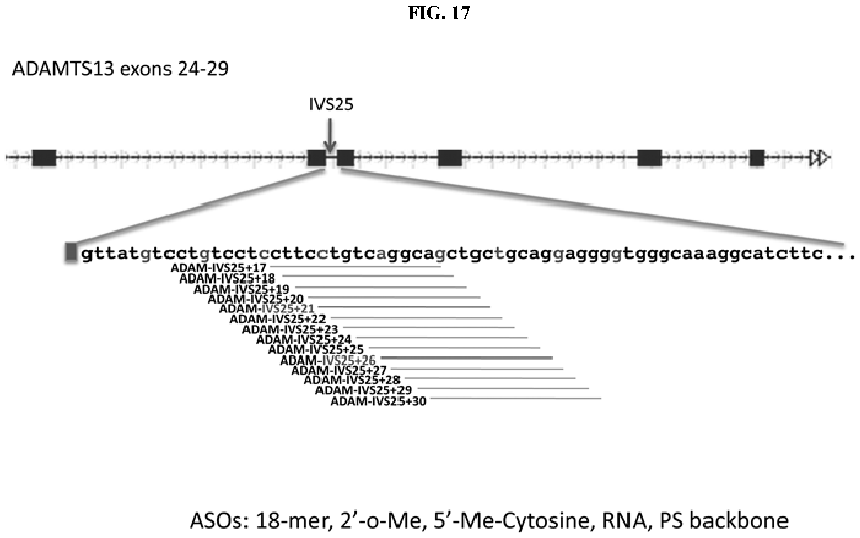

Figure 17 shows a graphic representation of the ASO microwalk performed for ADAMTSI13 IVS 25 targeting sequences in the region of ADAM-IVS25+21 and ADAM-IVS25+26 ASOs using 2'-O-Me, 5'-Me-Cytosine ASOs, as described in Example 11. ASOs were designed to cover the region by shifting 1 nucleotide at a time. Exons 24 to 29 and the intronic sequences are drawn to scale.

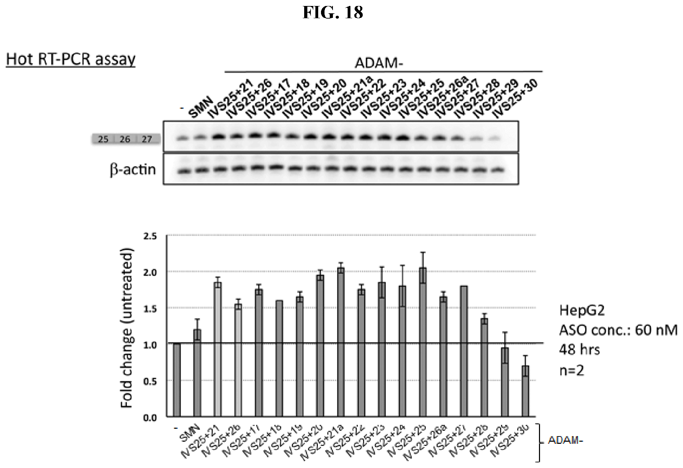

Figure 18 depicts the results of the ASO-microwalk targeting the ADAM-IVS25+21 and ADAM-IVS25+26 region in intron 25, as described in Example 12. At the top, a representative gel shows radioactive RT-PCR products of ADAMTS13 mock-treated (-), SMN-control ASO treated, or treated with a 2'-O-Me, 5'-Me-Cytosine ASOs (described in Figure 17) at 60nM concentration in HepG2. Quantification of the bands corresponding to ADAMTS13 products normalized to Beta actin from 2 independent experiments is plotted in the bar graph below as fold change relative to the mock-treated products. The black line indicates a ratio of 1, no change. The two light-grey bars indicate IVS25 2'-O-Me ASOs ADAM-IVS25+21 and ADAM-IVS25+26 described in Figure 14 and 15.

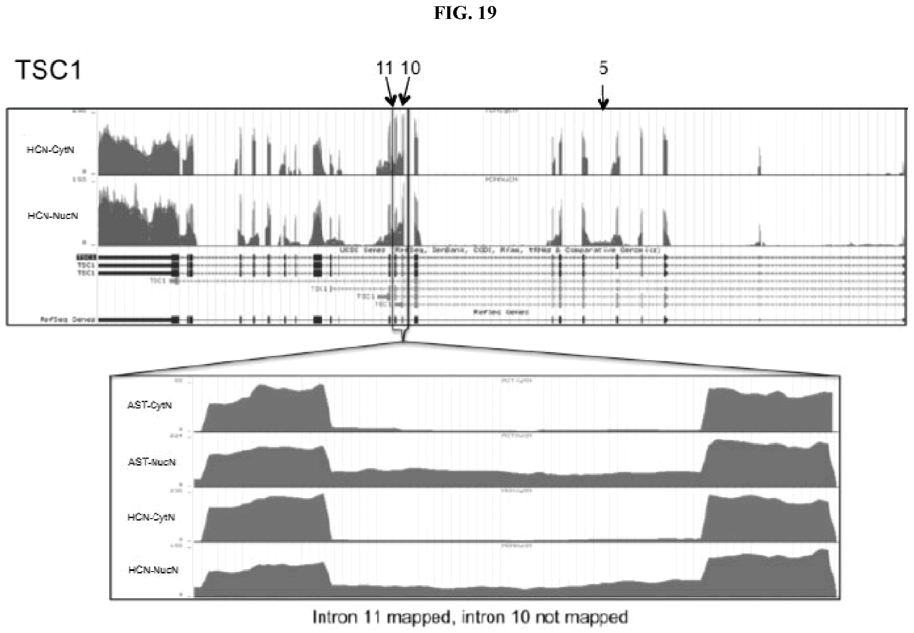

Figure 19 shows the identification of intron-retention events in the TSC1 gene using RNA sequencing (RNAseq), visualized in the UCSC genome browser as described in Example 13. The top panel shows the read density corresponding to the TSC1 transcript expressed in HCN (primary human cortical neuron) cells and localized in either the cytoplasmic (top) or nuclear fraction (bottom). At the bottom of this panel, a graphic representation of all the refseq. isoforms of the TSC1 gene is shown to scale. The read density is shown as peaks. The highest read density corresponds to exons (black boxes), while no reads are observed for the majority of the introns (lines with arrow heads) in neither cellular fraction. Higher read density is detected for introns 5, 10, and 11 (pointed by the arrows) in the nuclear fraction compared to the cytoplasmic fraction indicating that splicing efficiency of introns 5, 10 and 11 is low, resulting in intron retention. The retained-intron containing pre-mRNA transcripts are retained in the nucleus and are not exported out to the cytoplasm. The read density for intron 10 is shown in detail in the bottom picture for HCN cells and AST (primary human astrocyte) cells.

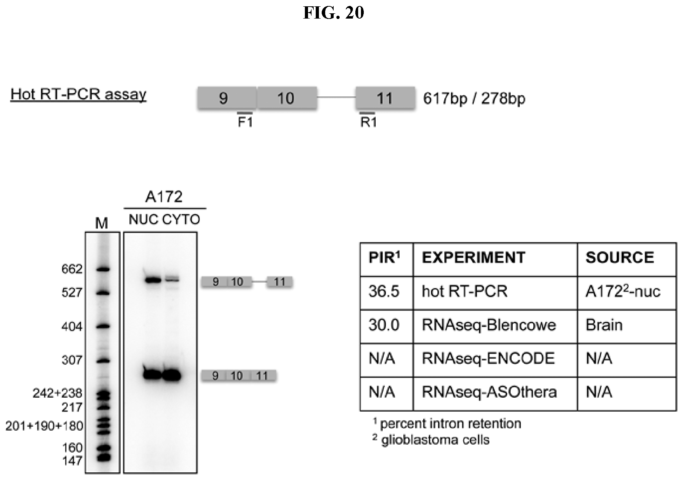

Figure 20 shows a schematic representation of the radioactive RT-PCR assay to validate the bioinformatic prediction shown in Figure 19, as described in Example 14. The numbered rectangles denote exons, and intervening lines denote introns. Black lines indicate forward ("F1") and reverse ("R1") primers used in the PCR amplification of the TSC1 transcript resulting in two products, the intron-10-retained (617 bp) and the correctly spliced (278 bp) products. Below are representative gels showing radioactive RT-PCR products from nuclear and cytoplasmic fractions of A172 (glioblastoma) cells separated in a 5% polyacrylamide gel. Results show a band corresponding to the intron-10 retained product in the nuclear fractions of A172 cells that is significantly reduced in the cytoplasmic fraction. Quantification of the bands indicated that approximately 36% of TSC1 transcripts contain intron 10 and that this product is retained in the nucleus. Moreover, as shown for ADAMTS13, the radioactive RT-PCR results validated the bioinformatic predictions. A summary of the quantification on TSC1 intron-10 retention calculated as percent intron retention (PIR) from radioactive RT-PCR and RNAseq experiments is shown on the table on the right.

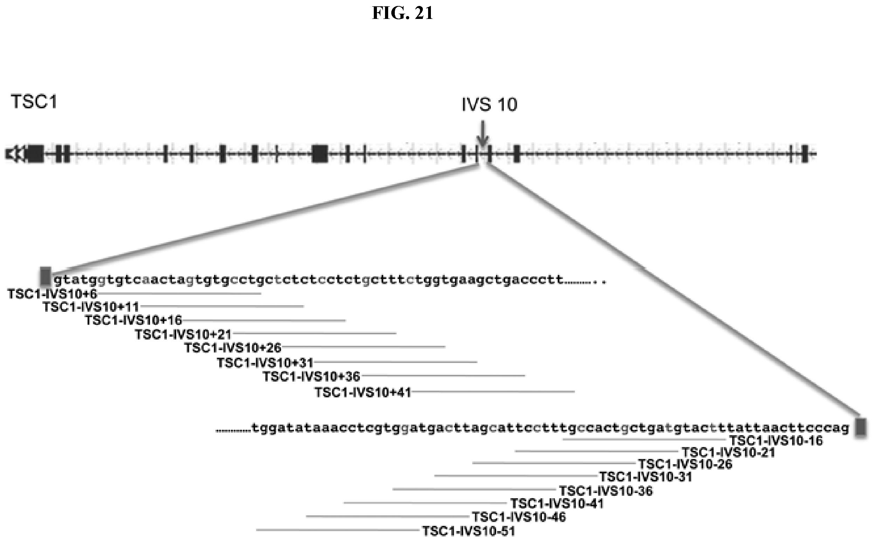

Figure 21 shows a graphic representation of the ASO walk performed for TSC1 IVS 10 targeting sequences immediately downstream of the 5' splice site or upstream of the 3' splice site using 2'-O-Me ASOs, PS backbone, as described in Example 15. ASOs were designed to cover these regions by shifting 5 nucleotides at a time. TSC1 exon-intron structure is drawn to scale.

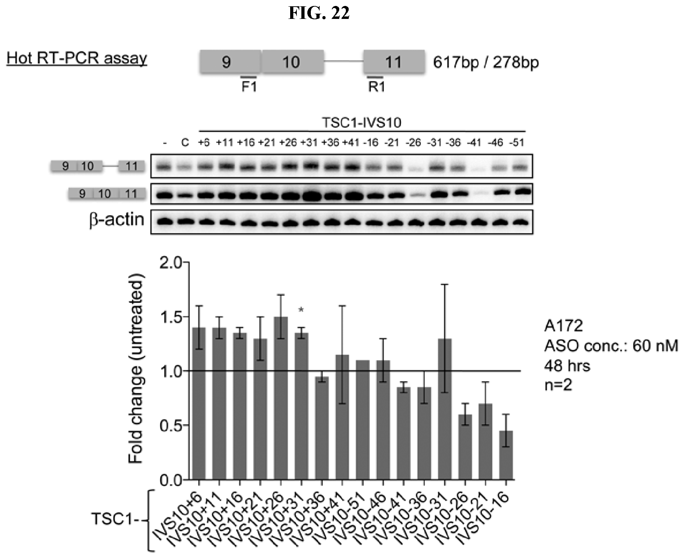

Figure 22 depicts the results of the ASO-walk targeting intron 10, as described in Example 16. At the top, a representative gel shows radioactive RT-PCR products of TSC1 mock-treated (-), SMN-control ASO treated, or treated with a 2'-O-Me ASO targeting intron 10 as described in Figure 21, at 60nM concentration in A172 cells. Quantification of the bands corresponding to TSC1 products normalized to Beta actin from 2 independent experiments is plotted in the bar graph below as fold change with respect to the mock-treated products. The black line indicates a ratio of 1, no change. Asterisks indicate ASOs that lead to an increase in TSC1 mRNA levels.

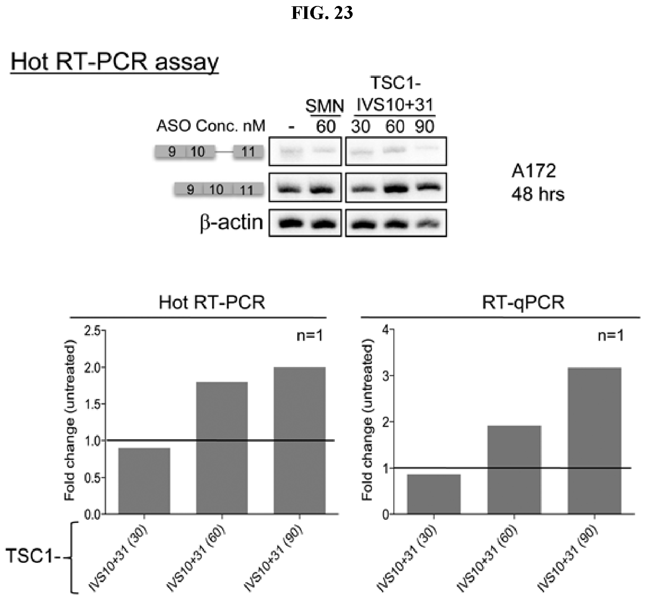

Figure 23 shows a dose-response curve for TSC1-IVS10+31 ASO, as described in Example 17. In the top panel a representative gel shows radioactive RT-PCR TSC1 products from A172 cells mock-, SMN-control-, or TSC1-IVS10+31-treated at the indicated concentrations. The RT-PCR products were separated in a 5% polyacrylamide gel. Quantification of the bands corresponding to TSC1 products normalized to Beta actin is plotted in the bar graph on the left below as fold change relative to the mock-treated products. RT-qPCR results of the same experiment are plotted relative to mock-treated products on the right bar graph confirming the radioactive RT-PCR results. The black line indicates a ratio of 1, no change.

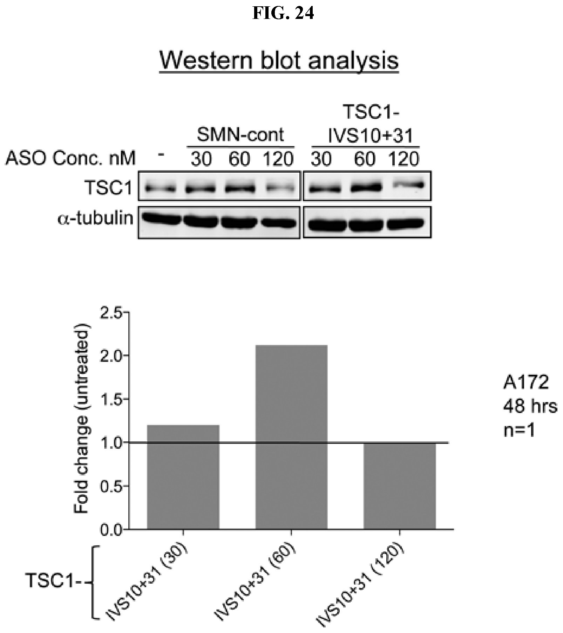

Figure 24 illustrates an increase in TSC1 protein resulting from the treatment of A172 cells with TSC1-IVS10+31 ASO, as described in Example 18. In the top panel a representative gel shows TSC1 protein products from A172 cells mock-, SMN-control-, or TSC1-IVS10+31 ASO-treated at the indicated concentrations. The protein products were separated on a 10% SDS-polyacrylamide gel. Antibodies against TSC1 and alpha tubulin were used to detect the protein products. The bar graph below shows the quantifications of the intensity of bands corresponding to TSC1 protein levels from TSC1-IVS10+31-treated cells, normalized to alpha tubulin. Fold change was plotted relative to the product from mock-treated cells. The black line indicates a ratio of 1, no change. TSC1-IVS10+31 increases TSC1 protein product.

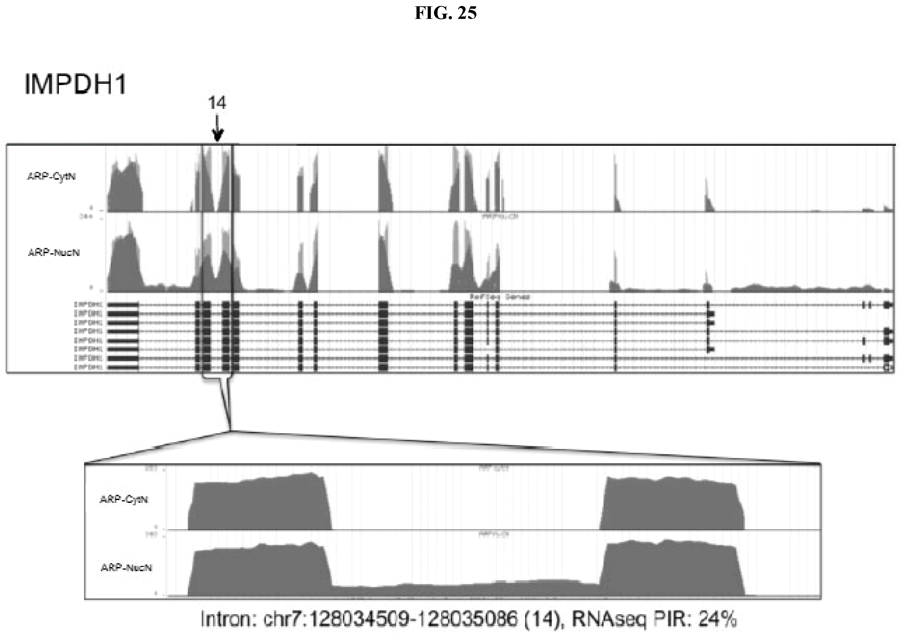

Figure 25 shows the identification of intron-retention events in the IMPDH1 gene using RNA sequencing (RNAseq), visualized in the UCSC genome browser as described in Example 19. The top panel shows the read density corresponding to the IMPDH1 transcript expressed in ARPE19 (human retinal epithelial) cells and localized in either the cytoplasmic (top) or nuclear fraction (bottom). At the bottom of this panel, a graphic representation of all the refseq. isoforms of the IMPDH1 gene is shown to scale. The read density is shown as peaks. The highest read density corresponds to exons (black boxes), while no reads are observed for the majority of the introns (lines with arrow heads) in either cellular fraction. Higher read density is detected for intron 14 (pointed by the arrow) in the nuclear fraction compared to the cytoplasmic fraction indicating that splicing efficiency of intron 14 is low, resulting in intron retention. The retained-intron containing pre-mRNA transcripts are retained in the nucleus and are not exported out to the cytoplasm. The read density for intron 14 is shown in detail in the bottom picture for ARPE19 cells.

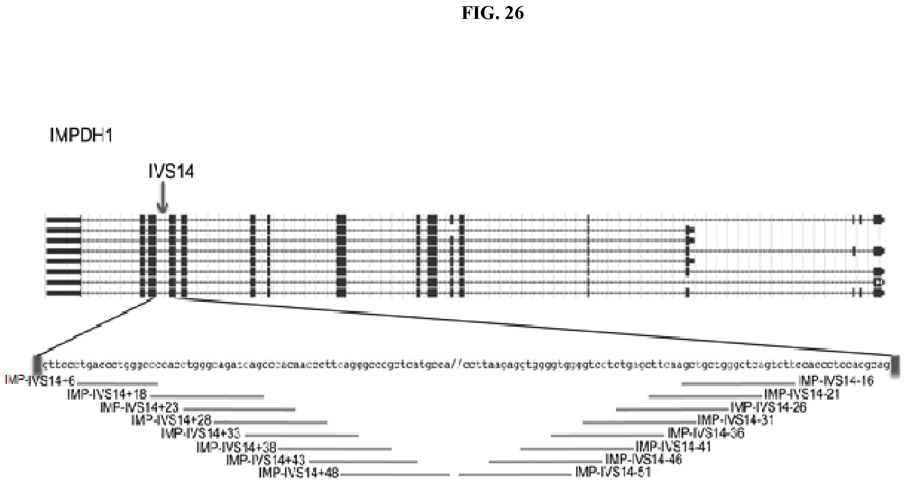

Figure 26 shows a graphic representation of the ASO walk performed for IMPDH1 IVS 14 targeting sequences immediately downstream of the 5' splice site or upstream of the 3' splice site using 2'-O-Me ASOs, as described in Example 20, PS backbone. ASOs were designed to cover these regions by shifting 5 nucleotides at a time, unless a stretch of four guanines is present in the ASOs. IMPDH1 exon-intron structure is drawn to scale.

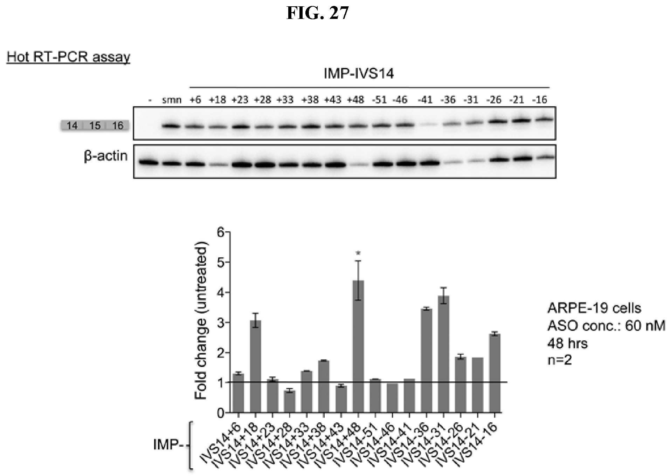

Figure 27 depicts the results of the ASO-walk targeting intron 14, as described in Example 21. At the top, a representative gel shows radioactive RT-PCR products of IMPDH1 mock-treated (-), SMN-control ASO-treated, or treated with a 2'-O-Me ASO targeting intron 14 as described in Figure 21, at 60nM concentration in ARPE19 cells. Quantification of the bands corresponding to IMPDH1 products normalized to Beta actin from 2 independent experiments is plotted in the bar graph below as fold change relative to the mock-treated products. The black line indicates a ratio of 1, no change. Asterisks indicate the ASO that lead to the highest increase in IMPDH1 mRNA levels.

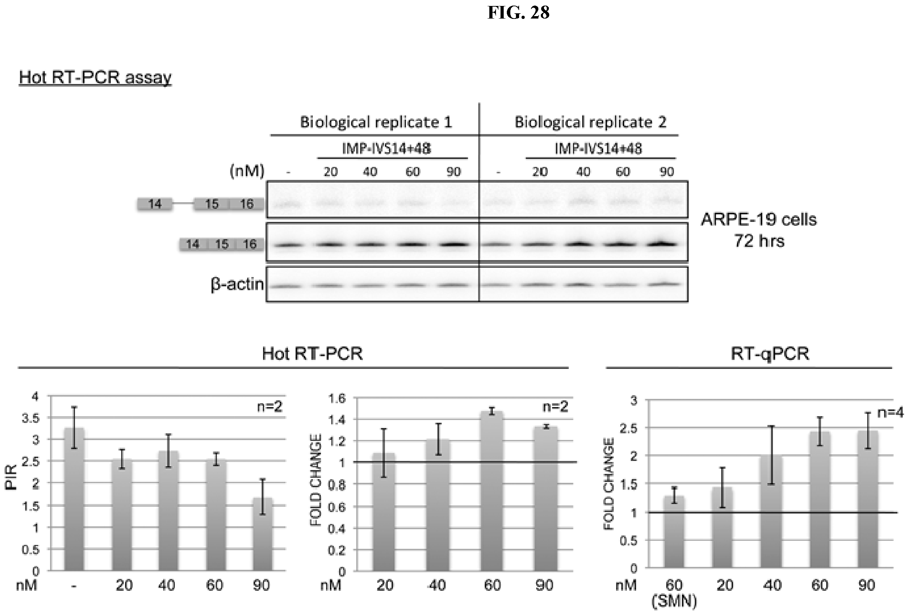

Figure 28 shows an increase in IMPDH1 gene expression levels in a dose-dependent manner resulting from the treatment of ARPE19 cells with IMP-IVS14+48 ASO at the indicated concentrations, as described in Example 22. Radioactive RT-PCR products of IMPDH1 (intron-14 retained and correctly spliced) and Beta actin from ARPE-19 cells were separated on a 5% polyacrylamide gel. The bar graph on the left demonstrates a dose-dependent reduction in percent intron retention (PIR) calculated relative to the total transcript (intron-14 retained and correctly spliced) from IMP-IVS14+48 ASO-treated cells compared to mock-treated cells (two independent experiments). Fold change of correctly spliced transcript level from two independent experiments was plotted relative to the mock-treated cells in the middle graph showing a dose-dependent increase in IMPDH1 transcript level. RT-qPCR (right bar graph) was performed and the resulting values were normalized to Beta actin. Fold change of four biological replicates was plotted relative mock-treated IMPDH1 products, confirming the radioactive RT-PCR results.

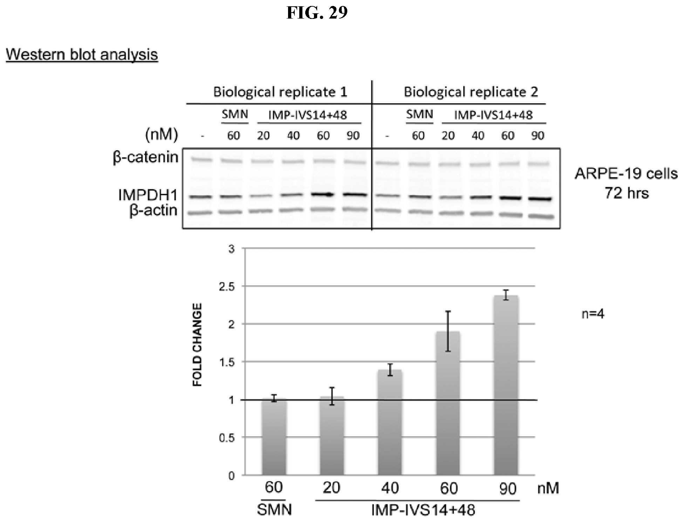

Figure 29 shows an increase in IMPDH1 protein levels achieved via IMP-IVS14+48 ASO targeting at the indicated concentrations in ARPE19 cells, as described in Example 23. Protein lysates from ARPE-19 cells were separated on a 4-20% SDS-polyacrylamide gel. Antibodies against IMPDH1, Beta actin and Beta catenin were used to detect protein products. The intensity of the IMPDH1 protein bands was normalized to the intensity of the Beta actin bands and the fold change was computed relative to the mock-treated products from four biological replicates, and plotted in the bar graph below.

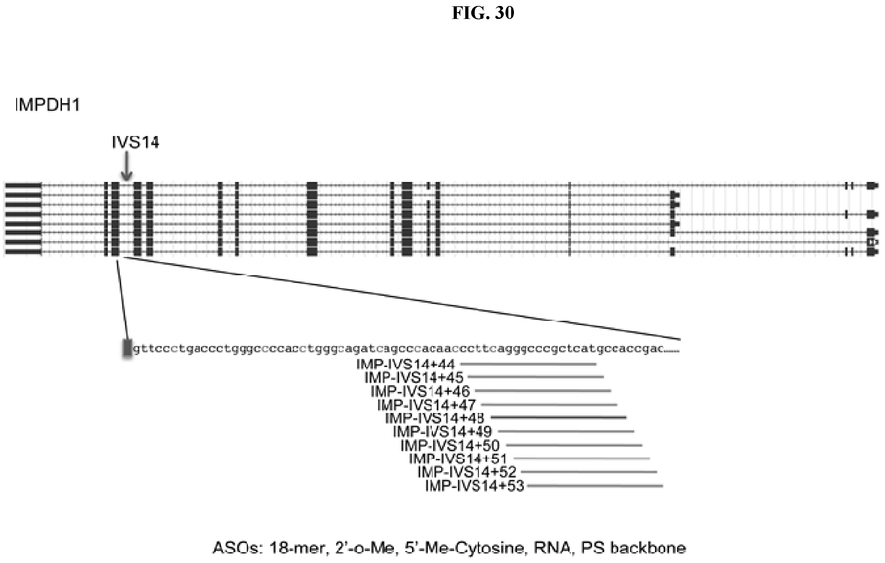

Figure 30 shows a graphic representation of the ASO microwalk performed for IMPDH1 IVS14 targeting sequences in the region of IMP-IVS14+48 ASO using 2'-O-Me, 5'-Me-Cytosine ASOs, as described in Example 24. ASOs were designed to cover the region by shifting 1 nucleotide at a time. IMPDH1 exon-intron structure is drawn to scale.

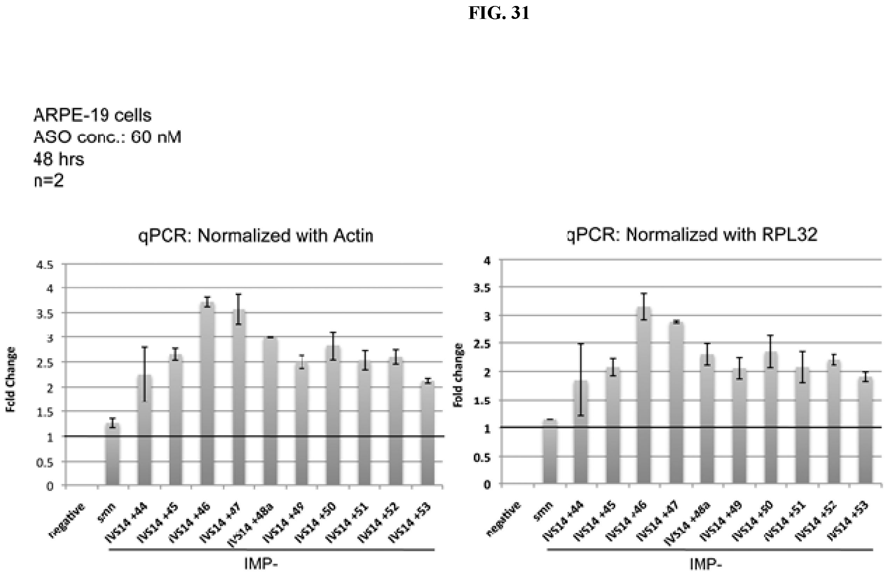

Figure 31 shows an increase in MPDH1 expression levels resulting from a microwalk as shown in Figure 30, as described in Example 25. RT-qPCR was performed on total RNA extracted from ARPE-19 cells, which were treated at an ASO concentration of 60nM. Ct values of the IMPDH1 gene were normalized to the ct values Beta Actin (left) and RPL32 (right) house keeping genes, and the fold change was plotted relative to the products from mock-treated cells in the bar graphs. The microwalk identified two additional ASOs that further increase IMPDH1 transcript levels.

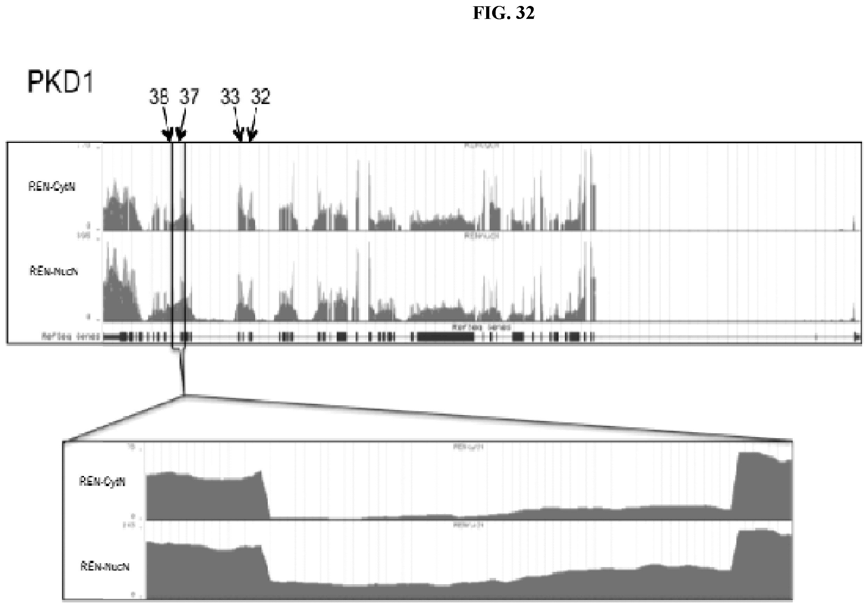

Figure 32 shows the identification of intron-retention events in the PKD1 gene using RNA sequencing (RNAseq), visualized in the UCSC genome browser as described in Example 26. The top panel shows the read density corresponding to the PKD1 transcript expressed in primary human renal epithelial cells (REN) and localized in either the cytoplasmic (top) or nuclear fraction (bottom). At the bottom of this panel, a graphic representation of the refseq. isoform of the PKD1 gene is shown to scale. The read density is shown as peaks. The highest read density corresponds to exons (black boxes), while no reads are observed for the majority of the introns (lines with arrow heads) in neither cellular fraction. Higher read density is detected for introns 32, 33, 37, and 38 (pointed by the arrows) in the nuclear fraction compared to the cytoplasmic fraction indicating that splicing efficiency of these introns is low, resulting in intron retention. The retained-intron containing pre-mRNA transcripts are retained in the nucleus and are not exported out to the cytoplasm. The read density for intron 37 is shown in detail in the bottom picture for REN cells.

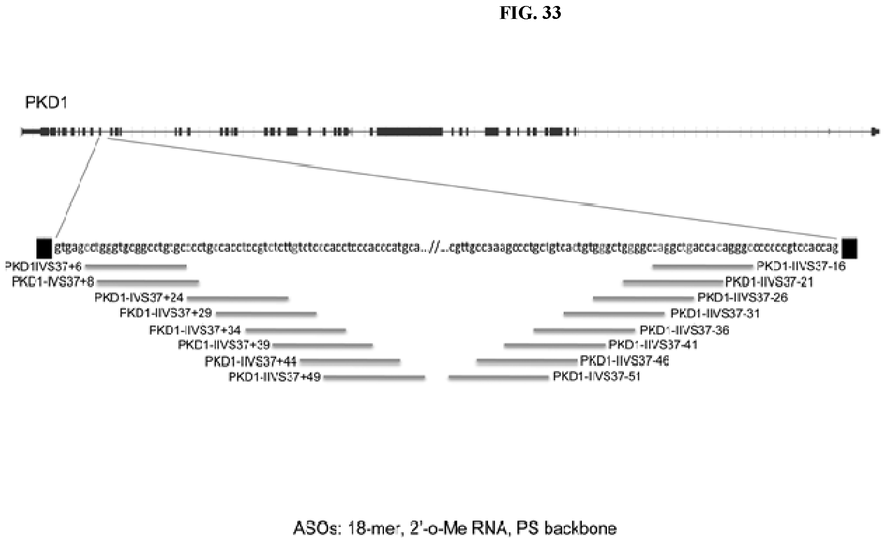

Figure 33 shows a graphic representation of the ASO walk performed for PKD1 IVS 37 targeting sequences immediately downstream of the 5' splice site or upstream of the 3' splice site using 2'-O-Me ASOs, PS backbone, as described in Example 27. ASOs were designed to cover these regions by shifting 5 nucleotides at a time, unless a stretch of four guanines is present in the ASOs. PKD1 exon-intron structure is drawn to scale.

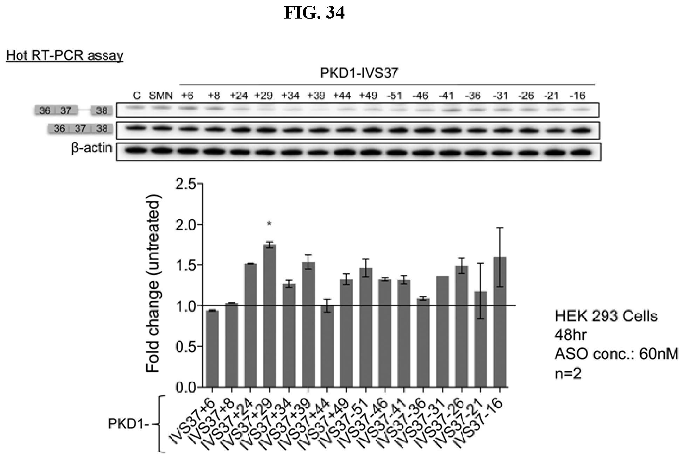

Figure 34 depicts the results of the ASO-walk targeting intron 37, as described in Example 28. At the top, a representative gel shows radioactive RT-PCR products of PKD1 mock-treated (C), SMN-control ASO-treated, or treated with a 2'-O-Me ASO targeting intron 37 as described in Figure 33, at 60nM concentration in HEK293 (human embryonic kidney epithelial) cells. Quantification of the bands corresponding to PKD1 products normalized to Beta actin from 2 independent experiments is plotted in the bar graph below as fold change relative to the mock-treated products. The black line indicates a ratio of 1, no change. Asterisks indicate the ASO that lead to the highest increase in PKD1 mRNA levels.

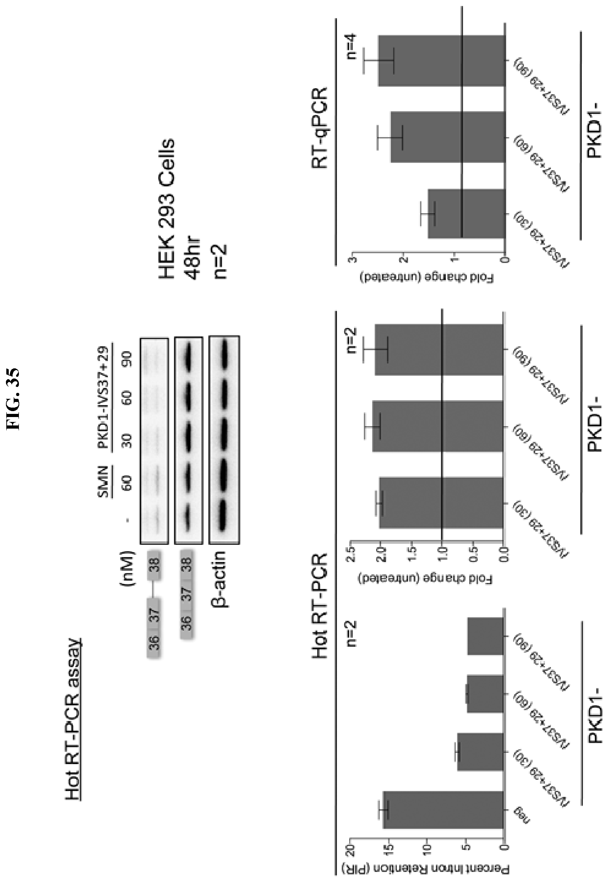

Figure 35 shows an increase in PKD1 gene expression levels in a dose-dependent manner resulting from the treatment of HEK293 cells with PKD1-IVS37+29 ASO at the indicated concentrations, as described in Example 29. Radioactive RT-PCR products of PKD1 (intron-37 retained and correctly spliced) and Beta actin from HEK293 cells were separated on a 5% polyacrylamide gel. The bar graph on the left demonstrates a dose-dependent reduction in percent intron retention (PIR) calculated relative to the total transcript (intron-37 retained and correctly spliced) from PKD1-IVS37+29 ASO-treated cells compared to mock-treated cells (two independent experiments). Fold change of correctly spliced transcript level from two independent experiments was plotted relative to the mock-treated cells in the middle graph showing an increase in PKD1 transcript level. RT-qPCR (right bar graph) was performed and the resulting values were normalized to Beta actin. Fold change of four biological replicates was plotted relative mock-treated PKD1 products, confirming the radioactive RT-PCR results and showing a dose-dependent increase in PKD1 transcript level.

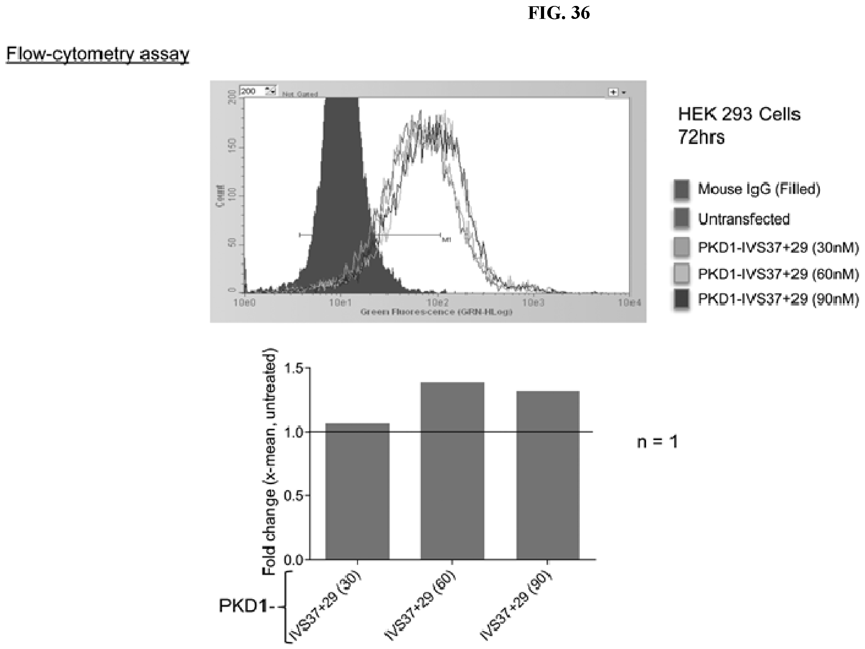

Figure 36 shows an increase in PKD1 protein levels achieved via PKD1-IVS37+29 ASO targeting at the indicated concentrations in HEK293 cells, as described in Example 30. HEK293 were fixed and permeabilized and treated with an antibodies against PKD1, or an IgG isotype control. Flow-cytometry analysis was performed for 10,000 treated cells in each condition and the fluorescence intensity was plotted. The fold change was computed relative to the mock-treated (untransfected) products and plotted in the bar graph below indicating an increase in PKD1 level upon treatment with PKD1-IVS37+29 ASO.

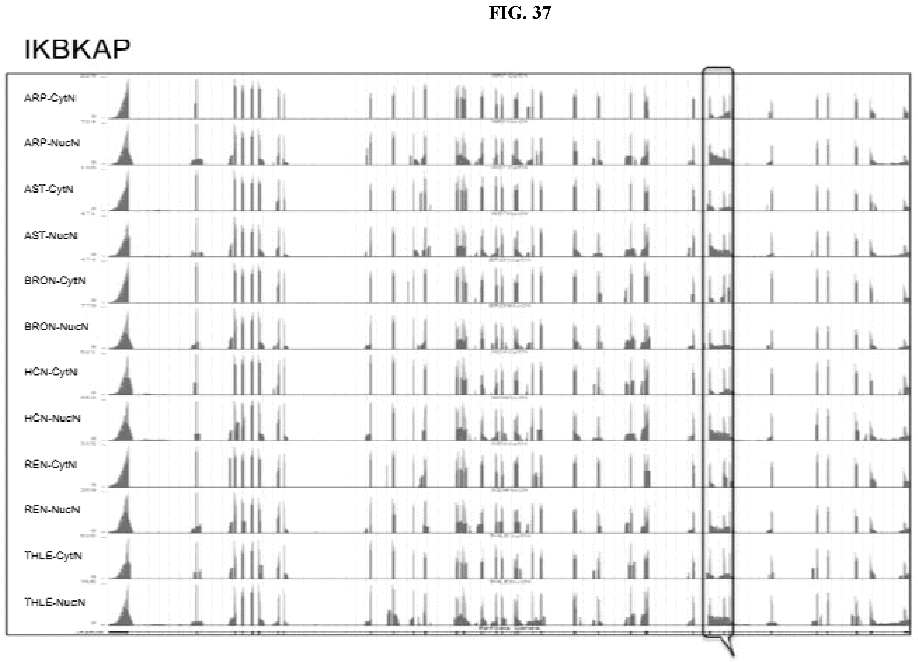

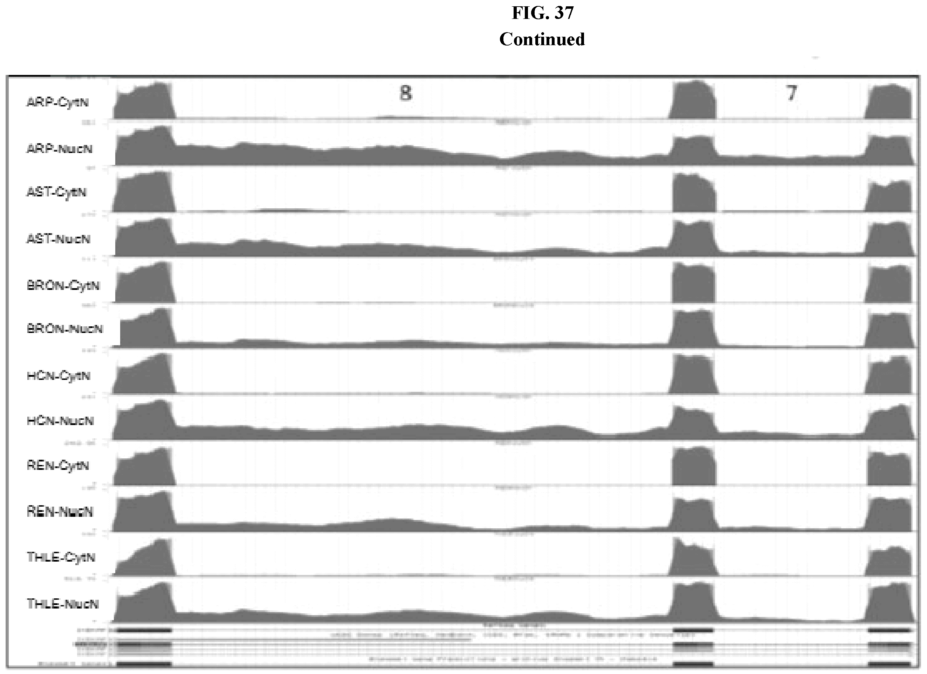

Figure 37 shows the identification of intron-retention events in the IKBKAP gene using RNA sequencing (RNAseq), visualized in the UCSC genome browser as described in Example 31. The top panel shows the read density corresponding to the PKD1 transcript expressed in ARPE19, AST, primary human bronchial epithelial cells (BRON), HCN, REN, and THLE3 cells and localized in either the cytoplasmic (top for each cell line) or nuclear fractions (bottom for each cell line). At the bottom of this panel, a graphic representation of the refseq. isoform of the IKBKAP gene is shown to scale. The read density is shown as peaks. The highest read density corresponds to exons (black boxes), while no reads are observed for the majority of the introns (lines with arrow heads) in neither cellular fraction. Higher read density is detected for introns 7 and 8 (pointed by the arrows) in the nuclear fraction compared to the cytoplasmic fraction indicating that splicing efficiency of these introns is low, resulting in intron retention. The retained-intron containing pre-mRNA transcripts are retained in the nucleus and are not exported out to the cytoplasm. The read densities for introns 7 and 8 are shown in detail in the bottom picture for all the analyzed cells.

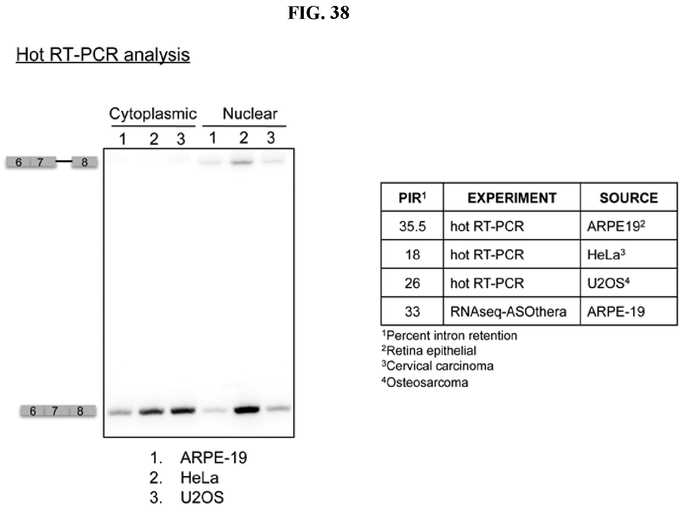

Figure 38 shows IKBKAP intron 7 retention levels in ARPE-19, HeLa and U2OS cell lines respectively, as described in Example 32. Nuclear and cytoplasmic RNA fractions were extracted from ARPE-19, Hela and U2OS cells and their corresponding radioactive RT-PCR products were separated on a 5% polyacrylamide gel. The numbered rectangles denote exons, and intervening lines denote introns. Results show a band corresponding to the intron-7 retained product in the nuclear fractions of the three cell lines that is absent from the corresponding cytoplasmic fractions. Quantification of the bands indicated that approximately 35% of IKBKAP transcripts contain intron 7 and that this product is retained in the nucleus. Once again, the radioactive RT-PCR results validated the bioinformatic predictions. A summary of the quantification of IKBKAP intron-7 retention calculated as percent intron retention (PIR) relative to the total transcript (intron-7 retained and correctly spliced) from radioactive RT-PCR, as well as RNAseq experiment results is shown on the table on the right.

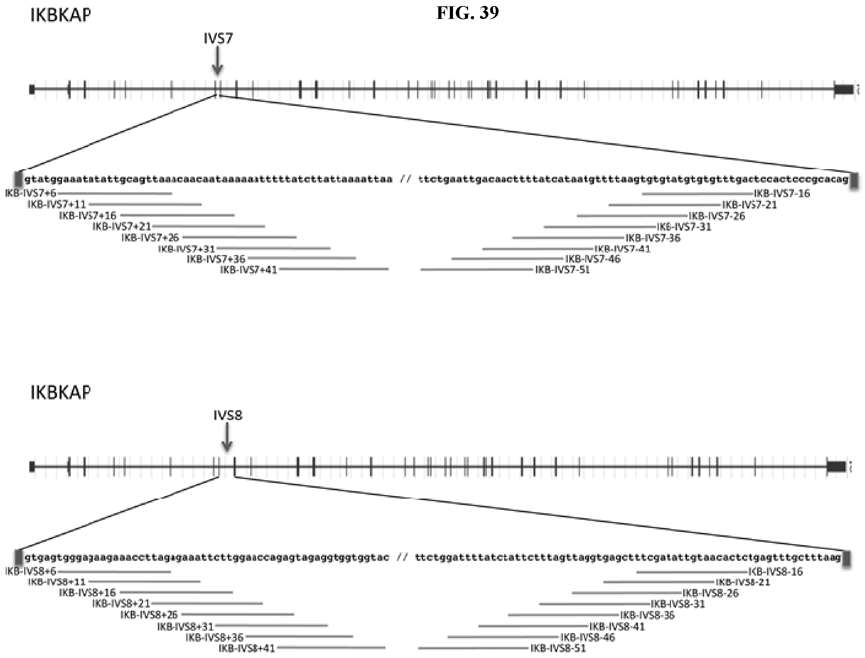

Figure 39 shows a graphic representation of the ASO walks performed for IKBKAP IVS7 (top) and IVS8 (bottom) targeting sequences immediately downstream of the 5' splice site or upstream of the 3' splice site using 2'-O-Me ASOs, PS backbone, as described in Example 33. ASOs were designed to cover these regions by shifting 5 nucleotides at a time. IKBKAP exon-intron structure is drawn to scale.

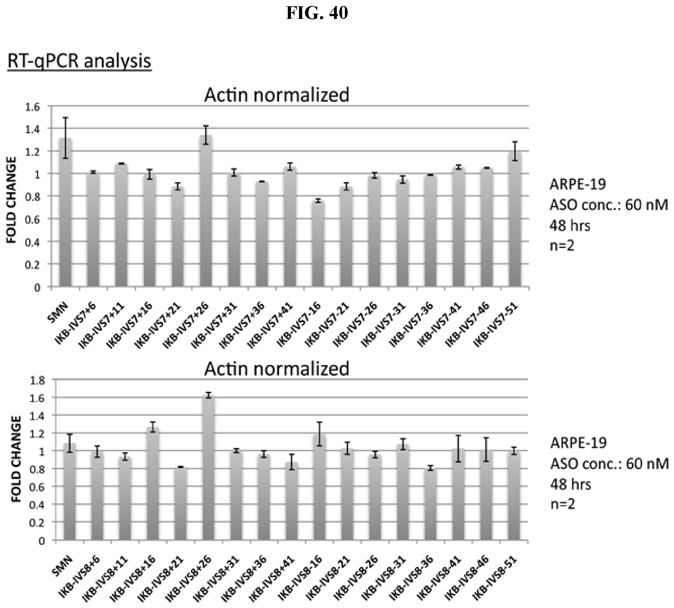

Figure 40 demonstrates an increase in IKBKAP gene expression level achieved via specific ASO targeting of introns 7 (top) and 8 (bottom) as shown in Figure 39, as described in Example 34. Cytoplasmic RNA was extracted from ARPE-19 cells mock-treated, SMN-control ASO-treated or treated with each ASOs at a concentration of 60nM. RT-qPCR was performed to measure IKBKAP expression levels and ct values from IKBKAP were normalized to the corresponding ct values of the Beta actin product. Fold change was plotted relative to mock-treated products.

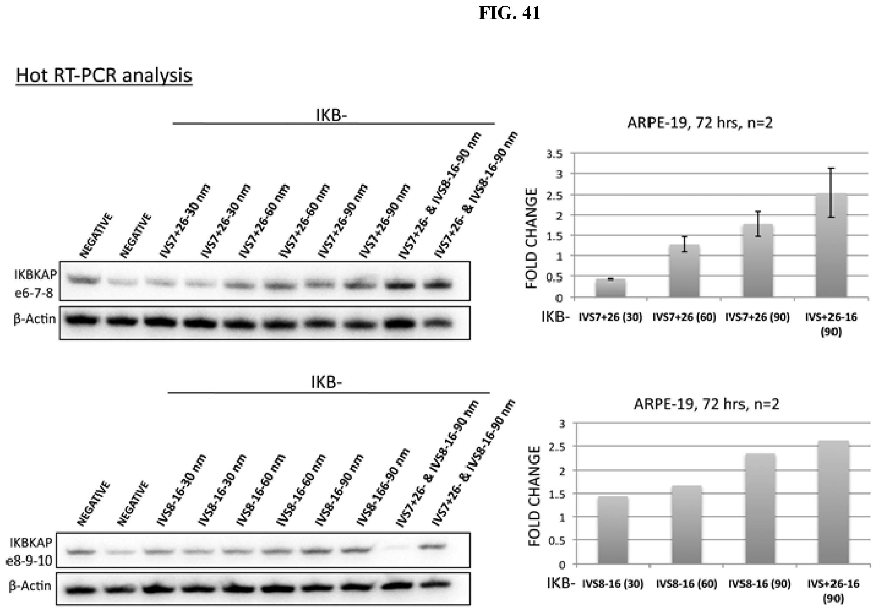

Figure 41 indicates an increase in IKBKAP transcript level in a dose-dependent manner in cells treated with IKB-IVS7+26 or IKB-IVS8-16 ASOs at the indicated concentrations or a combination of both ASOs at 45 nM each (total 90 nM), as described in Example 35. Radioactive RT-PCR products corresponding to exons 6-8 (IKB-IVS7+26, top) or exons 8-10 (IKB-IVS8-16, bottom) using cytoplasmic RNA from ARPE-19 cells were separated on a 5% polyacrylamide gel. The expression of IKBKAP was quantified by measuring the band intensity and the values were normalized to that of Beta-actin. Fold changes from two biological replicates were plotted relative to the product of mock-treated cells and shown in the bar graphs to the right of each representative gel.

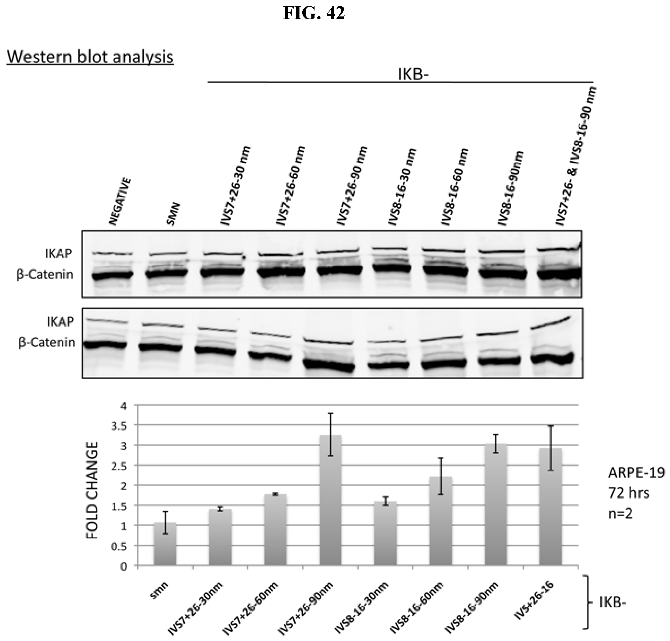

Figure 42 shows a dose-dependent increase in IKAP protein levels in in ARPE19 cells treated with IKB-IVS7+26 or IKB-IVS8-16 ASOs at the indicated concentrations or a combination of both ASOs at 45 nM each (total 90 nM), as described in Example 36. Protein lysates from ARPE-19 cells were extracted and separated on a 4-20% SDS-polyacrylamide gel. Antibodies against IKAP and Beta catenin were used to detect the separated protein products. The intensity of the IKAP protein bands was normalized to the intensity of the Beta catenin bands, and the fold change for two biological replicates was computed relative to the mock-treated cells and plotted in the bar graph below.

### SEQUENCES

This application includes nucleotide sequences SEQ ID NO: 1-374, and these nucleotide sequences are listed in Tables 2 to 8 and Tables 11 to 20 before the claims. The nucleotide sequences set forth as SEQ ID NOS: 1-102 in Tables 11 to 20 are examples of sequences that can be targeted by antisense oligomers by the methods described herein. The nucleotide sequences set forth as SEQ ID NOS 103-374 in Tables 2-8 are examples of antisense oligomers useful in the methods described herein. In all tables, upper case letters represent exon sequence and lower case represents intron sequence.

### DETAILED DESCRIPTION OF THE INVENTION

Eighty-five percent (85%) of human protein-coding genes have at least one intron; eight is the average number of introns per gene and the number of introns can range from 1 to 316. Individual introns are spliced from the primary transcript with different efficiencies and in most cases only the fully spliced mRNA is exported through nuclear pores for subsequent translation in the cytoplasm. Unspliced and partially spliced transcripts are detectable in the nucleus. It is generally thought that nuclear retention of transcripts that are not fully spliced is a mechanism to prevent the accumulation of potentially deleterious mRNAs in the cytoplasm that may be translated to protein. For some genes, splicing of the least efficient intron is a rate-limiting post-transcriptional step in gene expression, prior to translation in the cytoplasm. If splicing of an intron that is rate-limiting for the nuclear stages of gene expression can be made more efficient, steady-state production of fully-spliced, mature mRNA and translation of the corresponding protein can be augmented. Such methods would also aid in upregulating expression of target genes, which has innumerable clinical and research applications. Increasing the output of a gene (the normal and/or mutant allele) can be useful to compensate for any mutation that reduces the amount of activity of its gene product, *e.g.,* a protein or functional RNA. Many genetic diseases and disorders are the result of reduced protein production or the production a protein that is only partially functional.

As used herein, the term "comprise" or variations thereof such as "comprises" or "comprising" are to be read to indicate the inclusion of any recited feature *(e.g.* in the case of an antisense oligomer, a defined nucleobase sequence) but not the exclusion of any other features. Thus, as used herein, the term "comprising" is inclusive and does not exclude additional, unrecited features (*e.g.* in the case of an antisense oligomer, the presence of additional, unrecited nucleobases).

In embodiments of any of the compositions and methods provided herein, "comprising" may be replaced with "consisting essentially of " or "consisting of." The phrase "consisting essentially of" is used herein to require the specified feature(s) (e.g. nucleobase sequence) as well as those which do not materially affect the character or function of the claimed invention. As used herein, the term "consisting" is used to indicate the presence of the recited feature (e.g. nucleobase sequence) alone (so that in the case of an antisense oligomer consisting of a specified nucleobase sequence, the presence of additional, unrecited nucleobases is excluded).

### Targeted Augmentation of Nuclear Gene Output

Described herein are methods of increasing expression of a target protein referred to as Targeted Augmentation of Nuclear Gene Output (TANGO). The method involves contacting cells having (comprising) a retained-intron-containing pre-mRNA (RIC pre-mRNA) that comprises a retained intron, an exon flanking the 5' splice site, an exon flanking the 3' splice site, and encodes the target protein with antisense oligomers (ASO) complementary to a targeted portion of a RIC pre-mRNA. Hybridization of the ASOs to the portion of the RIC pre-mRNA results in enhanced splicing at the splice site (5' splice site or 3' splice site) of the retained intron and subsequently increases target protein production.

The terms "pre-mRNA," and "pre-mRNA transcript" may be used interchangeably and refer to any pre-mRNA species that contains at least one intron. Pre-mRNA or pre-mRNA transcripts may comprise a 5'-7-methylguanosine cap and/or a poly-A tail. In some embodiments, the pre-mRNA transcript does not comprise a 5'-7-methylguanosine cap and/or a poly-A tail. A pre-mRNA transcript is a non-productive messenger RNA (mRNA) molecule if it is not translated into a protein (or transported into the cytoplasm from the nucleus).

As used herein, a "retained-intron-containing pre-mRNA" ("RIC pre-mRNA") is a pre-mRNA transcript that contains at least one retained intron. The RIC pre-mRNA contains a retained intron, an exon flanking the 5' splice site of the retained intron, an exon flanking the 3' splice site of the retained intron, and encodes the target protein. An "RIC pre-mRNA encoding a target protein" is understood to encode the target protein when fully spliced. A "retained intron" is any intron that is present in a pre-mRNA transcript when one or more other introns, such as an adjacent intron, encoded by the same gene have been spliced out of the same pre-mRNA transcript. In some embodiments, the retained intron is the most abundant intron in RIC pre-mRNA encoding the target protein. In embodiments, the retained intron is the most abundant intron in a population of RIC pre-mRNAs transcribed from the gene encoding the target protein in a cell, wherein the population of RIC pre-mRNAs comprises two or more retained introns. In embodiments, an antisense oligomer targeted to the most abundant intron in the population of RIC pre-mRNAs encoding the target protein induces splicing out of two or more retained introns in the population, including the retained intron to which the antisense oligomer is targeted or binds. In embodiments, a mature mRNA encoding the target protein is thereby produced. The terms "mature mRNA," and "fully-spliced mRNA," are used interchangeably herein to describe a fully processed mRNA encoding a target protein (e.g., mRNA that is exported from the nucleus into the cytoplasm and translated into target protein) or a fully processed functional RNA. The term "productive mRNA," also can be used to describe a fully processed mRNA encoding a target protein.

In some embodiments, the targeted region is in a retained intron that is the second most abundant intron in RIC pre-mRNA encoding the target protein. For example, the second most abundant retained intron may be targeted rather than the most abundant retained intron due to the uniqueness of the nucleotide sequence of the second most abundant retained intron, ease of ASO design to target a particular nucleotide sequence, and/or amount of increase in protein production resulting from targeting the intron with an ASO. In embodiments, the retained intron is the second most abundant intron in a population of RIC pre-mRNAs transcribed from the gene encoding the target protein in a cell, wherein the population of RIC pre-mRNAs comprises two or more retained introns. In embodiments, an antisense oligomer targeted to the second most abundant intron in the population of RIC pre-mRNAs encoding the target protein induces splicing out of two or more retained introns in the population, including the retained intron to which the antisense oligomer is targeted or binds. In embodiments, fully-spliced (mature) RNA encoding the target protein is thereby produced.

In embodiments, an antisense oligomer is complementary to a targeted region that is within a non-retained intron in a RIC pre-mRNA. In embodiments, the targeted portion of the RIC pre-mRNA is within: the region +6 to +100 relative to the 5' splice site of the non-retained intron; or the region -16 to -100 relative to the 3' splice site of the non-retained intron. In embodiments, the targeted portion of the RIC pre-mRNA is within the region +100 relative to the 5' splice site of the non-retained intron to -100 relative to the 3' splice site of the non-retained intron. As used to identify the location of a region or sequence, "within" is understood to include the residues at the positions recited. For example, a region +6 to +100 includes the residues at positions +6 and +100. In embodiments, fully-spliced (mature) RNA encoding the target protein is thereby produced.

In some embodiments, the retained intron of the RIC pre-mRNA is an inefficiently spliced intron. As used herein, "inefficiently spliced" may refer to a relatively low frequency of splicing at a splice site adjacent to the retained intron (5' splice site or 3' splice site) as compared to the frequency of splicing at another splice site in the RIC pre-mRNA. The term "inefficiently spliced" may also refer to the relative rate or kinetics of splicing at a splice site, in which an "inefficiently spliced" intron may be spliced or removed at a slower rate as compared to another intron in a RIC pre-mRNA.

In some embodiments, the 9-nucleotide sequence at -3e to -1e of the exon flanking the 5' splice site and +1 to +6 of the retained intron is identical to the corresponding wild-type sequence. In some embodiments, the 16 nucleotide sequence at -15 to -1 of the retained intron and +1e of the exon flanking the 3' splice site is identical to the corresponding wild-type sequence. As used herein, the "wild-type sequence" refers to the nucleotide sequence for a target gene in the published reference genome deposited in the NCBI repository of biological and scientific information (operated by National Center for Biotechnology Information, National Library of Medicine, 8600 Rockville Pike, Bethesda, MD USA 20894). Also used herein, a nucleotide position denoted with an "e" indicates the nucleotide is present in the sequence of an exon (*e.g.,* the exon flanking the 5' splice site or the exon flanking the 3' splice site).

The methods involve contacting cells with an ASO that is complementary to a portion of a pre-mRNA encoding a target protein or functional RNA, resulting in increased expression of a target protein or a functional RNA. As used herein, "contacting" or administering to cells refers to any method of providing an ASO in immediate proximity with the cells such that the ASO and the cells interact. A cell that is contacted with an ASO will take up or transport the ASO into the cell. The method involves contacting a condition or disease-associated or condition or disease-relevant cell with any of the ASOs described herein. In some embodiments, the ASO may be further modified or attached (e.g., covalently attached) to another molecule to target the ASO to a cell type, enhance contact between the ASO and the condition or disease-associated or condition or disease-relevant cell, or enhance uptake of the ASO.

As demonstrated in Figure 2A, in the nucleus of a cell, a pre-mRNA transcript consisting of exons and introns undergoes splicing to generate an mRNA that can be exported from the nucleus into the cytoplasm of the cell where it is translated into protein. In the instance of a pre-mRNA transcript that contains at least one inefficiently spliced intron (a retained intron), a RIC pre-mRNA occurs, which is maintained in the nucleus, and if it is exported to the cytoplasm it is not translated into protein but is degraded. Without wishing to be bound by any particular theory, in the presence of an ASO that is complementary to a targeted portion of the pre-mRNA transcript, splicing of the retained intron is enhanced thereby increasing the amount of mRNA that can be exported and translated into protein is also increased (Figure 2B).

As used herein, the term "increasing protein production" or "increasing expression of a target protein" means enhancing the amount of protein (*e.g.,* a target protein) that is translated from an mRNA in a cell. A "target protein" may be any protein for which increased expression/production is desired. In some embodiments, the target protein is a disease-associated protein, such as any of the proteins presented in Table 1. For example, contacting a cell that expresses a RIC pre-mRNA with an ASO that is complementary to a targeted portion of the RIC pre-mRNA transcript results in a measurable increase in the amount of the protein (*e.g*., a target protein) encoded by the pre-mRNA. Methods of measuring or detecting production of a protein will be evident to one of skill in the art and include, for example, Western blotting, flow cytometry, immunofluorescence microscopy, and ELISA.

In some embodiments, contacting cells with an ASO that is complementary to a targeted portion of a RIC pre-mRNA transcript results in an increase in the amount of protein (*e.g.,* target protein) produced by at least 10, 20, 30, 40, 50, 60, 80, 100, 200, 300, 400, 500, or 1000%, compared to the amount of the protein produced by a cell in the absence of the ASO/absence of treatment. In embodiments, the total amount of target protein produced by the cell to which the antisense oligomer was contacted is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to the amount of target protein produced by an control compound. A control compound can be, for example, an oligonucleotide that is not complementary to the targeted portion of the RIC pre-mRNA.

In some embodiments, contacting cells with an ASO that is complementary to a targeted portion of a RIC pre-mRNA transcript results in an increase in the amount of mRNA encoding the target protein or functional RNA, including the mature mRNA encoding the target protein or functional RNA. In some embodiments, the amount of mRNA encoding the target protein or functional RNA, or the mature mRNA encoding the target protein or functional RNA, is increased by at least 10, 20, 30, 40, 50, 60, 80, 100, 200, 300, 400, 500, or 1000%, compared to the amount of the protein produced by a cell in the absence of the ASO/absence of treatment. In embodiments, the total amount of the mRNA encoding the target protein or functional RNA, or the mature mRNA encoding the target protein or functional RNA produced in the cell to which the antisense oligomer was contacted is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold compared to the amount of mature RNA produced in an untreated cell, *e.g.,* an untreated cell or a cell treated with a control compound. A control compound can be, for example, an oligonucleotide that is not complementary to the targeted portion of the RIC pre-mRNA.

In embodiments, contacting cells with an ASO that is complementary to a targeted portion of a RIC pre-mRNA transcript results in an increase in the amount of a functional RNA. In some embodiments, the amount of the functional RNA is increased by at least 10, 20, 30, 40, 50, 60, 80, 100, 200, 300, 400, 500, or 1000%, compared to the amount of the functional RNA produced by the cell in the absence of the ASO/absence of treatment. In embodiments, the total amount of the functional RNA produced in the cell to which the antisense oligomer was contacted is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold compared to the amount of the functional RNA produced in an untreated cell, *e.g.,* an untreated cell or a cell treated with a control compound. A control compound can be, for example, an oligonucleotide that is not complementary to the targeted portion of the RIC pre-mRNA. Any of the methods provided herein may be used to increase production of a functional RNA, *e.g.,* an mRNA that does not encode a protein, such as a non-protein-coding RNA. In some embodiments, the functional RNA or non-protein-coding RNA is associated with a condition, e.g., a disease or disorder.

### Constitutive Splicing of a Retained Intron from a RIC pre-mRNA

The methods and antisense oligonucleotide compositions provided herein are useful for increasing the expression of a target protein or functional RNA in cells, for example, in a subject having a condition caused by a deficiency in the amount or activity of the target protein or functional RNA, by increasing the level of mRNA encoding the target protein or functional RNA, or the mature mRNA encoding the target protein or functional RNA. In particular, the methods and compositions as described herein induce the constitutive splicing of a retained intron from a RIC pre-mRNA transcript encoding the target protein or functional RNA, thereby increasing the level of mRNA encoding the target protein or functional RNA, or the mature mRNA encoding the target protein or functional RNA and increasing the expression of the target protein or functional RNA.

Constitutive splicing of a retained intron from a RIC pre-mRNA correctly removes the retained intron from the RIC pre-mRNA, wherein the retained intron has wild-type splice sequences. Constitutive splicing, as used herein, does not encompass splicing of a retained intron from a RIC pre-mRNA transcribed from a gene or allele having a mutation that causes alternative splicing or aberrant splicing of a pre-mRNA transcribed from the gene or allele. For example, constitutive splicing of a retained intron, as induced using the methods and antisense oligonucleotides provided herein, does not correct aberrant splicing in or influence alternative splicing of a pre-mRNA to result in an increased expression of a target protein or functional RNA.

In embodiments, constitutive splicing correctly removes a retained intron from a RIC pre-mRNA, wherein the RIC pre-mRNA is transcribed from a wild-type gene or allele, or a polymorphic gene or allele, that encodes a fully-functional target protein or functional RNA, and wherein the gene or allele does not have a mutation that causes alternative splicing or aberrant splicing of the retained intron.

In some embodiments, constitutive splicing of a retained intron from a RIC pre-mRNA encoding the target protein or functional RNA correctly removes a retained intron from a RIC pre-mRNA encoding the target protein or functional RNA, wherein the RIC pre-mRNA is transcribed from a gene or allele from which the target gene or functional RNA is produced at a reduced level compared to production from a wild-type allele, and wherein the gene or allele does not have a mutation that causes alternative splicing or aberrant splicing of the retained intron. In these embodiments, the correct removal of the constitutively spliced retained intron results in production of target protein or functional RNA that is functional when compared to an equivalent wild-type protein or functional RNA.

In other embodiments, constitutive splicing correctly removes a retained intron from a RIC pre-mRNA, wherein the RIC pre-mRNA is transcribed from a gene or allele that encodes a target protein or functional RNA produced in a form having reduced function compared to an equivalent wild-type protein or functional RNA, and wherein the gene or allele does not have a mutation that causes alternative splicing or aberrant splicing of the retained intron. In these embodiments, the correct removal of the constitutively spliced retained intron results in production of partially functional target protein, or functional RNA that is partially functional when compared to an equivalent wild-type protein or functional RNA.

"Correct removal" of the retained intron by constitutive splicing refers to removal of the entire intron, without removal of any part of an exon.

In embodiments, an antisense oligomer as described herein or used in any method described herein does not increase the amount of mRNA encoding the target protein or functional RNA, the amount of the target protein, or the amount of the functional RNA, by modulating alternative splicing or aberrant splicing of a pre-mRNA transcribed from a gene encoding the functional RNA or target protein. Modulation of alternative splicing or aberrant splicing can be measured using any known method for analyzing the sequence and length of RNA species, *e.g.,* by RT-PCR and using methods described elsewhere herein and in the literature. In embodiments, modulation of alternative or aberrant splicing is determined based on an increase or decrease in the amount of the spliced species of interest of at least 10% or 1.1-fold. In embodiments, modulation is determined based on an increase or decrease at a level that is at least 10% to 100% or 1.1 to 10-fold, as described herein regarding determining an increase in mRNA encoding the target protein or functional RNA in the methods of the invention.

In embodiments, the method is a method wherein the RIC pre-mRNA was produced by partial splicing of a wild-type pre-mRNA. In embodiments, the method is a method wherein the RIC pre-mRNA was produced by partial splicing of a wild-type pre-mRNA. In embodiments, the RIC pre-mRNA that was produced by partial splicing of a full-length pre-mRNA. In these embodiments, a full-length pre-mRNA may have a polymorphism in a splice site of the retained intron that does not impair correct splicing of the retained intron as compared to splicing of the retained intron having the wild-type splice site sequence.

In embodiments, the mRNA encoding the target protein or functional RNA is a full-length mature mRNA, or a wild-type mature mRNA. In these embodiments, a full-length mature mRNA may have a polymorphism that does not affect the activity of the target protein or the functional RNA encoded by the mature mRNA, as compared to the activity of the target protein or functional RNA encoded by the wild-type mature mRNA.

### Antisense Oligomers

One aspect of the present disclosure is a composition comprising antisense oligomers that enhances splicing by binding to a targeted portion of a RIC pre-mRNA. As used herein, the terms "ASO" and "antisense oligomer" are used interchangeably and refer to an oligomer such as a polynucleotide, comprising nucleobases, that hybridizes to a target nucleic acid *(e.g.,* a RIC pre-mRNA) sequence by Watson-Crick base pairing or wobble base pairing (G-U). The ASO may have exact sequence complementary to the target sequence or near complementarity (*e.g*., sufficient complementarity to bind the target sequence and enhancing splicing at a splice site). ASOs are designed so that they bind (hybridize) to a target nucleic acid (*e.g.,* a targeted portion of a pre-mRNA transcript) and remain hybridized under physiological conditions. Typically, if they hybridize to a site other than the intended (targeted) nucleic acid sequence, they hybridize to a limited number of sequences that are not a target nucleic acid (to a few sites other than a target nucleic acid). Design of an ASO can take into consideration the occurrence of the nucleic acid sequence of the targeted portion of the pre-mRNA transcript or a sufficiently similar nucleic acid sequence in other locations in the genome or cellular pre-mRNA or transcriptome, such that the likelihood the ASO will bind other sites and cause "off-target" effects is limited. Any antisense oligomers known in the art, for example in PCT Application No. PCT/US2014/054151, published as WO 2015/035091, titled "Reducing Nonsense-Mediated mRNA Decay," can be used to practice the methods described herein.

In some embodiments, ASOs "specifically hybridize" to or are "specific" to a target nucleic acid or a targeted portion of a RIC pre-mRNA. Typically such hybridization occurs with a Tm substantially greater than 37°C, preferably at least 50°C, and typically between 60°C to approximately 90°C. Such hybridization preferably corresponds to stringent hybridization conditions. At a given ionic strength and pH, the Tm is the temperature at which 50% of a target sequence hybridizes to a complementary oligonucleotide.

Oligomers, such as oligonucleotides, are "complementary" to one another when hybridization occurs in an antiparallel configuration between two single-stranded polynucleotides. A double-stranded polynucleotide can be "complementary" to another polynucleotide, if hybridization can occur between one of the strands of the first polynucleotide and the second. Complementarity (the degree to which one polynucleotide is complementary with another) is quantifiable in terms of the proportion (*e.g*., the percentage) of bases in opposing strands that are expected to form hydrogen bonds with each other, according to generally accepted base-pairing rules. The sequence of an antisense oligomer (ASO) need not be 100% complementary to that of its target nucleic acid to hybridize. In certain embodiments, ASOs can comprise at least 70%, at least 75%, at least 80%, at least 85%, at least 90%, at least 95%, at least 96%, at least 97%, at least 98%, or at least 99% sequence complementarity to a target region within the target nucleic acid sequence to which they are targeted. For example, an ASO in which 18 of 20 nucleobases of the oligomeric compound are complementary to a target region, and would therefore specifically hybridize, would represent 90 percent complementarity. In this example, the remaining noncomplementary nucleobases may be clustered together or interspersed with complementary nucleobases and need not be contiguous to each other or to complementary nucleobases. Percent complementarity of an ASO with a region of a target nucleic acid can be determined routinely using BLAST programs (basic local alignment search tools) and PowerBLAST programs known in the art (Altschul et al., J. Mol. Biol., 1990, 215, 403-410; Zhang and Madden, Genome Res., 1997, 7, 649-656).

An ASO need not hybridize to all nucleobases in a target sequence and the nucleobases to which it does hybridize may be contiguous or noncontiguous. ASOs may hybridize over one or more segments of a pre-mRNA transcript, such that intervening or adjacent segments are not involved in the hybridization event *(e.g.,* a loop structure or hairpin structure may be formed). In certain embodiments, an ASO hybridizes to noncontiguous nucleobases in a target pre-mRNA transcript. For example, an ASO can hybridize to nucleobases in a pre-mRNA transcript that are separated by one or more nucleobase(s) to which the ASO does not hybridize.

The ASOs described herein comprise nucleobases that are complementary to nucleobases present in a target portion of a RIC pre-mRNA. The term ASO embodies oligonucleotides and any other oligomeric molecule that comprises nucleobases capable of hybridizing to a complementary nucleobase on a target mRNA but does not comprise a sugar moiety, such as a peptide nucleic acid (PNA). The ASOs may comprise naturally-occurring nucleotides, nucleotide analogs, modified nucleotides, or any combination of two or three of the preceding. The term "naturally occurring nucleotides" includes deoxyribonucleotides and ribonucleotides. The term "modified nucleotides" includes nucleotides with modified or substituted sugar groups and/or having a modified backbone. In some embodiments, all of the nucleotides of the ASO are modied nucleotides. Chemical modifications of ASOs or components of ASOs that are compatible with the methods and compositions described herein will be evident to one of skill in the art and can be found, for example, in U.S. Patent No. 8,258,109 B2, U.S. Patent No. 5,656,612, U.S. Patent Publication No. 2012/0190728, and Dias and Stein, Mol. Cancer Ther. 2002, 1 , 347-355, herein incorporated by reference in their entirety.

The nucleobase of an ASO may be any naturally occurring, unmodified nucleobase such as adenine, guanine, cytosine, thymine and uracil, or any synthetic or modified nucleobase that is sufficiently similar to an unmodified nucleobase such that it is capable of hydrogen bonding with a nucleobase present on a target pre-mRNA. Examples of modified nucleobases include, without limitation, hypoxanthine, xanthine, 7-methylguanine, 5,6-dihydrouracil, 5-methylcytosine, and 5-hydroxymethoylcytosine.

The ASOs described herein also comprise a backbone structure that connects the components of an oligomer. The term "backbone structure" and "oligomer linkages" may be used interchangeably and refer to the connection between monomers of the ASO. In naturally occurring oligonucleotides, the backbone comprises a 3'-5' phosphodiester linkage connecting sugar moieties of the oligomer. The backbone structure or oligomer linkages of the ASOs described herein may include (but are not limited to) phosphorothioate, phosphorodithioate, phosphoroselenoate, phosphorodiselenoate, phosphoroanilothioate, phosphoraniladate, phosphoramidate, and the like. See *e.g*., LaPlanche et al. Nucleic Acids Res. 14:9081 (1986); Stec et al. J. Am. Chem. Soc. 106:6077 (1984), Stein et al. Nucleic Acids Res. 16:3209 (1988), Zon et al. Anti Cancer Drug Design 6:539 (1991); Zon et al. Oligonucleotides and Analogues: A Practical Approach, pp. 87-108 (F. Eckstein, Ed., Oxford University Press, Oxford England (1991)); Stec et al. U.S. Pat. No. 5,151,510; Uhlmann and Peyman Chemical Reviews 90:543 (1990). In some embodiments, the backbone structure of the ASO does not contain phosphorous but rather contains peptide bonds, for example in a peptide nucleic acid (PNA), or linking groups including carbamate, amides, and linear and cyclic hydrocarbon groups. In some embodiments, the backbone modification is a phosphothioate linkage. In some embodiments, the backbone modification is a phosphoramidate linkage.

Any of the ASOs described herein may contain a sugar moiety that comprises ribose or deoxyribose, as present in naturally occurring nucleotides, or a modified sugar moiety or sugar analog, including a morpholine ring. Non-limiting examples of modified sugar moieties include 2' substitutions such as 2'-O-methyl (2'-O-Me), 2'-O-methoxyethyl (2'MOE), 2'-O-aminoethyl, 2'F; N3'->P5' phosphoramidate, 2'dimethylaminooxyethoxy, 2'dimethylaminoethoxyethoxy, 2'-guanidinidium, 2'-O-guanidinium ethyl, carbamate modified sugars, and bicyclic modified sugars. In some embodiments, the sugar moiety modification is selected from 2'-O-Me, 2'F, and 2'MOE. In some embodiments, the sugar moiety modification is an extra bridge bond, such as in a locked nucleic acid (LNA). In some embodiments the sugar analog contains a morpholine ring, such as phosphorodiamidate morpholino (PMO). In some embodiments, the sugar moiety comprises a ribofuransyl or 2' deoxyribofuransyl modification. In some embodiments, the sugar moiety comprises 2'4'-constrained 2'O-methyloxyethyl (cMOE) modifications. In some embodiments, the sugar moiety comprises cEt 2', 4' constrained 2'-O ethyl BNA modifications. In some embodiments, the sugar moiety comprises tricycloDNA (tcDNA) modifications. In some embodiments, the sugar moiety comprises ethylene nucleic acid (ENA) modifications. In some embodiments, the sugar moiety comprises MCE modifications. Modifications are known in the art and described in the literature, *e.g*., by Jarver, et al., 2014, "A Chemical View of Oligonucleotides for Exon Skipping and Related Drug Applications," Nucleic Acid Therapeutics 24(1): 37-47, incorporated by reference for this purpose herein.

In some examples, each monomer of the ASO is modified in the same way, for example each linkage of the backbone of the ASO comprises a phosphorothioate linkage or each ribose sugar moiety comprises a 2'O-methyl modification. Such modifications that are present on each of the monomer components of an ASO are referred to as "uniform modifications." In some examples, a combination of different modifications may be desired, for example, an ASO may comprise a combination of phosphorodiamidate linkages and sugar moieties comprising morpholine rings (morpholinos). Combinations of different modifications to an ASO are referred to as "mixed modifications" or "mixed chemistries."

In some embodiments, the ASO comprises one or more backbone modification. In some embodiments, the ASO comprises one or more sugar moiety modification. In some embodiments, the ASO comprises one or more backbone modification and one or more sugar moiety modification. In some embodiments, the ASO comprises 2'MOE modifications and a phosphorothioate backbone. In some embodiments, the ASO comprises a phosphorodiamidate morpholino (PMO). In some embodiments, the ASO comprises a peptide nucleic acid (PNA). Any of the ASOs or any component of an ASO (*e.g.,* a nucleobase, sugar moiety, backbone) described herein may be modified in order to achieve desired properties or activities of the ASO or reduce undesired properties or activities of the ASO. For example, an ASO or one or more component of any ASO may be modified to enhance binding affinity to a target sequence on a pre-mRNA transcript; reduce binding to any non-target sequence; reduce degradation by cellular nucleases (i.e., RNase H); improve uptake of the ASO into a cell and/or into the nucleus of a cell; alter the pharmacokinetics or pharmacodynamics of the ASO; and modulate the half-life of the ASO.

In some embodiments, the ASOs are comprised of 2'-O-(2-methoxyethyl) (MOE) phosphorothioate-modified nucleotides. ASOs comprised of such nucleotides are especially well-suited to the methods disclosed herein; oligomers having such modifications have been shown to have significantly enhanced resistance to nuclease degradation and increased bioavailability, making them suitable, for example, for oral delivery in some embodiments described herein. See *e.g.,* Geary et al., J Pharmacol Exp Ther. 2001; 296(3):890-7; Geary et al., J Pharmacol Exp Ther. 2001; 296(3):898-904.

Methods of synthesizing ASOs will be known to one of skill in the art. Alternatively or in addition, ASOs may be obtained from a commercial source.

Unless specified otherwise, the left-hand end of single-stranded nucleic acid (*e.g.,* pre-mRNA transcript, oligonucleotide, ASO, etc.) sequences is the 5' end and the left-hand direction of single or double-stranded nucleic acid sequences is referred to as the 5' direction. Similarly, the right-hand end or direction of a nucleic acid sequence (single or double stranded) is the 3' end or direction. Generally, a region or sequence that is 5' to a reference point in a nucleic acid is referred to as "upstream," and a region or sequence that is 3' to a reference point in a nucleic acid is referred to as "downstream." Generally, the 5' direction or end of an mRNA is where the initiation or start codon is located, while the 3' end or direction is where the termination codon is located. In some aspects, nucleotides that are upstream of a reference point in a nucleic acid may be designated by a negative number, while nucleotides that are downstream of a reference point may be designated by a positive number. For example, a reference point (*e.g*., an exon-exon junction in mRNA) may be designated as the "zero" site, and a nucleotide that is directly adjacent and upstream of the reference point is designated "minus one," *e.g.,* "-1," while a nucleotide that is directly adjacent and downstream of the reference point is designated "plus one," *e.g.,* "+1."

In other embodiments, the ASOs are complementary to (and bind to) a targeted portion of a RIC pre-mRNA that is downstream (in the 3' direction) of the 5' splice site of the retained intron in a RIC pre-mRNA (*e.g*., the direction designated by positive numbers relative to the 5' splice site) (Figure 1). In some embodiments, the ASOs are complementary to a targeted portion of the RIC pre-mRNA that is within the region +6 to +100 relative to the 5' splice site of the retained intron. In some embodiments, the ASO is not complementary to nucleotides +1 to +5 relative to the 5' splice site (the first five nucleotides located downstream of the 5' splice site). In some embodiments, the ASOs may be complementary to a targeted portion of a RIC pre-mRNA that is within the region between nucleotides +6 and +50 relative to the 5' splice site of the retained intron. In some aspects, the ASOs are complementary to a targeted portion that is within the region +6 to +90, +6 to +80, +6 to +70, +6 to +60, +6 to +50, +6 to +40, +6 to +30, or +6 to +20 relative to 5' splice site of the retained intron.

In some embodiments, the ASOs are complementary to a targeted region of a RIC pre-mRNA that is upstream (5' relative) of the 3' splice site of the retained intron in a RIC pre-mRNA (*e*.*g*., in the direction designated by negative numbers) (Figure 1). In some embodiments, the ASOs are complementary to a targeted portion of the RIC pre-mRNA that is within the region -16 to -100 relative to the 3' splice site of the retained intron. In some embodiments, the ASO is not complementary to nucleotides -1 to -15 relative to the 3' splice site (the first 15 nucleotides located upstream of the 3' splice site). In some embodiments, the ASOs are complementary to a targeted portion of the RIC pre-mRNA that is within the region - 16 to -50 relative to the 3' splice site of the retained intron. In some aspects, the ASOs are complementary to a targeted portion that is within the region -16 to -90, -16 to -80, -16 to -70, - 16 to -60, -16 to -50, -16 to -40, or -16 to -30 relative to 3' splice site of the retained intron.

In embodiments, the targeted portion of the RIC pre-mRNA is within the region +100 relative to the 5' splice site of the retained intron to -100 relative to the 3' splice site of the retained intron.

In some embodiments, the ASOs are complementary to a targeted portion of a RIC pre-mRNA that is within the exon flanking the 5' splice site (upstream) of the retained intron (Figure 1). In some embodiments, the ASOs are complementary to a targeted portion of the RIC pre-mRNA that is within the region +2e to -4e in the exon flanking the 5' splice site of the retained intron. In some embodiments, the ASOs are not complementary to nucleotides -1e to - 3e relative to the 5' splice site of the retained intron. In some embodiments, the ASOs are complementary to a targeted portion of the RIC pre-mRNA that is within the region -4e to-100e, -4e to -90e, -4e to -80e, -4e to -70e, -4e to -60e, -4e to -50e, -4 to -40e, -4e to -30e, or -4e to -20e relative to the 5' splice site of the retained intron.

In some embodiments, the ASOs are complementary to a targeted portion of a RIC pre-mRNA that is within the exon flanking the 3' splice site (downstream) of the retained intron (Figure 1). In some embodiments, the ASOs are complementary to a targeted portion to the RIC pre-mRNA that is within the region +2e to -4e in the exon flanking the 3' splice site of the retained intron. In some embodiments, the ASOs are not complementary to nucleotide +1e relative to the 3' splice site of the retained intron. In some embodiments, the ASOs are complementary to a targeted portion of the RIC pre-mRNA that is within the region+2e to +100e, +2e to +90e, +2e to +80e, +2e to +70e, +2e to +60e, +2e to +50e, +2e to +40e, +2e to +30e, or +2 to +20e relative to the 3' splice site of the retained intron. The ASOs may be of any length suitable for specific binding and effective enhancement of splicing. In some embodiments, the ASOs consist of 8 to 50 nucleobases. For example, the ASO may be 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, or 50 nucleobases in length. In some embodiments, the ASOs consist of more than 50 nucleobases. In some embodiments, the ASO is from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, 12 to 15 nucleobases, 13 to 50 nucleobases, 13 to 40 nucleobases, 13 to 35 nucleobases, 13 to 30 nucleobases, 13 to 25 nucleobases, 13 to 20 nucleobases, 14 to 50 nucleobases, 14 to 40 nucleobases, 14 to 35 nucleobases, 14 to 30 nucleobases, 14 to 25 nucleobases, 14 to 20 nucleobases, 15 to 50 nucleobases, 15 to 40 nucleobases, 15 to 35 nucleobases, 15 to 30 nucleobases, 15 to 25 nucleobases, 15 to 20 nucleobases, 20 to 50 nucleobases, 20 to 40 nucleobases, 20 to 35 nucleobases, 20 to 30 nucleobases, 20 to 25 nucleobases, 25 to 50 nucleobases, 25 to 40 nucleobases, 25 to 35 nucleobases, or 25 to 30 nucleobases in length. In some embodiments, the ASOs are 18 nucleotides in length. In some embodiments, the ASOs are 15 nucleotides in length. In some embodiments, the ASOs are 25 nucleotides in length.

In some embodiments, two or more ASOs with different chemistries but complementary to the same targeted portion of the RIC pre-mRNA are used. In some embodiments, two or more ASOs that are complementary to different targeted portions of the RIC pre-mRNA are used.

In embodiments, the antisense oligonucleotides of the invention are chemically linked to one or more moieties or conjugates, *e.g*., a targeting moiety or other conjugate that enhances the activity or cellular uptake of the oligonucleotide. Such moieties include, but are not limited to, a lipid moiety, *e.g.,* as a cholesterol moiety, a cholesteryl moiety, an aliphatic chain, *e.g.,* dodecandiol or undecyl residues, a polyamine or a polyethylene glycol chain, or adamantane acetic acid. Oligonucleotides comprising lipophilic moieties, and preparation methods have been described in the published literature. In embodiments, the antisense oligonucleotide is conjugated with a moiety including, but not limited to, an abasic nucleotide, a polyether, a polyamine, a polyamide, a peptides, a carbohydrate, *e.g.*, N-acetylgalactosamine (GalNAc), N-Ac-Glucosamine (GluNAc), or mannose (*e.g*., mannose-6-phosphate), a lipid, or a polyhydrocarbon compound. Conjugates can be linked to one or more of any nucleotides comprising the antisense oligonucleotide at any of several positions on the sugar, base or phosphate group, as understood in the art and described in the literature, e.g., using a linker. Linkers can include a bivalent or trivalent branched linker. In embodiments, the conjugate is attached to the 3' end of the antisense oligonucleotide. Methods of preparing oligonucleotide conjugates are described, e.g., in U.S. Pat. No. 8,450,467, "Carbohydrate conjugates as delivery agents for oligonucleotides," incorporated by reference herein.

In some embodiments, the nucleic acid to be targeted by an ASO is a RIC pre-mRNA expressed in a cell, such as a eukaryotic cell. In some embodiments, the term "cell" may refer to a population of cells. In some embodiments, the cell is in a subject. In some embodiments, the cell is isolated from a subject. In some embodiments, the cell is *ex vivo.* In some embodiments, the cell is a condition or disease-relevant cell or a cell line. In some embodiments, the cell is *in vitro* (*e.g.,* in cell culture).

### Pharmaceutical Compositions

Pharmaceutical compositions or formulations comprising the antisense oligonucleotide of the described compositions and for use in any of the described methods can be prepared according to conventional techniques well known in the pharmaceutical industry and described in the published literature. In embodiments, a pharmaceutical composition or formulation for treating a subject comprises an effective amount of any antisense oligomer as described above, or a pharmaceutically acceptable salt, solvate, hydrate or ester thereof, and a pharmaceutically acceptable diluent. The antisense oligomer of a pharmaceutical formulation may further comprise a pharmaceutically acceptable excipient, diluent or carrier.

Pharmaceutically acceptable salts are suitable for use in contact with the tissues of humans and lower animals without undue toxicity, irritation, allergic response, etc., and are commensurate with a reasonable benefit/risk ratio. (See, e.g., S. M. Berge, et al., J. Pharmaceutical Sciences, 66: 1-19 (1977), incorporated herein by reference for this purpose. The salts can be prepared in situ during the final isolation and purification of the compounds, or separately by reacting the free base function with a suitable organic acid. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other documented methodologies such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

In embodiments, the compositions are formulated into any of many possible dosage forms such as, but not limited to, tablets, capsules, gel capsules, liquid syrups, soft gels, suppositories, and enemas. In embodiments, the compositions are formulated as suspensions in aqueous, non-aqueous or mixed media. Aqueous suspensions may further contain substances that increase the viscosity of the suspension including, for example, sodium carboxymethylcellulose, sorbitol and/or dextran. The suspension may also contain stabilizers. In embodiments, a pharmaceutical formulation or composition of the present invention includes, but is not limited to, a solution, emulsion, microemulsion, foam or liposome-containing formulation (e.g., cationic or noncationic liposomes).

The pharmaceutical composition or formulation of the present invention may comprise one or more penetration enhancer, carrier, excipients or other active or inactive ingredients as appropriate and well known to those of skill in the art or described in the published literature. In embodiments, liposomes also include sterically stabilized liposomes, e.g., liposomes comprising one or more specialized lipids. These specialized lipids result in liposomes with enhanced circulation lifetimes. In embodiments, a sterically stabilized liposome comprises one or more glycolipids or is derivatized with one or more hydrophilic polymers, such as a polyethylene glycol (PEG) moiety. In embodiments, a surfactant is included in the pharmaceutical formulation or compositions. The use of surfactants in drug products, formulations and emulsions is well known in the art. In embodiments, the present invention employs a penetration enhancer to effect the efficient delivery of the antisense oligonucleotide, e.g., to aid diffusion across cell membranes and /or enhance the permeability of a lipophilic drug. In embodiments, the penetration enhancers is a surfactant, fatty acid, bile salt, chelating agent, or non-chelating nonsurfactant.

In embodiments, the pharmaceutical formulation comprises multiple antisense oligonucleotides. In embodiments, the antisense oligonucleotide is administered in combination with another drug or therapeutic agent. In embodiments, the antisense oligonucleotide is administered with one or more agents capable of promoting penetration of the subject antisense oligonucleotide across the blood-brain barrier by any method known in the art. For example, delivery of agents by administration of an adenovirus vector to motor neurons in muscle tissue is described in U.S. Pat. No. 6,632,427, "Adenoviral-vector-mediated gene transfer into medullary motor neurons," incorporated herein by reference. Delivery of vectors directly to the brain, e.g., the striatum, the thalamus, the hippocampus, or the substantia nigra, is described, e.g., in U.S. Pat. No. 6,756,523, "Adenovirus vectors for the transfer of foreign genes into cells of the central nervous system particularly in brain," incorporated herein by reference.

In embodiments, the antisense oligonucleotides are linked or conjugated with agents that provide desirable pharmaceutical or pharmacodynamic properties. In embodiments, the antisense oligonucleotide is coupled to a substance, known in the art to promote penetration or transport across the blood-brain barrier, e.g., an antibody to the transferrin receptor. In embodiments, the antisense oligonucleotide is linked with a viral vector, e.g., to render the antisense compound more effective or increase transport across the blood-brain barrier. In embodiments, osmotic blood brain barrier disruption is assisted by infusion of sugars, e.g.,, meso erythritol, xylitol, D(+) galactose, D(+) lactose, D(+) xylose, dulcitol, myo-inositol, L(-) fructose, D(-) mannitol, D(+) glucose, D(+) arabinose, D(-) arabinose, cellobiose, D(+) maltose, D(+) raffinose, L(+) rhamnose, D(+) melibiose, D(-) ribose, adonitol, D(+) arabitol, L(-) arabitol, D(+) fucose, L(-) fucose, D(-) lyxose, L(+) lyxose, and L(-) lyxose, or amino acids, e.g., glutamine, lysine, arginine, asparagine, aspartic acid, cysteine, glutamic acid, glycine, histidine, leucine, methionine, phenylalanine, proline, serine, threonine, tyrosine, valine, and taurine. Methods and materials for enhancing blood brain barrier penetration are described, e.g., in U.S. Pat. No. 4,866,042, "Method for the delivery of genetic material across the blood brain barrier," U.S. Pat. No. 6,294,520, "Material for passage through the blood-brain barrier," and U.S. Pat. No. 6,936,589, "Parenteral delivery systems," each incorporated herein by reference.

In embodiments, the antisense oligonucleotides of the invention are chemically linked to one or more moieties or conjugates, e.g., a targeting moiety or other conjugate that enhances the activity or cellular uptake of the oligonucleotide. Such moieties include, but are not limited to, a lipid moiety, e.g., as a cholesterol moiety, a cholesteryl moiety, an aliphatic chain, e.g., dodecandiol or undecyl residues, a polyamine or a polyethylene glycol chain, or adamantane acetic acid. Oligonucleotides comprising lipophilic moieties, and preparation methods have been described in the published literature. In embodiments, the antisense oligonucleotide is conjugated with a moiety including, but not limited to, an abasic nucleotide, a polyether, a polyamine, a polyamide, a peptides, a carbohydrate, e.g., N-acetylgalactosamine (GalNAc), N-Ac-Glucosamine (GluNAc), or mannose (e.g., mannose-6-phosphate), a lipid, or a polyhydrocarbon compound. Conjugates can be linked to one or more of any nucleotides comprising the antisense oligonucleotide at any of several positions on the sugar, base or phosphate group, as understood in the art and described in the literature, e.g., using a linker. Linkers can include a bivalent or trivalent branched linker. In embodiments, the conjugate is attached to the 3' end of the antisense oligonucleotide. Methods of preparing oligonucleotide conjugates are described, *e.g*., in U.S. Pat. No. 8,450,467, "Carbohydrate conjugates as delivery agents for oligonucleotides," incorporated by reference herein.

### Diseases and disorders

Any condition, *e.g*., disease or disorder, that is associated with reduced production or activity of a protein or functional RNA encoded by a pre-mRNA that comprises at least one intron (*e.g.*, 1, 2, 3, 4,5, 6, 7, 8, 9, 10, or more introns) can be treated by the methods and compositions provided herein. The disease or disorder to be treated may be a result of haploinsufficiency in which one allele of a gene encodes a functional (wild-type) protein and one allele of the gene is mutated and encodes a nonfunctional protein or a protein with reduced/partial function. Other diseases or disorders may be due to hemizygous deletions in which one allele of a gene is lost and the amount of protein produced by the other allele of the gene is not sufficient. Yet other diseases or disorder maybe due to hypomorphic mutations in which the gene encoding a protein is mutated resulting in production of a protein with partial function.

In some embodiments, the methods described herein are used to increase the production of a functional protein. As used herein, the term "functional" refers to the amount of activity or function of a protein that is necessary to eliminate any one or more symptoms of a disease. In some embodiments, the methods are used to increase the production of a partially functional protein or RNA. As used herein, the term "partially functional" refers to any amount of activity or function of a protein or RNA that is less than the amount of activity or function that is necessary to eliminate or prevent any one or more symptoms of a disease. In some embodiments, a partially functional protein or RNA will have at least 10%, at least 20%, at least 30%, at least 40%, at least 50%, at least 60%, at least 70%, at least 75%, at least 80%, 85%, at least 90%, or at least 95% less activity relative to the fully functional protein or RNA.

In embodiments, the method is a method of increasing the expression of a target protein or functional RNA by cells of a subject having a RIC pre-mRNA encoding the target protein or functional RNA, wherein the subject has a condition caused by a deficient amount of activity of the target protein or functional RNA, and wherein the deficient amount of the target protein or functional RNA is caused by haploinsufficiency of the target protein or functional RNA. In such an embodiment, the subject has a first allele encoding a functional target protein or functional functional RNA, and a second allele from which the target protein or functional RNA is not produced. In another such embodiment, the subject has a first allele encoding a functional target protein or functional functional RNA, and a second allele encoding a nonfunctional target protein or nonfunctional functional RNA. In either of these embodiments, the antisense oligomer binds to a targeted portion of the RIC pre-mRNA transcribed from the first allele (encoding functional target protein), thereby inducing constitutive splicing of the retained intron from the RIC pre-mRNA, and causing an increase in the level of mRNA encoding the target protein or functional RNA, and an increase in the expression of the target protein or functional RNA in the cells of the subject.

In related embodiments, the method is a method of increasing the expression of a target protein or functional RNA by cells of a subject having a RIC pre-mRNA encoding the target protein or functional RNA, wherein the subject has a condition caused by an autosomal recessive disorder resulting from a deficiency in the amount or function of the target protein or functional RNA. In these embodiments, the subject has:
a. a first mutant allele from which
   i) the target protein or functional RNA is produced at a reduced level compared to production from a wild-type allele,
   ii) the target protein or functional RNA is produced in a form having reduced function compared to an equivalent wild-type protein, or
   iii) the target protein or functional RNA is not produced; and
b.a second mutant allele from which
   i) the target protein or functional RNA is produced at a reduced level compared to production from a wild-type allele,
   ii) the target protein or functional RNA is produced in a form having reduced function compared to an equivalent wild-type protein, or
   iii) the target protein or functional RNA is not produced, and
   wherein the RIC pre-mRNA is transcribed from the first allele and/or the second allele. In these embodiments, the antisense oligomer binds to a targeted portion of the RIC pre-mRNA transcribed from the first allele or the second allele, thereby inducing constitutive splicing of the retained intron from the RIC pre-mRNA, and causing an increase in the level of mRNA encoding the target protein or functional RNA and an increase in the expression of the target protein or functional RNA in the cells of the subject. In these embodiments, the target protein or functional RNA having an increase in expression level resulting from the constitutive splicing of the retained intron from the RIC pre-mRNA is either in a form having reduced function compared to the equivalent wild-type protein (partially-functional), or having full function compared to the equivalent wild-type protein (fully-functional).

In embodiments, the level of mRNA encoding the target protein, the target protein or the functional RNA is increased 1.1 to 10-fold, as set forth elsewhere herein, when compared to the amount of mRNA encoding the target protein, the target protein or the functional RNA produced in a control cell, *e.g.,* one that is not treated with the antisense oligomer or one that is treated with an antisense oligomer that does not bind to the targeted portion of the RIC pre-mRNA.

In embodiments, the condition caused by a deficient amount or activity of the target protein or a deficient amount or activity of the functional RNA is not a condition caused by alternative or aberrant splicing of the retained intron to which the ASO is targeted. In embodiments, the condition caused by a deficient amount or activity of the target protein or a deficient amount or activity of the functional RNA is not a condition caused by alternative or aberrant splicing of any retained intron in a RIC pre-mRNA encoding the target protein or functional RNA.

Table 1 provides examples of diseases and target genes associated with each disease that may be treatable using the methods and compositions provided herein.

**Table 1**

| **DISEASE** | **TARGET GENE** | **NUMBER OF POTENTIAL INTRON TARGETS** |
|---|---|---|
| Retinitis pigmentosa type 11 | PRPF31 | 2 |
| Retinoblastoma | RB1 | 1 |
| Beta thalassemia (BTI) | HBB | 1 |
| Beta thalassemia | HBG1/2 | 2 |
| Sickle cell disease | HBG1/2 | 2 |
| Cystic fibrosis | CFTR | 26 |
| Thrombotic thrombocytopenic purpura | ADAMTS13 | 2 |
| Tuberous sclerosis complex | TSC1 | 3 |
| Retinitis pigmentosa 10 | IMPDH1 | 1 |
| Polycystic kidney disease | PKD1 | 4 |
| Familial dysautonomia | IKBKAP | 2 |

In some embodiments, the pre-mRNA transcript that encodes the protein that is causative of the disease is targeted by the ASOs described herein. In some embodiments, a pre-mRNA transcript that encodes a protein is not causative of the disease is targeted by the ASOs. For example, a disease that is the result of a mutation or deficiency of a first protein in a particular pathway may be ameliorated by targeting a pre-mRNA that encodes a second protein, thereby increasing production of the second protein. In some embodiments, the function of the second protein is able to compensate for the mutation or deficiency of the first protein.

Any of the compositions provided herein may be administered to an individual. "Individual" maybe used interchangeably with "subject" or "patient." An individual may be a mammal, for example a human or animal such as a non-human primate, a rodent, a rabbit, a rat, a mouse, a horse, a donkey, a goat, a cat, a dog, a cow, a pig, or a sheep. In some embodiments, the individual is a human. In other embodiments, the individual may be another eukaryotic organism, such as a plant. In some embodiments, the compositions provided herein are administered to a cell *ex vivo.*

In some embodiments, the compositions provided herein are administered to an individual as a method of treating a disease or disorder. In some embodiments, the individual has a genetic disease, such as any of the diseases described herein. In some embodiments, the individual is at risk of having the disease, such as any of the diseases described herein. In some embodiments, the individual is at increased risk of having a disease or disorder caused by insufficient amount of a protein or insufficient activity of a protein. If an individual is "at an increased risk" of having a disease or disorder caused insufficient amount of a protein or insufficient activity of a protein, the method involves preventative or prophylactic treatment. For example, an individual may be at an increased risk of having such a disease or disorder because of family history of the disease. Typically, individuals at an increased risk of having such a disease or disorder benefit from prophylactic treatment (e.g., by preventing or delaying the onset or progression of the disease or disorder).

Table 2 provides a non-limiting list of sequences of ASOs for increasing production of a protein encoded by the *HBB* gene by targeting a region of a RIC pre-mRNA transcribed from the *HBB* gene.

**Table 2. List of ASOs targeting the HBB gene**

| ASO | Sequence | SEQ ID NO |
|---|---|---|
| Non-targeting | CCAGTGGTATTGCTTACC | 103 |
| HBBIVS1+6 | ctgtcttgtaaccttgat | 104 |
| HBBIVS1+7 | cctgtcttgtaaccttga | 105 |
| HBBIVS1+8 | acctgtcttgtaaccttg | 106 |
| HBBIVS1+9 | aacctgtcttgtaacctt | 107 |
| HBBIVS1+10 | aaacctgtcttgtaacct | 108 |
| HBBIVS1+11 | taaacctgtcttgtaacc | 109 |
| HBBIVS1+12 | ttaaacctgtcttgtaac | 110 |
| HBBIVS1+13 | cttaaacctgtcttgtaa | 111 |
| HBBIVS1+14 | ccttaaacctgtcttgta | 112 |
| HBBIVS1+15 | tccttaaacctgtcttgt | 113 |
| HBBIVS1+16 | ctccttaaacctgtcttg | 114 |
| HBBIVS1+17 | tctccttaaacctgtctt | 115 |
| HBBIVS1+18 | gtctccttaaacctgtct | 116 |
| HBBIVS1+19 | ggtctccttaaacctgtc | 117 |
| HBBIVS1+20 | tggtctccttaaacctgt | 118 |
| HBBIVS1+21 | ttggtctccttaaacctg | 119 |
| HBBIVS1+22 | attggtctccttaaacct | 120 |
| HBBIVS1+23 | tattggtctccttaaacc | 121 |
| HBBIVS1+24 | ctattggtctccttaaac | 122 |
| HBBIVS1+25 | tctattggtctccttaaa | 123 |
| HBBIVS1+26 | ttctattggtctccttaa | 124 |
| HBBIVS1+27 | tttctattggtctcctta | 125 |
| HBBIVS1+28 | gtttctattggtctcctt | 126 |

Table 3 provides a non-limiting list of sequences of ASOs for increasing production of a protein encoded by the *PRPF31* gene by targeting a region of a RIC pre-mRNA transcribed from the PRPF31 gene.

**Table 3. List of ASOs targeting the PRPF31 gene**

| ASO | Sequence | SEQ ID NO |
|---|---|---|
| P31-IVS10+6 | accggacccccagggccc | 127 |
| P31-IVS10+11 | tgcctaccggacccccag | 128 |
| P31-IVS10+16 | ccccatgcctaccggacc | 129 |
| P31-IVS10+21 | atgacccccatgcctacc | 130 |
| P31-IVS10+26 | cctccatgacccccatgc | 131 |
| P31-IVS10+31 | tctcccctccatgacccc | 132 |
| P31-IVS10-41 | gaggaggacgccggcttc | 133 |
| P31-IVS10-36 | gctgggaggaggacgccg | 134 |
| P31-IVS10-31 | agtcggctgggaggagga | 135 |
| P31-IVS10-26 | cagggagtcggctgggag | 136 |
| P31-IVS10-21 | ggcgccagggagtcggct | 137 |
| P31-IVS10-16 | tgggcggcgccagggagt | 138 |
| P31-IVS12+6 | ccccacctgggtctggcc | 139 |
| P31-IVS12+11 | cccagccccacctgggtc | 140 |
| P31-IVS12+16 | cggtccccagccccacct | 141 |
| P31-IVS12+21 | tccctcggtccccagccc | 142 |
| P31-IVS12-16 | ggaggctgcgatctgggc | 143 |
| P31-IVS12-21 | ctgcgatctgggctcccc | 144 |
| P31-IVS12-26 | atctgggctccccccacc | 145 |
| P31-IVS12-31 | ggctccccccaccttgtg | 146 |
| P31-IVS12+26 | ttgtgtccctcggtcccc | 147 |
| P31-IVS12+31 | ccaccttgtgtccctcgg | 148 |
| P31-IVS12+36 | tccccccaccttgtgtcc | 149 |

Table 4 provides a non-limiting list of sequences of ASOs for increasing production of a protein encoded by the *ADAMTS13* gene by targeting a region of a RIC pre-mRNA transcribed from the *ADAMTS13* gene.

**Table 4. List of ASOs targeting the ADAMTS13 gene**

| ASO | Sequence | SEQ ID NO |
|---|---|---|
| ADAM-IVS25+6 | caggaaggaggacaggac | 150 |
| ADAM-IVS25+11 | ccugacaggaaggaggac | 151 |
| ADAM-IVS25+16 | agcugccugacaggaagg | 152 |
| ADAM-IVS25+21 | gcagcagcugccugacag | 153 |
| ADAM-IVS25+26 | cuccugcagcagcugccu | 154 |
| ADAM-IVS25+31 | caccccuccugcagcagc | 155 |
| ADAM-IVS25+36 | uugcccaccccuccugca | 156 |
| ADAM-IVS25+41 | ugccuuugcccaccccuc | 157 |
| ADAM-IVS25+46 | gaagaugccuuugcccac | 158 |
| ADAM-IVS25-16 | gagacagguaagcagugc | 159 |
| ADAM-IVS25-21 | agguaagcagugcuuccc | 160 |
| ADAM-IVS25-26 | agcagugcuuccccgauu | 161 |
| ADAM-IVS25-31 | ugcuuccccgauucccag | 162 |
| ADAM-IVS25-36 | ccccgauucccagcaggg | 163 |
| ADAM-IVS25-41 | auucccagcagggcaggc | 164 |
| ADAM-IVS25-46 | cagcagggcaggcuccgg | 165 |
| ADAM-IVS25-47 | agcagggcaggcuccggg | 166 |
| ADAM-IVS25-62 | gggcuuccaagcugagga | 167 |
| ADAM-IVS27+6 | agguggagaaggccuggc | 168 |
| ADAM-IVS27+11 | aagggagguggagaaggc | 169 |
| ADAM-IVS27+16 | cacccaagggagguggag | 170 |
| ADAM-IVS27+21 | uggagcacccaagggagg | 171 |
| ADAM-IVS27+26 | aggacuggagcacccaag | 172 |
| ADAM-IVS27+31 | cugccaggacuggagcac | 173 |
| ADAM-IVS27+36 | ccucccugccaggacugg | 174 |
| ADAM-IVS27+41 | cccagccucccugccagg | 175 |
| ADAM-IVS27-16 | agggacauaggaacccag | 176 |
| ADAM-IVS27-21 | cauaggaacccagacaga | 177 |
| ADAM-IVS27-26 | gaacccagacagaccggu | 178 |
| ADAM-IVS27-31 | cagacagaccgguggugc | 179 |
| ADAM-IVS27-36 | agaccgguggugccagag | 180 |
| ADAM-IVS27-41 | gguggugccagaggccag | 181 |
| ADAM-IVS27-46 | ugccagaggccaggacaa | 182 |
| ADAM-IVS27-51 | gaggccaggacaacucac | 183 |
| ADAM-IVS25+17 | cagcugccugacaggaag | 184 |
| ADAM-IVS25+18 | gcagcugccugacaggaa | 185 |
| ADAM-IVS25+19 | agcagcugccugacagga | 186 |
| ADAM-IVS25+20 | cagcagcugccugacagg | 187 |
| ADAM-IVS25+21a | gcagcagcugccugacag | 188 |
| ADAM-IVS25+22 | ugcagcagcugccugaca | 189 |
| ADAM-IVS25+23 | cugcagcagcugccugac | 190 |
| ADAM-IVS25+24 | ccugcagcagcugccuga | 191 |
| ADAM-IVS25+25 | uccugcagcagcugccug | 192 |
| ADAM-IVS25+26a | cuccugcagcagcugccu | 193 |
| ADAM-TIVS25+27 | ccuccugcagcagcugcc | 194 |
| ADAM-IVS25+28 | cccuccugcagcagcugc | 195 |
| ADAM-IVS25+29 | ccccuccugcagcagcug | 196 |
| ADAM-IVS25+30 | accccuccugcagcagcu | 197 |

Table 5 provides a non-limiting list of sequences of ASOs for increasing production of a protein encoded by the *TSC1* gene by targeting a region of a RIC pre-mRNA transcribed from the *TSC1* gene.

**Table 5. List of ASOs targeting the TSC1 gene**

| ASO | Sequence | SEQ ID NO |
|---|---|---|
| TSC1-IVS5+6 | ucaaauccuuacaaacau | 198 |
| TSC1-IVS5+11 | uucauucaaauccuuaca | 199 |
| TSC1-IVS5+16 | accauuucauucaaaucc | 200 |
| TSC1-IVS5+21 | auaaaaccauuucauuca | 201 |
| TSC1-IVS5+26 | uacucauaaaaccauuuc | 202 |
| TSC1-IVS5+31 | aacuauacucauaaaacc | 203 |
| TSC1-IVS5+36 | ucagaaacuauacucaua | 204 |
| TSC1-IVS5+41 | aaauuucagaaacuauac | 205 |
| TSC1-IVS5-16 | ucaaacaggaaacgucug | 206 |
| TSC1-IVS5-21 | caggaaacgucugucagg | 207 |
| TSC1-IVS5-26 | aacgucugucaggcacug | 208 |
| TSC1-IVS5-31 | cugucaggcacuggcacc | 209 |
| TSC1-IVS5-36 | aggcacuggcaccaggau | 210 |
| TSC1-IVS5-41 | cuggcaccaggaucggca | 211 |
| TSC1-IVS5-46 | accaggaucggcauugua | 212 |
| TSC1-IVS5-51 | gaucggcauuguacagua | 213 |
| TSC1-IVS10+6 | aggcacacuaguugacac | 214 |
| TSC1-IVS10+11 | agagcaggcacacuaguu | 215 |
| TSC1-IVS10+16 | aggagagagcaggcacac | 216 |
| TSC1-IVS10+21 | agcagaggagagagcagg | 217 |
| TSC1-IVS10+26 | cagaaagcagaggagaga | 218 |
| TSC1-IVS10+31 | uucaccagaaagcagagg | 219 |
| TSC1-IVS10+36 | ucagcuucaccagaaagc | 220 |
| TSC1-IVS10+41 | aagggucagcuucaccag | 221 |
| TSC1-IVS10-16 | aguacaucagcaguggca | 222 |
| TSC1-IVS10-21 | aucagcaguggcaaagga | 223 |
| TSC1-IVS10-26 | caguggcaaaggaaugcu | 224 |
| TSC1-IVS10-31 | gcaaaggaaugcuaaguc | 225 |
| TSC1-IVS10-36 | ggaaugcuaagucaucca | 226 |
| TSC1-IVS10-41 | gcuaagucauccacgagg | 227 |
| TSC1-IVS10-46 | gucauccacgagguuuau | 228 |
| TSC1-IVS10-51 | ccacgagguuuauaucca | 229 |
| TSC1-IVS11+6 | aauccaaccuaagacaua | 230 |
| TSC1-IVS11+11 | aaucaaauccaaccuaag | 231 |
| TSC1-IVS11+16 | caacuaaucaaauccaac | 232 |
| TSC1-IVS11+21 | aaaaccaacuaaucaaau | 233 |
| TSC1-IVS11+26 | aggccaaaaccaacuaau | 234 |
| TSC1-IVS11+31 | aaggcaggccaaaaccaa | 235 |
| TSC1-IVS11+36 | cauuaaaggcaggccaaa | 236 |
| TSC1-IVS11+41 | ccugccauuaaaggcagg | 237 |
| TSC1-IVS11-16 | agaacauauaugaacacu | 238 |
| TSC1-IVS11-21 | auauaugaacacugagcc | 239 |
| TSC1-IVS11-26 | ugaacacugagcccaacu | 240 |
| TSC1-IVS11-31 | acugagcccaacuauuag | 241 |
| TSC1-IVS11-36 | gcccaacuauuagaaaaa | 242 |
| TSC1-IVS11-41 | acuauuagaaaaacugcc | 243 |
| TSC1-IVS11-46 | uagaaaaacugccgauuu | 244 |
| TSC1-IVS11-51 | aaacugccgauuuuuuuu | 245 |

Table 6 provides a non-limiting list of sequences of ASOs for increasing production of a protein encoded by the *IMPDH1* gene by targeting a region of a RIC pre-mRNA transcribed from the *IMPDH1* gene.

**Table 6. List of ASOs targeting the IMPDH1 gene**

| ASO | Sequence | SEQ ID NO |
|---|---|---|
| IMP-IVS14+6 | gggcccagggucag | 246 |
| IMP-IVS14+18 | cugaucugcccagguggg | 247 |
| IMP-IVS14+23 | gugggcugaucugcccag | 248 |
| IMP-IVS14+28 | ggguugugggcugaucug | 249 |
| IMP-IVS14+33 | cugaaggguugugggcug | 250 |
| IMP-IVS14+38 | gggcccugaaggguugug | 251 |
| IMP-IVS14+43 | ugagcgggcccugaaggg | 252 |
| IMP-IVS14+48 | uggcaugagcgggcccug | 253 |
| IMP-IVS14-16 | aagacugagcccagcagc | 254 |
| IMP-IVS14-21 | ugagcccagcagcuugaa | 255 |
| IMP-IVS14-26 | ccagcagcuugaagcuca | 256 |
| IMP-IVS14-31 | agcuugaagcucagagga | 257 |
| IMP-IVS14-36 | gaagcucagaggacccca | 258 |
| IMP-IVS14-41 | ucagaggaccccacccca | 259 |
| IMP-IVS14-46 | ggaccccaccccaccucu | 260 |
| IMP-IVS14-51 | ccaccccaccucuuaagg | 261 |
| IMP-IVS14+44 | augagcgggcccugaagg | 262 |
| IMP-IVS14+45 | caugagcgggcccugaag | 263 |
| IMP-IVS14+46 | gcaugagcgggcccugaa | 264 |
| IMP-IVS14+47 | ggcaugagcgggcccuga | 265 |
| IMP-IVS14+48a | uggcaugagcgggcccug | 266 |
| IMP-IVS14+49 | guggcaugagcgggcccu | 267 |
| IMP-IVS14+50 | gguggcaugagcgggccc | 268 |
| IMP-IVS14+51 | cgguggcaugagcgggcc | 269 |
| IMP-IVS14+52 | ucgguggcaugagcgggc | 270 |
| IMP-IVS14+53 | gucgguggcaugagcggg | 271 |

Table 7 provides a non-limiting list of sequences of ASOs for increasing production of a protein encoded by the *PKD1* gene by targeting a region of a RIC pre-mRNA transcribed from the *PKD1* gene.

**Table 7. List of ASOs targeting the PKD1 gene**

| ASO | Sequence | SEQ ID NO |
|---|---|---|
| PKD1-IVS32+6 | cgagguuucucuagggaa | 272 |
| PKD1-IVS32+11 | gggcucgagguuucucua | 273 |
| PKD1-IVS32+16 | caccagggcucgagguuu | 274 |
| PKD1-IVS32+21 | accugcaccagggcucga | 275 |
| PKD1-IVS32+26 | cagugaccugcaccaggg | 276 |
| PKD1-IVS32+31 | agacacagugaccugcac | 277 |
| PKD1-IVS32+36 | accccagacacagugacc | 278 |
| PKD1-IVS32+41 | ccggcaccccagacacag | 279 |
| PKD1-IVS32-16 | gucagcaagguaccaggg | 280 |
| PKD1-IVS32-32 | gggaugugucacacacac | 281 |
| PKD1-IVS32-37 | gugucacacacacagccc | 282 |
| PKD1-IVS32-42 | acacacacagcccacccc | 283 |
| PKD1-IVS32-47 | cacagcccacccccgucc | 284 |
| PKD1-IVS32-52 | cccacccccguccaguca | 285 |
| PKD1-IVS32-57 | ccccguccagucacgcac | 286 |
| PKD1-IVS32-62 | uccagucacgcacggaca | 287 |
| PKD1-IVS33+6 | ccccuccucucaccccag | 288 |
| PKD1-IVS33+11 | agagcccccuccucucac | 289 |
| PKD1-IVS33+16 | gcuucagagcccccuccu | 290 |
| PKD1-IVS33+21 | ggugagcuucagagcccc | 291 |
| PKD1-IVS33+26 | gcaagggugagcuucaga | 292 |
| PKD1-IVS33-31 | cagcugcaagggugagcu | 293 |
| PKD1-IVS33-26 | gggcccagcugcaagggu | 294 |
| PKD1-IVS33-21 | agggugggcccagcugca | 295 |
| PKD1-IVS33-16 | gcauagggugggcccagc | 296 |
| PKD1-IVS37+6 | gcacaggccgcacccagg | 297 |
| PKD1-IVS37+8 | gggcacaggccgcaccca | 298 |
| PKD1-IVS37+24 | gagacggagguggcaggg | 299 |
| PKD1-IVS37+29 | gacaagagacggaggugg | 300 |
| PKD1-IVS37+34 | ugggagacaagagacgga | 301 |
| PKD1-IVS37+39 | ggaggugggagacaagag | 302 |
| PKD1-IVS37+44 | gggugggaggugggagac | 303 |
| PKD1-IVS37+49 | ugcaugggugggaggugg | 304 |
| PKD1-IVS37-16 | gcccuguggucagccugg | 305 |
| PKD1-IVS37-21 | guggucagccuggcccca | 306 |
| PKD1-IVS37-26 | cagccuggccccagccca | 307 |
| PKD1-IVS37-31 | uggccccagcccacagug | 308 |
| PKD1-IVS37-36 | ccagcccacagugacagc | 309 |
| PKD1-IVS37-41 | ccacagugacagcagggc | 310 |
| PKD1-IVS37-46 | gugacagcagggcuuugg | 311 |
| PKD1-IVS37-51 | agcagggcuuuggcaacg | 312 |
| PKD1-IVS38+6 | accagugcaccggaugcc | 313 |
| PKD1-IVS38+11 | gacagaccagugcaccgg | 314 |
| PKD1-IVS38+16 | cagaagacagaccagugc | 315 |
| PKD1-IVS38+21 | aagcccagaagacagacc | 316 |
| PKD1-IVS38+26 | aacuaaagcccagaagac | 317 |
| PKD1-IVS38+31 | ggcaaaacuaaagcccag | 318 |
| PKD1-IVS38+36 | cuaaaggcaaaacuaaag | 319 |
| PKD1-IVS38+41 | cuggacuaaaggcaaaac | 320 |
| PKD1-IVS38-16 | ucacacgcuccagccccu | 321 |
| PKD1-IVS38-21 | cgcuccagccccuacugc | 322 |
| PKD1-IVS38-26 | cagccccuacugccccau | 323 |
| PKD1-IVS38-31 | ccuacugccccaugcccg | 324 |
| PKD1-IVS38-36 | ugccccaugcccgccucg | 325 |
| PKD1-IVS38-41 | caugcccgccucgaguga | 326 |
| PKD1-IVS38-46 | ccgccucgagugagcggc | 327 |
| PKD1-IVS38-51 | ucgagugagcggccacca | 328 |

Table 8 provides a non-limiting list of sequences of ASOs for increasing production of a protein encoded by the *IKBKAP* gene by targeting a region of a RIC pre-mRNA transcribed from the *IKBKAP* gene.

**Table 8. List of ASOs targeting the IKBKAP gene**

| ASO | Sequence | SEQ ID NO |
|---|---|---|
| IKB-IVS7+6 | uuaacugcaauauauuuc | 329 |
| IKB-IVS7+11 | guuguuuaacugcaauau | 330 |
| IKB-IVS7+16 | uuauuguuguuuaacugc | 331 |
| IKB-IVS7+21 | auuuuuuauuguuguuua | 332 |
| IKB-IVS7+26 | uaaaaauuuuuuauuguu | 333 |
| IKB-IVS7+31 | uaagauaaaaauuuuuua | 334 |
| IKB-IVS7+36 | uuuaauaagauaaaaauu | 335 |
| IKB-IVS7+41 | uuaauuuuaauaagauaa | 336 |
| IKB-IVS7-16 | gucaaacacacauacaca | 337 |
| IKB-IVS7-21 | acacacauacacacuuaa | 338 |
| IKB-IVS7-26 | cauacacacuuaaaacau | 339 |
| IKB-IVS7-31 | acacuuaaaacauuauga | 340 |
| IKB-IVS7-36 | uaaaacauuaugauaaaa | 341 |
| IKB-IVS7-41 | cauuaugauaaaaguugu | 342 |
| IKB-IVS7-46 | ugauaaaaguugucaauu | 343 |
| IKB-IVS7-51 | aaaguugucaauucagaa | 344 |
| IKB-IVS8+6 | cuaagguuucuucuccca | 345 |
| IKB-IVS8+11 | uuucucuaagguuucuuc | 346 |
| IKB-IVS8+16 | aagaauuucucuaagguu | 347 |
| IKB-IVS8+21 | guuccaagaauuucucua | 348 |
| IKB-IVS8+26 | cucugguuccaagaauuu | 349 |
| IKB-IVS8+31 | cucuacucugguuccaag | 350 |
| IKB-IVS8+36 | accaccucuacucugguu | 351 |
| IKB-IVS8+41 | guaccaccaccucuacuc | 352 |
| IKB-IVS8-16 | gaguguuacaauaucgaa | 353 |
| IKB-IVS8-21 | uuacaauaucgaaagcuc | 354 |
| IKB-IVS8-26 | auaucgaaagcucaccua | 355 |
| IKB-IVS8-31 | gaaagcucaccuaacuaa | 356 |
| IKB-IVS8-36 | cucaccuaacuaaagaau | 357 |
| IKB-IVS8-41 | cuaacuaaagaauagaua | 358 |
| IKB-IVS8-46 | uaaagaauagauaaaauc | 359 |
| IKB-IVS8-51 | aauagauaaaauccagaa | 360 |
| IKB-IVS7+22M | aauuuuuuauuguuguuu | 361 |
| IKB-IVS7+23M | aaauuuuuuauuguuguu | 362 |
| IKB-IVS7+24M | aaaauuuuuuauuguugu | 363 |
| IKB-IVS7+25M | aaaaauuuuuuauuguug | 364 |
| IKB-IVS7+26M | uaaaaauuuuuuauuguu | 365 |
| IKB-IVS7+27M | auaaaaauuuuuuauugu | 366 |
| IKB-IVS7+28M | gauaaaaauuuuuuauug | 367 |
| IKB-IVS7+29M | agauaaaaauuuuuuauu | 368 |
| IKB-IVS7+30M | aagauaaaaauuuuuuau | 369 |
| IKB-IVS8-16M | gaguguuacaauaucgaa | 370 |
| IKB-IVS8-17M | aguguuacaauaucgaaa | 371 |
| IKB-IVS8-18M | guguuacaauaucgaaag | 372 |
| IKB-IVS8-19M | uguuacaauaucgaaagc | 373 |
| IKB-IVS8-20M | guuacaauaucgaaagcu | 374 |

### Methods of identifying a retained intron

Also within the scope of the present disclosure are methods of identifying (determining) a retained intron in a pre-mRNA transcript while an adjacent (upstream or downstream) intron is spliced out of the pre-mRNA in a cell. In one example, the extent of splicing and joining of the exons and *removal* of each intron from a target gene can be measured by the following method. It will be appreciated by one of skill in the art that any method may be used to determine whether an intron is retained in a pre-mRNA transcript relative to an adjacent intron that is spliced out of the pre-mRNA transcript and whether a target intron is retained to greater extent relative to one or more other introns within the pre-mRNA encoded by the same gene.

### I. Screening for retained introns

A first round of screening for intron retention can be performed using nuclear RNA isolated from cells or tissues (e.g., disease-relevant cells) and analyzed by reverse transcriptase-PCR (RT-PCR), for example, investigating a pre-RNA encoded by a target gene. A target gene may be any gene that contains at least one intron and encodes a protein or a functional RNA that is associated with a disease or disorder or suspected of being associated or causative of a disease or disorder. For RT-PCR analysis, each intron is assessed for retention in the pre-mRNA encoded by a gene by designing a series of primer pairs in which one of the primers of the pair is specific to a region of an intron of the target pre-mRNA and the other primer of the pair is specific to a region of an exon that is two exons upstream or downstream of the intron (Figure 3). In some embodiments, the upstream or forward primer may be complementary and hybridize to a region within an intron, for example the intron between exons 1 and 2 in Figure 3; and the downstream or reverse primer may be complementary and hybridize to a region within an exon that is located two exons away from the intron that is being assess, for example within exon 3 as shown in Figure 3. Alternatively, the upstream or forward primer may be complementary and hybridize to a region within an exon, for example in exon 2 in Figure 3; and the downstream or reverse primer may be complementary and hybridize to a region within an intron that is two exons away from the forward primer, for example within the intron between exons 3 and 4 as shown in Figure 3. Design of primer pairs may be repeated for each of the introns encoded by the gene.

Following RT-PCR using each of the primer pairs, the RT-PCR products are analyzed by any method known in the art, for example, separation and visualization in an agarose gel. The approximate size of the RT-PCR product that is expected if the target intron is present may be estimated based on the nucleic acid sequence of the gene and/or pre-mRNA. The absence of a product from the RT-PCR analysis indicates that the target intron was not present and was removed/spliced from the pre-mRNA, and therefore under the conditions tested, is not a retained intron. The presence of a product from the RT-PCR reaction that is of approximately the size of the estimated product indicates that the target intron is present in the pre-mRNA and was not removed/spliced from the pre-mRNA under the conditions tested, such introns are referred to as "retained introns."

In examples in which analysis is desired for many pre-RNAs or on a transcriptome-wide level, the screening for intron retention can be analyzed by RNA-seq or any other high-throughput transcriptional analysis method. RNA-seq analysis is carried out using appropriate mapping of deep sequencing reads and statistical methods to determine intron-retention events across the entire transcriptome.

### II. Confirmation of intron retention events

A second round of screening of introns within a pre-mRNA may be performed to confirm intron-retention events using methods such as RT-PCR. Each of the introns that were identified to be retained introns on the first round of screening described above can be assessed again. For RT-PCR analysis, each retained intron is assessed for retention in the pre-mRNA encoded by gene by designing primer pairs in which one of the primers of the pair is specific to a region of an intron of the target pre-mRNA and the other primer of the pair is specific to a region of an exon that is three, four, or five exons upstream or downstream of the intron (Figure 4). In the schematic presented in Figure 4, the retained intron to be assessed is located between exons 1 and 2. The upstream or forward primer is specific to a region and hybridizes within the retained intron and a downstream or reverse primer is designed to hybridize to a region in exon 4, exon 5, and exon 6, exons which are 3, 4, and 5 exons away from the retained intron, respectively. RT-PCR reactions are performed using the forward primer and each of the reverse primers.

Following RT-PCR, the RT-PCR products are analyzed by any method known in the art, for example, separation and visualization in an agarose gel. Based on the molecular size of RT-PCR products from each reaction, it can be determined whether each of the introns (e.g., the intron between exons 2 and 3, 3 and 4, and 4 and 5) is retained in addition to the intron being tested (the retained intron identified above). Retained introns that are found to be retained when one or more adjacent introns have been removed/spliced may be referred to as a an "inefficiently spliced intron."

### III. Determining intron splicing efficiency

Any introns in pre-mRNA encoded by a target gene that are identified as persistent introns or inefficiently spliced introns relative to other introns in the same pre-mRNA that are removed/spliced, may be further assessed to determine the proportion or efficiency of intron retention.

An intron may be assessed to determine the efficiency of intron retention by performing an assay such as an RNase protection assay (Figure 5). A pair of RNA probes (e.g., radioactively-labeled RNA probes) are designed in which each of the probes is specific to a region spanning the end of the retained intron and the adjacent exon. For example, an RNA probe is designed that hybridizes to the region spanning the 5' end of the retained intron and 3' end of the exon that is upstream of the retained intron; and a second RNA probe is designed that hybridizes to the region spanning the 3' end of the retained intron and the 5' end of the exon that is downstream of the retained intron. In some embodiments, the portion of the probe that hybridizes to the intron is at least 100 nucleotides in length and the portion of the probe that hybridizes to the exon is at least 50 nucleotides in length (Figure 5). Nuclear RNA extracted from disease-relevant cells, tissues or cell lines is incubated with the pair of RNA probes under conditions in which the probes hybridize to the regions of the pre-mRNA forming regions of double-stranded RNA. The mixture of pre-mRNA and RNA probes digested with RNases that degrade single-stranded RNA, such as RNaseA and/or RNase T1. Double-stranded RNA is protected from degradation.

The RNase digestion reactions are analyzed by any method known in the art, for example, separation and visualization in an agarose gel. The quantity of an RNA molecule that corresponds to the full-length of the RNA probe (*e.g.,* 150 nucleotides) indicates that amount of the retained intron present in the pre-mRNA. The quantity of RNA molecules that corresponds to digested RNA probes (*e.g.,* RNA molecules of approximately 50 nucleotides in length) represented the amount of spliced RNA as the intron to which the RNA probe hybridizes is not present in the pre-mRNA (*e.g.*, was spliced out). The ratio of intron retention (amount of full-length RNA probe, *e*.*g*., 100 nucleotide RNA molecules) over spliced RNA (amount of degraded RNA probe, *e.g.,* 50 nucleotide RNA molecules) indicates the efficiency of splicing of the intron. The intron of a pre-mRNA having the highest ratio relative to other introns of the same pre-mRNA indicates the intron is the least efficiently spliced intron or the most highly retained intron of the pre-mRNA encoded by the target gene.

### Methods of identifying an ASO that enhances splicing

Also within the scope of the present invention are methods for identifying (determining) ASOs that enhance splicing of a target pre-mRNA, specifically at the target intron. ASOs that specifically hybridize to different nucleotides within the target region of the pre-mRNA may be screened to identify (determine) ASOs that improve the rate and/or extent of splicing of the target intron. In some embodiments, the ASO may block or interfere with the binding site(s) of a splicing repressor(s)/silencer. Any method known in the art may be used to identify (determine) an ASO that when hybridized to the target region of the intron results in the desired effect (e.g., enhanced splicing, protein or functional RNA production). These methods also can be used for identifying ASOs that enhance splicing of the retained intron by binding to a targeted region in an exon flanking the retained intron, or in a non-retained intron. An example of a method that may be used is provided below

A round of screening, referred to as an ASO "walk" may be performed using ASOs that have been designed to hybridize to a target region of a pre-mRNA. For example, the ASOs used in the ASO walk can be tiled every 5 nucleotides from approximately 100 nucleotides upstream of the 5' splice site of the retained intron (*e.g.,* a portion of sequence of the exon located upstream of the target/retained intron) to approximately 100 nucleotides downstream of the 5' splice site of the target/retained intron and/or from approximately 100 nucleotides upstream of the 3' splice site of the retained intron to approximately 100 nucleotides downstream of the 3' splice site of the target/retained intron (*e.g.,* a portion of sequence of the exon located downstream of the target/retained intron). For example, a first ASO of 15 nucleotides in length may be designed to specifically hybridize to nucleotides +6 to +20 relative to the 5' splice site of the target/retained intron. A second ASO is designed to specifically hybridize to nucleotides +11 to +25 relative to the 5' splice site of the target/retained intron. ASOs are designed as such spanning the target region of the pre-mRNA. In embodiments, the ASOs can be tiled more closely, *e.g.,* every 1, 2, 3, or 4 nucleotides. Further, the ASOs can be tiled from 100 nucleotides downstream of the 5' splice site, to 100 nucleotides upstream of the 3' splice site.

One or more ASOs, or a control ASO (an ASO with a scrambled sequence, sequence that is not expected to hybridize to the target region) are delivered, for example by transfection, into a disease-relevant cell line that expresses the target pre-mRNA (e.g., the RIC pre-mRNA described elsewhere herein). The splicing-inducing effects of each of the ASOs may be assessed by any method known in the art, for example by reverse transcriptase (RT)-PCR using primers that span the splice junction, as described herein (see "Identification of intron-retention events"). A reduction or absence of the RT-PCR product produced using the primers spanning the splice junction in ASO-treated cells as compared to in control ASO-treated cells indicates that splicing of the target intron has been enhanced. In some embodiments, the splicing efficiency, the ratio of spliced to unspliced pre-mRNA, the rate of splicing, or the extent of splicing may be improved using the ASOs described herein. The amount of protein or functional RNA that is encoded by the target pre-mRNA can also be assessed to determine whether each ASO achieved the desired effect (*e.g.,* enhanced protein production). Any method known in the art for assessing and/or quantifying protein production, such as Western blotting, flow cytometry, immunofluorescence microscopy, and ELISA, can be used.

A second round of screening, referred to as an ASO "micro-walk" may be performed using ASOs that have been designed to hybridize to a target region of a pre-mRNA. The ASOs used in the ASO micro-walk are tiled every 1 nucleotide to further refine the nucleotide acid sequence of the pre-mRNA that when hybridized with an ASO results in enhanced splicing.

Regions defined by ASOs that promote splicing of the target intron are explored in greater detail by means of an ASO "micro-walk", involving ASOs spaced in 1-nt steps, as well as longer ASOs, typically 18-25 nt.

As described for the ASO walk above, the ASO micro-walk is performed by delivering one or more ASOs, or a control ASO (an ASO with a scrambled sequence, sequence that is not expected to hybridize to the target region), for example by transfection, into a disease-relevant cell line that expresses the target pre-mRNA. The splicing-inducing effects of each of the ASOs may be assessed by any method known in the art, for example by reverse transcriptase (RT)-PCR using primers that span the splice junction, as described herein (see "Identification of intron-retention events"). A reduction or absence of the RT-PCR product produced using the primers spanning the splice junction in ASO-treated cells as compared to in control ASO-treated cells indicates that splicing of the target intron has been enhanced. In some embodiments, the splicing efficiency, the ratio of spliced to unspliced pre-mRNA, the rate of splicing, or the extent of splicing may be improved using the ASOs described herein. The amount of protein or functional RNA that is encoded by the target pre-mRNA can also be assessed to determine whether each ASO achieved the desired effect (*e.g*., enhanced protein production). Any method known in the art for assessing and/or quantifying protein production, such as Western blotting, flow cytometry, immunofluorescence microscopy, and ELISA, can be used.

ASOs that when hybridized to a region of a pre-mRNA result in enhanced splicing and increased protein production may be tested *in vivo* using animal models, for example transgenic mouse models in which the full-length human gene has been knocked-in or in humanized mouse models of disease. Suitable routes for administration of ASOs may vary depending on the disease and/or the cell types to which delivery of the ASOs is desired. ASOs may be administered, for example, by intravitreal injection, intrathecal injection, intraperitoneal injection, subcutaneous injection, or intravenous injection. Following administration, the cells, tissues, and/or organs of the model animals may be assessed to determine the effect of the ASO treatment by for example evaluating splicing (efficiency, rate, extent) and protein production by methods known in the art and described herein. The animal models may also be any phenotypic or behavioral indication of the disease or disease severity.

### EXAMPLES

The present invention will be more specifically illustrated by the following Examples. However, it should be understood that the present invention is not limited by these examples in any manner.

### Example 1: Intron-retention events are intrinsic to genes and are non-productive

A first round of screening was performed for intron-retention events in the *PRPF31* (retinitis pigmentosa type 11) and *RB1* (retinoblastoma) genes using the methods described herein (Figure 3). Briefly, RNA extracts were isolated from nuclear fractions of HeLa (human epithelial cervical adenocarcinoma) and 293T (human embryonic kidney epithelial) cells, and nuclear and cytoplasmic fractions of ARPE-19 (human retina) cells. Reverse transcriptase PCR (RT-PCR) was performed using the RNA extracts from each of the cell types. In brief, cDNA synthesis was carried out with oligo dT to generate a DNA copy of Poly-A RNA (fully transcribed RNA) only, and PCR was performed to assess for intron retention in *PRPF31* and *RBI* transcripts. The PCR products were separated on a 1.5% ethidium-bromide-stained agarose gel (Figures 6A-6D). Results show several intron-retention events (marked by black asterisk) for both genes (*PRPF31* and *RB1*) in the nucleus of each of the three cell lines tested (Figures 6A-6D).

Tables 9 and 10 list all intron-retention events that occur in the three cell-lines tested for PRPF31 and RB1, respectively. The events (presence or absence of intron retention) that occur across all three cell-lines are indicated with an asterisk. The tables show that there is a very high concordance across the three cell lines indicating that the intron-retention events are intrinsic to the genes and are not affected by different cellular environments.
To address whether these events are non-productive (i.e. able to result in protein production), RT-PCR was performed using the cytoplasmic fraction of ARPE-19 cells (Figure 6E). Results show that the majority of the observed intron-retention events are not present in the cytoplasm of ARPE-19 cells (Figure 6E, asterisks mark where the bands should be) indicating, as expected, that the intron-retention events are result in the transcript being either retained in the nucleus or degraded by nonsense-mediated mRNA decay in the cytoplasm, and are therefore non-productive transcripts.

**Table 9: Summary of results for intron-retention events in the PRPF31 gene. "Yes" indicates the presence of intron retention; "no" indicates the absence of intron retention; and "?" indicates non-conclusive results. Cases in which there is concordance between the three cell lines are labeled with an asterisk.**

| PRPF31 | | | |
|---|---|---|---|
| **293T** | **Retina** | **HeLa** | **Intron** |
| Yes | Yes | Yes | 1* |
| No | No | No | 2* |
| Yes | Yes | Yes | 3* |
| Yes | Yes | Yes | 4* |
| No | Yes | No | 5 |
| No | No | No | 6* |
| No | No | No | 7* |
| No | No | No | 8* |
| ? | Yes | ? | 9 |
| ? | Yes | ? | 10 |
| No | No | No | 11* |
| Yes | Yes | Yes | 12* |
| No | No | No | 14* |

**Table 10. Summary of results for intron-retention events in the RB1 gene. "Yes" indicates the presence of intron retention; "no" indicates the absence of intron retention. Cases in which there is concordance between the three cell lines are labeled with an asterisk.**

| RB1 | | | |
|---|---|---|---|
| **293T** | **Retina** | **HeLa** | **Intron** |
| No | No | No | 1* |
| No | No | No | 2* |
| Yes | Yes | No | 3 |
| No | No | No | 4* |
| Yes | Yes | Yes | 5* |
| Yes | Yes | Yes | 6* |
| Yes | Yes | No | 7 |
| No | Yes | Yes | 8 |
| Yes | Yes | Yes | 9* |
| No | Yes | No | 10 |
| No | No | No | 11* |
| Yes | No | Yes | 12 |
| No | No | No | 13* |
| Yes | Yes | Yes | 14* |
| No | No | No | 15* |
| No | Yes | No | 16 |
| No | Yes | No | 17 |
| No | Yes | Yes | 18 |
| No | Yes | Yes | 19 |
| Yes | No | No | 20 |
| No | No | Yes | 21 |
| Yes | Yes | Yes | 22* |
| Yes | Yes | Yes | 23* |
| No | No | No | 24* |
| Yes | Yes | Yes | 25* |

### Example 2: Confirmation of intron retention events

A second round of screening was performed for intron-retention events in the *PRPF31* (retinitis pigmentosa type 11) and *RB1* (retinoblastoma) genes using the methods described herein (Figure 4). Briefly, nuclear RNA extracts from ARPE-19 (human retina) cells were used to perform reverse transcriptase PCR (RT-PCR) as described in Example 1. In this example, intron retention was assessed in the scenario in which more than one intron has been spliced out (removed) from the pre-mRNA. Results show fewer intron-retention events (marked by black asterisk) for both genes (*PPPF31* and *RB1*) (Figures 7A-7B) compared to results in Figures 6A-D) narrowing down the number of candidate intron retention events.

### Example 3: Improved splicing efficiency via mutagenesis or ASO targeting of intronic regions increases gene expression

We aimed to improve the splicing efficiency of each of the two introns of the *HBB* (human beta globin) gene, which is involved in beta thalassemia, and assess whether this would result in increased transcript level. The entire *HBB* open reading frame was cloned in a minigene reporter. Mutations were introduced into the 5' and 3' splice sites of both introns in order to bring them to perfect consensus sequences. Figure 8A shows a schematic representation of the *HBB* gene and the mutations introduced at the splice sites. Minigene reporters carrying mutations in each splice site as well as combinations of these mutations were transfected into HEK293 (human embryonic kidney epithelial) cells, independently, for 24 hrs using Fugene transfection reagent. Radioactive RT-PCR results show that mutations improving only the 5' splice site of intron 1 (IVS1) increase *HBB* transcript level (Figure 8B). Quantification of the intensity of the bands corresponding to *HBB* PCR products of mutant minigenes were normalized to that of GFP and plotted in relation to wild type *HBB.* The bars indicate an increase of more than 2-fold in the expression level of *HBB* when the splicing efficiency of intron 1 is improved (Figure 8C). We have previously observed that that *HBB* intron 1 is inefficiently spliced and is the rate limiting intron in the gene (data not shown). Here we show that by improving splicing efficiency of an inefficiently spliced intron, a significant increase in gene expression can be achieved.

To determine whether we can also achieve an increase in HBB-reporter gene (minigene) expression by improving splicing efficiency of HBB intron 1 using ASOs. To this end an 18-mer 2'-O-Me ASO was generated to target intron 1 starting at positions +7 and two 18-mer PMO-ASOs were generated to target intron 1 starting at positions +6 and +7, respectively, relative to the 5' splice junction (Figure 9A; Table 2, SEQ ID NO: 104 and 105, respectively). HEK293 cells were first co-transfected with wild-type HBB minigene reporter and GFP (as a transfection control) using Fugene transfection reagent. Four hours later, cells were either untransfected, mock-transfected, or transfected with each of the targeting ASOs or a non-targeting ASO control, independently, using RNAiMAX (RiM) (Invitrogen) or EndoPorter (EP) (GeneTools) delivery reagents. Experiments were performed using increasing concentrations of the ASOs as indicated in Figure 9B) for 48 hrs. Radioactive RT-PCR results show that the +7 targeting ASO with both chemistries increase *HBB* transcript level compared to the mock-transfected or non-targeting ASO (Figure 9B). Similar results were obtained for the +6 PMO-ASO (data not shown). Intensities of the bands corresponding to the *HBB* PCR products from targeting-ASO-transfected cells were normalized to GFP and plotted relative to the normalized *HBB* PCR product from mock-treated cells. Results of this analysis indicate that both targeting ASOs (+6 and +7) increase *HBB* transcript level by nearly 50% (Figure 9C). These results indicate that improving the splicing efficiency of the rate limiting intron in the *HBB* gene using ASOs leads to an increase in gene expression.

### Example 4: Improved splicing efficiency via ASO targeting an intronic region increases protein production

In order to detect an increase in protein production upon targeting *HBB* intron 1 with the +7 2'-O-Me ASO, we generated a reporter construct consisting of the HBB minigene flanked upstream by the GFP open reading frame and downstream by a sequence coding the T7 tag (Figure 10A). This reporter was integrated in the genome of U2OS cells mimicking an endogenous gene. U2OS cells expressing the GFP-HBB-T7 reporter were mock-transfected or transfected with the +7 2'-O-Me ASO and protein extracts were analyzed by western blot. Briefly, protein extracts from two independent biological replicates were run on a 4-20% SDS-polyacrylamide gel, transferred to a nitrocellulose membrane. To evidence an increase in protein production, an anti-GFP antibody was used to detect a protein product from the GFP-HBB-T7 reporter and an anti-Beta tubulin antibody was used to detect Beta tubulin as a loading control. Figure 10B shows western blots results indicating that GFP-HBB-T7 protein (bottom band) is increased upon treatment with the +7 2'-O-Me ASO. Intensities of the bands corresponding to the GFP-HBB-T7 protein from targeting-ASO-transfected cells were normalized to endogenous Beta tubulin and plotted relative to the normalized GFP-HBB-T7 protein band from mock-treated cells.

Results of this analysis indicate that the targeting ASO (+7) increase GFP-HBB-T7 protein level by more than 2.5 fold (Figure 10C). These results demonstrate that promoting splicing efficiency by using an ASO targeted to a region downstream of the 5' splice site of the rate-limiting intron leads to an increase in target protein production as depicted in Figure 2.

### Example 5: Identification of intron retention events in ADAMTS13 transcripts by RNAseq using next generation sequencing

We performed whole transcriptome shotgun sequencing using next generation sequencing to reveal a snapshot of transcripts produced by the *ADAMTS13* gene to identify intron-retention events. For this purpose, we isolated polyA+ RNA from nuclear and cytoplasmic fractions of THLE-3 (human liver epithelial) cells and constructed cDNA libraries using Illumina's TruSeq Stranded mRNA library Prep Kit. The libraries were pair-end sequenced resulting in 100-nucleotide reads that were mapped to the human genome (Feb. 2009, GRCh37/hg19 assembly). The sequencing results for ADAMTS13 are shown in Figure 11. Briefly, Figure 11 shows the mapped reads visualized using the UCSC genome browser, operated by the UCSC Genome Informatics Group (Center for Biomolecular Science & Engineering, University of California, Santa Cruz, 1156 High Street, Santa Cruz, CA 95064) and described by, e.g., Rosenbloom, et al., 2015, "The UCSC Genome Browser database: 2015 update," Nucleic Acids Research 43, Database Issue (doi: 10.1093/nar/gku1177) and the coverage and number of reads can be inferred by the peak signals. The height of the peaks indicates the level of expression given by the density of the reads in a particular region. A schematic representation of all ADAMTS13 isoforms (drawn to scale) is provided by the UCSC genome browser (below the read signals) so that peaks can be matched to ADAMTS13 exonic and intronic regions. Based on this display, we identified two introns (25 and 27, indicated by arrows) that have high read density in the nuclear fraction of THLE-3 cells, but have very low to no reads in the cytoplasmic fraction of these cells (as shown for intron 25 in the bottom diagram of Figure 11). This indicates that both introns are retained and that the intron-25 and intron-27 containing transcripts remain in the nucleus. This suggests that these retained intron-containing (RIC) ADAMTS13 pre-mRNAs are non-productive, as they are not exported out to the cytoplasm.

### Example 6: Validation of intron retention events identified by RNAseq analysis of ADAMTS13

Validation of the intron 25-retention event in the *ADAMTS13* (thrombotic thrombocytopenic purpura) gene was performed using the methods described herein (Figure 12). Briefly, nuclear and cytoplasmic RNA extracts from A172 (human glioblastoma) and HepG2 (human hepatocellular carcinoma) cells were used to perform radioactive reverse transcriptase PCR (RT-PCR) as described in Example 1. In this example, intron retention was assessed using primers positioned in exon 25 and exon 27 leading to the amplification of both intron-25 containing transcript and correctly spliced transcript. The products were run in a 5% polyacrylamide gel and visualized by phosphorimaging. Intron 25 retention levels were calculated as percent intron retention (PIR) of the intensity of the band corresponding to the intron-25 containing product over total transcript (intron-containing plus correctly spliced). Quantification of the bands indicated that approximately 80% of ADAMTS13 transcripts contain intron 25 and that this product is retained in the nucleus. Moreover, the radioactive RT-PCR results validated the bioinformatic predictions demonstrating that the bioinformatic analysis of the RNAseq results is a powerful tool to identify intron-retention events.

### Example 7: Design of ASO-walk targeting intron 25 of ADAMTS13

An ASO walk was designed to target intron 25 using the method described herein (Figure 13). A region immediately downstream of intron 25 5' splice site spanning nucleotides +6 to +58 and a region immediately upstream of intron 25 3' splice site spanning nucleotides -16 to -79 of the intron were targeted with 2'-O-Me RNA, PS backbone, 18-mer ASOs shifted by 5-nucleotide intervals (with the exception of 1 ASO, ADAM-IVS25-47, to avoid a stretch of four guanines) (Figure 13; Table 4, SEQ ID NO:150 to 167). These target regions were selected based on the knowledge that intronic regulatory elements concentrate in sequences adjacent to splice sites.

### Example 8: Improved splicing efficiency via ASO-targeting of ADAMTS13 intron 25 increases transcript levels

To determine whether we can achieve an increase in *ADAMTS13* expression by improving splicing efficiency of *ADAMTS13* intron 25 using ASOs we used the method described herein (Figure 14). To this end, HepG2 cells were mock-transfected, or transfected with each of the targeting ASOs described in Figure 13 and Table 4, SEQ ID NO: 150 to 167, or a non-targeting SMN-ASO control, independently, using RNAiMAX (RiM) (Invitrogen) delivery reagents. Experiments were performed using 60 nM ASOs (as indicated in Figure 14) for 48 hrs. Radioactive RT-PCR results show that the +21 and +26 targeting ASOs increase *ADAMTS13* transcript level compared to the mock-transfected or non-targeting ASO (Figure 14). Intensities of the bands corresponding to the *ADAMTS13* PCR products from targeting-ASO-transfected cells were normalized to Beta actin and plotted relative to the normalized *ADAMTS13* PCR product from control ASO-treated cells. Results of this analysis indicate that both targeting ASOs (+21 and +26) increase *ADAMTS13* transcript level nearly 2.5 fold (Figure 14). These results indicate that improving the splicing efficiency of a rate limiting intron in the *ADAMTS13* gene using ASOs leads to an increase in gene expression.

### Example 9: Dose response effect of ASOs targeting ADAMTS13 intron 25

To determine a dose-response effect of the +21 and +26 ASOs, as well as the -46 ASOs that showed the opposite effect (Figure 14), we used the method described herein (Figure 15). HepG2 cells were mock-transfected, or transfected with each of the three ASOs, or a non-targeting SMN-ASO control, independently, using RNAiMAX (RiM) (Invitrogen) delivery reagents at increasing concentrations as indicated in Figure 15 for 48 hrs. Radioactive RT-PCR results show that the +21 and +26 targeting ASOs increase *ADAMTS13* transcript level compared to the mock-transfected or non-targeting ASO whereas the -46 ASO decreases *ADAMTS13* transcript level compared to the mock-transfected or non-targeting ASO (Figure 15). Intensities of the bands corresponding to the *ADAMTS13* PCR products from targeting-ASO-transfected cells were normalized to Beta actin and plotted relative to the normalized *ADAMTS13* PCR product from control ASO-treated cells. Results of this analysis indicate that both targeting ASOs (+21 and +26) increase *ADAMTS13* transcript level nearly 2.5 fold (Figure 15). These results confirm that improving the splicing efficiency of a rate limiting intron in the *ADAMTS13* gene using ASOs leads to an increase in gene expression.

### Example 10: Improved splicing efficiency via ASO-targeting of ADAMTS13 intron 25 increases protein levels

In order to detect an increase in protein production upon targeting *ADAMTS13* intron 25 with the +21 or +26 ASOs, we used the method described herein (Figure 16). HepG2 cells were mock-transfected, or transfected with each of the three ASOs, or a non-targeting SMN-ASO control, independently, using RNAiMAX (RiM) (Invitrogen) delivery reagents at increasing concentrations as indicated in Figure 16 for 48 hrs. Briefly, protein extracts from HepG2 treated cells were run on an 8% SDS-polyacrylamide gel, and transferred to a nitrocellulose membrane. To evidence an increase in protein production, an anti-ADAMTS13 antibody or anti-Alpha tubulin antibody was used to detect ADAMTS13 and Alpha tubulin as a loading control, respectively. Figure 16 shows western blot results indicating that ADAMTS13 (top panel) is increased in a dose dependent manner upon treatment with the +21 or +26 ASO. Intensities of the bands corresponding to the ADAMTS13 protein from targeting-ASO-transfected cells were normalized to endogenous Alpha tubulin and plotted relative to the normalized ADAMTS13 protein band from mock-treated cells. Results of this analysis indicate that the targeting ASOs (+21 and +26) increase ADAMTS13 protein level more than 3 fold (Figure 16). These results demonstrate that promoting splicing efficiency by using an ASO targeted to a region downstream of the 5' splice site of ADAMTS13 intron 25, a rate-limiting intron, leads to an increase in target protein production as depicted in Figure 2.

### Example 11: Design of ASO-microwalk targeting the +21 to +26 region of ADAMTS13 intron 25

An ASO microwalk was designed to target intron 25 +21 to +26 region using the method described herein (Figure 17). A region downstream of intron 25 5' splice site spanning +17 to +46 were targeted with 2'-O-Me, 5'-Me-Cytosine RNA, PS backbone, 18-mer ASOs shifted by 1-nucleotide interval (Figure 17; Table 4, SEQ ID NO:184 to 197). This target region was selected based on the observed effect of ASOs +21 and +26 (Figure 16).

### Example 12: Improved splicing efficiency via ASO microwalk targeting of ADAMTS13 intron 25 +21 to +26 region increases transcript levels

To determine whether we can achieve an increase in *ADAMTS13* expression by improving splicing efficiency of *ADAMTS13* intron 25 using microwalk ASOs, we employed the method described herein (Figure 18). To this end, HepG2 cells were mock-transfected, or transfected with each of the targeting ASOs described in Figure 17 and Table 4 SEQ ID NO:184 to 197, or a non-targeting SMN-ASO control, independently, using RNAiMAX (RiM) (Invitrogen) delivery reagents. Experiments were performed using 60 nM ASOs (as indicated in Figure 18) for 48 hrs. Radioactive RT-PCR results show that the +21 with 5'-Me-Cytosines and +25 targeting ASOs further increase *ADAMTS13* transcript level compared to the mock-transfected or non-targeting ASO, as well as the two original +21 and +26 ASOs (light grey bars, Figure 18). Intensities of the bands corresponding to the *ADAMTS13* PCR products from targeting-ASO-transfected cells were normalized to Beta actin and plotted relative to the normalized *ADAMTS13* PCR product from control ASO-treated cells. Results of this analysis indicate that both targeting ASOs (+21 and +25) increase *ADAMTS13* transcript level by nearly 2.0 fold (Figure 18). These results indicate that improving the splicing efficiency of a rate limiting intron in the *ADAMTS13* gene using ASOs leads to an increase in gene expression, and the refinement of the target region by a microwalk can lead to the identification of more efficient ASOs.

### Example 13: Identification of intron retention events in TSC1 transcripts by RNAseq using next generation sequencing

We performed whole transcriptome shotgun sequencing using next generation sequencing to reveal a snapshot of transcripts produced by the *TSC1* gene to identify intron-retention events. For this purpose, we isolated polyA+ RNA from nuclear and cytoplasmic fractions of primary human astrocytes (AST) and primary human cortical neuron (HCN) cells and constructed cDNA libraries using Illumina's TruSeq Stranded mRNA library Prep Kit. The libraries were pair-end sequenced resulting in 100-nucleotide reads that were mapped to the human genome (Feb. 2009, GRCh37/hg19 assembly). The sequencing results for TSC1 are shown in Figure 19. Briefly, Figure 19 shows the mapped reads visualized using the UCSC genome browser and the coverage and number of reads can be inferred by the peak signals. The height of the peaks indicates the level of expression given by the density of the reads in a particular region. A schematic representation of all TSC1 isoforms (drawn to scale) is provided by the UCSC genome browser (below the read signals) so that peaks can be matched to TSC1 exonic and intronic regions. Based on this display, we identified three introns (5, 10 and 11, indicated by arrows) that have high read density in the nuclear fraction of AST and HCN cells, but have very low to no reads in the cytoplasmic fraction of these cells (as shown for intron 10 in the bottom diagram of Figure 19). This indicates that both introns are retained and that the intron-5, intron-10, and intron-11 containing transcripts remain in the nucleus. This suggests that these retained intron-containing (RIC) TSC1 pre-mRNAs are non-productive, as they are not exported out to the cytoplasm.

### Example 14: Validation of intron retention events identified by RNAseq analysis of TSC1

Validation of the intron 10-retention event in the *TSC1* (tuberous sclerosis complex 1) gene was performed using the methods described herein (Figure 20). Briefly, nuclear and cytoplasmic RNA extracts from A172 (human glioblastoma) cells were used to perform radioactive reverse transcriptase PCR (RT-PCR) as described in Example 1. In this example, intron retention was assessed using primers positioned in exon 9 and exon 11 leading to the amplification of both intron-10 containing transcript and correctly spliced transcript. The products were run in a 5% polyacrylamide gel and visualized by phosphorimaging. Intron 10 retention levels were calculated as percent intron retention (PIR) of the intensity of the band corresponding to the intron-10 containing product over total transcript (intron-containing plus correctly spliced). Quantification of the bands indicated that approximately 36% of TSC1 transcripts contain intron 10 and that this product is retained in the nucleus. Moreover, the radioactive RT-PCR results validated the bioinformatic predictions demonstrating that the bioinformatic analysis of the RNAseq results is a powerful tool to identify intron-retention events.

### Example 15: Design of ASO-walk targeting intron 10 of TSC1

An ASO walk was designed to target intron 10 using the method described herein (Figure 21). A region immediately downstream of intron 10 5' splice site spanning nucleotides +6 to +58 and a region immediately upstream of intron 10 3' splice site spanning nucleotides -16 to -68 of the intron were targeted with 2'-O-Me RNA, PS backbone, 18-mer ASOs shifted by 5-nucleotide intervals (Figure 21; Table 5, SEQ ID NOS: 214 to 229). These target regions were selected based on the knowledge that intronic regulatory elements concentrate in sequences adjacent to splice sites.

### Example 16: Improved splicing efficiency via ASO-targeting of TSC1 intron 10 increases transcript levels

To determine whether we can achieve an increase in *TSC1* expression by improving splicing efficiency of *TSC1* intron 10 using ASOs, we used the method described herein (Figure 22). To this end, A172 cells were mock-transfected, or transfected with each of the targeting ASOs described in Figure 21 and Table 5, SEQ ID NOS: 214 to 229, or a non-targeting SMN-ASO control, independently, using RNAiMAX (RiM) (Invitrogen) delivery reagents. Experiments were performed using 60 nM ASOs (as indicated in Figure 22) for 48 hrs. Radioactive RT-PCR results show that the +31 targeting ASO increases *TSC1* transcript level compared to the mock-transfected or non-targeting ASO (Figure 22). Intensities of the bands corresponding to the *TSC1* PCR products from targeting-ASO-transfected cells were normalized to Beta actin and plotted relative to the normalized *TSC1* PCR product from mock-treated cells. Results of this analysis indicate that several ASOs (including +31) increase *TSC1* transcript level nearly 1.5 fold (Figure 22). These results indicate that improving the splicing efficiency of a rate limiting intron in the *TSC1* gene using ASOs leads to an increase in gene expression.

### Example 17: Dose response effect of ASOs targeting TSC1 intron 10

To determine a dose-response effect of the +31 ASO, we used the method described herein (Figure 23). A172 cells were mock-transfected, or transfected with the +31 ASO, or a non-targeting SMN-ASO control, independently, using RNAiMAX (RiM) (Invitrogen) delivery reagents at increasing concentrations as indicated in Figure 23 for 72 hrs. Radioactive RT-PCR results show that the +31 targeting ASO increases *TSC1* transcript level compared to the mock-transfected or non-targeting ASO (Figure 23). Intensities of the bands corresponding to the *TSC1* PCR products from targeting-ASO-transfected cells were normalized to Beta actin and plotted relative to the normalized *TSC1* PCR product from mock-treated cells. Results of this analysis indicate the +31 targeting ASO increases *TSC1* transcript level in a dose-dependent manner nearly 2.0 fold (Figure 23). These results were confirmed by RTqPCR using primers elsewhere in the *TSC1* transcript, showing a 3-fold increase, and a dose-dependant response to the ASO treatment. These results confirm that improving the splicing efficiency of a rate limiting intron in the *TSC1* gene using ASOs leads to an increase in gene expression.

### Example 18: Improved splicing efficiency via ASO-targeting of TSC1 intron 10 increases protein levels

In order to detect an increase in protein production upon targeting *TSC1* intron 10 with the +31 ASO, we used the method described herein (Figure 24). A172 cells were mock-transfected, or transfected with the +31 ASO, or a non-targeting SMN-ASO control, independently, using RNAiMAX (RiM) (Invitrogen) delivery reagents at increasing concentrations as indicated in Figure 24 for 72 hrs. Briefly, protein extracts from A172 treated cells were run on a 10% SDS-polyacrylamide gel, and transferred to a nitrocellulose membrane. To evidence an increase in protein production, an anti-TSC1 antibody or anti-Alpha tubulin antibody was used to detect TSC1 and Alpha tubulin as a loading control, respectively. Figure 24 shows western blot results indicating that TSC1 (top panel) is increased in a dose dependent manner upon treatment with the +31 ASO at 30 and 60 nM. Intensities of the bands corresponding to the TSC1 protein from targeting-ASO-transfected cells were normalized to endogenous Alpha tubulin and plotted relative to the normalized TSC1 protein band from mock-treated cells. Results of this analysis indicate that the targeting ASO (+31) increases TSC1 protein level more than 2 fold (Figure 24). These results demonstrate that promoting splicing efficiency by using an ASO targeted to a region downstream of the 5' splice site of TSC1 intron 10, a rate-limiting intron, leads to an increase in target protein production as depicted in Figure 2.

### Example 19: Identification of intron retention events in IMPDH1 transcripts by RNAseq using next generation sequencing

We performed whole transcriptome shotgun sequencing using next generation sequencing to reveal a snapshot of transcripts produced by the *IMPDHI* gene (retinitis pigmentosa 10) to identify intron-retention events. For this purpose, we isolated polyA+ RNA from nuclear and cytoplasmic fractions of ARPE-19 (human retina epithelial) cells and constructed cDNA libraries using Illumina's TruSeq Stranded mRNA library Prep Kit. The libraries were pair-end sequenced resulting in 100-nucleotide reads that were mapped to the human genome (Feb. 2009, GRCh37/hg19 assembly). The sequencing results for IMPDH1 are shown in Figure 25. Briefly, Figure 25 shows the mapped reads visualized using the UCSC genome browser and the coverage and number of reads can be inferred by the peak signals. The height of the peaks indicates the level of expression given by the density of the reads in a particular region. A schematic representation of all IMPDH1 isoforms (drawn to scale) is provided by the UCSC genome browser (below the read signals), so that peaks can be matched to IMPDH1 exonic and intronic regions. Based on this display, we identified one intron (14, indicated by arrow) that has high read density in the nuclear fraction of ARPE-19 cells, but has no reads in the cytoplasmic fraction of these cells (as shown for intron 14 in the bottom diagram of Figure 25). This indicates that intron 14 is retained and that the intron-14 containing transcript remains in the nucleus. This suggests that the retained intron-containing (RIC) IMPDH1 pre-mRNAs is non-productive, as it is not exported out to the cytoplasm.

### Example 20: Design of ASO-walk targeting intron 14 of IMPDH1

An ASO walk was designed to target intron 14 using the method described herein (Figure 26). A region immediately downstream of intron 14 5' splice site spanning nucleotides +6 to +65 and a region immediately upstream of intron 14 3' splice site spanning nucleotides -16 to -68 of the intron were targeted with 2'-O-Me RNA, PS backbone, 18-mer ASOs shifted by 5-nucleotide intervals (with the exception of 1 ASO, IMP-IVS14+18, to avoid a stretch of four guanines) (Figure 26; Table 6, SEQ ID NOS: 246 to 261). These target regions were selected based on the knowledge that intronic regulatory elements concentrate in sequences adjacent to splice sites.

### Example 21: Improved splicing efficiency via ASO-targeting of IMPDH1 intron 14 increases transcript levels

To determine whether we can achieve an increase in *IMPDHI* expression by improving splicing efficiency of *IMPDH1* intron 14 using ASOs, we used the method described herein (Figure 27). To this end, ARPE-19 cells were mock-transfected, or transfected with each of the targeting ASOs described in Figure 26 and Table 6, SEQ ID NOS: 246 to 261, or a non-targeting SMN-ASO control, independently, using RNAiMAX (RiM) (Invitrogen) delivery reagents. Experiments were performed using 60 nM ASOs (as indicated in Figure 27) for 48 hrs. Radioactive RT-PCR results show that the +48 targeting ASO increases *IMPDHI* transcript level compared to the mock-transfected or non-targeting ASO (Figure 27). Intensities of the bands corresponding to the *IMPDH1* PCR products from targeting-ASO-transfected cells were normalized to Beta actin and plotted relative to the normalized *IMPDHI* PCR product from control ASO-treated cells. Results of this analysis indicate that the targeting ASO (+48) increases *IMPDHI* transcript level 4.0 fold (Figure 27). These results indicate that improving the splicing efficiency of a rate limiting intron in the *IMPDH1* gene using ASOs leads to an increase in gene expression.

### Example 22: Dose response effect of ASO +48 targeting IMPDHI intron 14

To determine a dose-response effect of the +48 ASO, we used the method described herein (Figure 28). ARPE-19 cells were mock-transfected, or transfected with the +48 ASO, or a non-targeting SMN-ASO control, independently, using RNAiMAX (RiM) (Invitrogen) delivery reagents at increasing concentrations as indicated in Figure 28 for 72 hrs. Radioactive RT-PCR results show that the +48 targeting ASO increases *IMPDHI* transcript level compared to the mock-transfected or non-targeting ASO in a dose-dependant manner (Figure 28). Intensities of the bands corresponding to the *IMPDH1* PCR products from targeting-ASO-transfected cells were normalized to Beta actin and plotted relative to the normalized *IMPDH1* PCR product from mock-treated cells. Results of this analysis indicate that the targeting ASO (+48) increases *IMPDHI* transcript level nearly 1.5 fold (Figure 28, middle graph). These results were confirmed by RTqPCR using primers elsewhere in the *IMPDH1* transcript, showing a 2.5-fold increase, and a dose-dependant response to the ASO treatment (Figure 28, right graph). In addition PIR was calculated (as described in Example 6) for intron 14 retention and the values were plotted indicating that as the ASO concentration and the correctly spliced transcript increases, a reduction in intron 14 retention is observed (Figure 28, left graph). These results confirm that improving the splicing efficiency of a rate limiting intron in the *IMPDH1* gene using ASOs leads to an increase in gene expression.

### Example 23: Improved splicing efficiency via ASO-targeting of IMPDH1 intron 14 increases protein levels

In order to detect an increase in protein production upon targeting *IMPDHI* intron 14 with the +48 ASO, we used the method described herein (Figure 29). ARPE-19 cells were mock-transfected, or transfected with the +48 ASO, or a non-targeting SMN-ASO control, independently, using RNAiMAX (RiM) (Invitrogen) delivery reagents at increasing concentrations as indicated in Figure 29 for 72 hrs. Briefly, protein extracts from ARPE-19 treated cells were run on an 4-20% SDS-polyacrylamide gel, and transferred to a nitrocellulose membrane. To evidence an increase in protein production, an anti-IMPDH1 antibody, anti-Beta catenin antibody, or Beta actin was used to detect IMPDH1, and Beta catenin or Beta actin as loading controls, respectively. Figure 29 shows western blot results indicating that IMPDH1 is increased in a dose dependent manner upon treatment with the +48 ASO. Intensities of the bands corresponding to the IMPDH1 protein from targeting-ASO-transfected cells were normalized to endogenous Beta actin and plotted relative to the normalized IMPDH1 protein band from mock-treated cells. Results of this analysis indicate that the targeting ASO (+48) increase IMPDH1 protein level nearly 2.5 fold (Figure 29). These results demonstrate that promoting splicing efficiency using an ASO targeted to a region downstream of the 5' splice site of IMPDH1 intron 14, a rate-limiting intron, leads to an increase in target protein production as depicted in Figure 2.

### Example 24: Design of ASO-microwalk targeting the +48 region of IMPDH1 intron 14

An ASO microwalk was designed to target intron 14 +44 to +70 region using the method described herein (Figure 30). A region downstream of intron 14 5' splice site spanning +44 to +70 were targeted with 2'-O-Me, 5'-Me-Cytosine RNA, PS backbone, 18-mer ASOs shifted by 1-nucleotide interval (Figure 30; Table 6, SEQ ID NOS: 262 to 271). This target region was selected based on the observed effect of ASO +48 (Figure 29).

### Example 25: Improved splicing efficiency via ASO microwalk targeting of IMPDH1 intron 14 +48 region increases transcript levels

To determine whether we can achieve an increase in *IMPDHI* expression by improving splicing efficiency of *IMPDH1* intron 14 using microwalk ASOs, we employed the method described herein (Figure 31). To this end, ARPE-19 cells were mock-transfected, or transfected with each of the targeting ASOs described in Figure 30 and Table 6, SEQ ID NOS: 262 to 271, or a non-targeting SMN-ASO control, independently, using RNAiMAX (RiM) (Invitrogen) delivery reagents. Experiments were performed using 60 nM ASOs (as indicated in Figure 31) for 48 hrs. RT-qPCR results show that the +46 and +47 targeting ASOs further increase *IMPDHI* transcript level compared to the mock-transfected or non-targeting ASO, as well as the original +48 ASO (Figure 31). Results of this analysis indicate that both targeting ASOs (+46 and +47) increase *IMPDHI* transcript level more than 3.0 fold (Figure 31). These results indicate that improving the splicing efficiency of a rate limiting intron in the *IMPDH1* gene using ASOs leads to an increase in gene expression, and the refinement of the target region by a microwalk can lead to the identification of more efficient ASOs.

### Example 26: Identification of intron retention events in PKD1 transcripts by RNAseq using next generation sequencing

We performed whole transcriptome shotgun sequencing using next generation sequencing to reveal a snapshot of transcripts produced by the *PKD1* gene (polycystic kidney disease) to identify intron-retention events. For this purpose, we isolated polyA+ RNA from nuclear and cytoplasmic fractions of primary human renal epithelial (REN) cells and constructed cDNA libraries using Illumina's TruSeq Stranded mRNA library Prep Kit. The libraries were pair-end sequenced resulting in 100-nucleotide reads that were mapped to the human genome (Feb. 2009, GRCh37/hg19 assembly). The sequencing results for *PKD1* are shown in Figure 32. Briefly, Figure 32 shows the mapped reads visualized using the UCSC genome browser and the coverage and number of reads can be inferred by the peak signals. The height of the peaks indicates the level of expression given by the density of the reads in a particular region. A schematic representation of all *PKD1* isoforms (drawn to scale) is provided by the UCSC genome browser (below the read signals) so that peaks can be matched to *PKD1* exonic and intronic regions. Based on this display, we identified four introns (32, 33, 37 and 38, indicated by arrows) that have high read density in the nuclear fraction of REN cells, but have very low to no reads in the cytoplasmic fraction of these cells (as shown for intron 37 in the bottom diagram of Figure 32). This indicates that the four introns are retained and that the intron-32, intron-33, intron-37, and intron-38 containing transcripts remain in the nucleus. This suggests that these retained intron-containing (RIC) PKD1 pre-mRNAs are non-productive, as they are not exported out to the cytoplasm.

### Example 27: Design of ASO-walk targeting intron 37 of PKD1

An ASO walk was designed to target intron 37 using the method described herein (Figure 33). A region immediately downstream of intron 37 5' splice site spanning nucleotides +6 to +66 and a region immediately upstream of intron 37 3' splice site spanning nucleotides -16 to -51 of the intron were targeted with 2'-O-Me RNA, PS backbone, 18-mer ASOs shifted by 5-nucleotide intervals (with the exception of 2 ASOs, PKD1-IVS37+8 and +24, to avoid a stretch of four guanines) (Figure 33; Table 7, SEQ ID NOS: 297 to 312). These target regions were selected based on the knowledge that intronic regulatory elements concentrate in sequences adjacent to splice sites.

### Example 28: Improved splicing efficiency via ASO-targeting of PKD1 intron 37 increases transcript levels

To determine whether we can achieve an increase in *PKD1* expression by improving splicing efficiency of *PKD1* intron 37 using ASOs, we used the method described herein (Figure 34). To this end, HEK293 cells were mock-transfected, or transfected with each of the targeting ASOs described in Figure 33 and Table 7, SEQ ID NOS: 297 to 312, or a non-targeting SMN-ASO control, independently, using RNAiMAX (RiM) (Invitrogen) delivery reagents. Experiments were performed using 60 nM ASOs (as indicated in Figure 34) for 48 hrs. Radioactive RT-PCR results show that the +29 targeting ASO increases *PKD1* transcript level compared to the mock-transfected or non-targeting ASO (Figure 34). Intensities of the bands corresponding to the *PKD1* PCR products from targeting-ASO-transfected cells were normalized to Beta actin and plotted relative to the normalized *PKD1* PCR product from mock-treated cells. Results from this analysis indicate that the +29 ASO increases *PKD1* transcript level 1.8 fold (Figure 34). These results indicate that improving the splicing efficiency of a rate limiting intron in the *PKD1* gene using ASOs leads to an increase in gene expression.

### Example 29: Dose response effect of ASOs targeting PKD1 intron 37

To determine a dose-response effect of the +29 ASO, we used the method described herein (Figure 35). HEK293 cells were mock-transfected, or transfected with the +29 ASO, or a non-targeting SMN-ASO control, independently, using RNAiMAX (RiM) (Invitrogen) delivery reagents at increasing concentrations as indicated in Figure 35 for 48 hrs. Radioactive RT-PCR results show that the +29 targeting ASO increases *PKD1* transcript level compared to the mock-transfected or non-targeting ASO (Figure 35). Intensities of the bands corresponding to the *PKD1* PCR products from targeting-ASO-transfected cells were normalized to Beta actin and plotted relative to the normalized *PKD1* PCR product from mock-treated cells. Results of this analysis indicate the +29 targeting ASO increases *PKD1* transcript level in a dose-dependent manner more than 2.0 fold (Figure 35, middle graph). These results were confirmed by RTqPCR using primers elsewhere in the *PKD1* transcript, showing more than 2-fold increase, and a dose-dependant response to the ASO treatment (Figure 35, right graph). In addition, PIR was calculated (as described in Example 6) for intron 37 retention and the values were plotted indicating that as the ASO concentration and the correctly spliced transcript increases, a reduction in intron 37 retention is observed (Figure 35, left graph). These results confirm that improving the splicing efficiency of a rate limiting intron in the *PKD1* gene using ASOs leads to an increase in gene expression.

### Example 30: Improved splicing efficiency via ASO-targeting of PKD1 intron 37 increases protein levels

In order to detect an increase in protein production upon targeting *PKD1* intron 37 with the +29 ASO, we used the method described herein (Figure 36). HEK293 cells were mock-transfected, or transfected with the +29 ASO, or a non-targeting SMN-ASO control, independently, using RNAiMAX (RiM) (Invitrogen) delivery reagents at increasing concentrations as indicated in Figure 36 for 72 hrs. Briefly, cells were fixed and permeabilized and treated with an anti-PKD1 antibody or IgG isotype control antibody. Cells were analyzed by flow cytometry by counting 10,000 cells. Figure 36 shows a plot of the fluorescence intensity/per cell count indicating that a higher ASO concentrations cell have a stronger PKD1 signal compared to mock-treated (untreated) cells. Fold change of the fluorescence intensity corresponding to the +29 ASO-treated cells relative to the fluorescence intensity corresponding to the mock-teated cells was plotted. Results of this analysis indicate that the targeting ASO (+29) increases PKD1 protein level nearly 1.5 fold (Figure 36). These results demonstrate that promoting splicing efficiency by using an ASO targeted to a region downstream of the 5' splice site of PKD1 intron 37, a rate-limiting intron, leads to an increase in target protein production as depicted in Figure 2.

### Example 31: Identification of intron retention events in IKBKAP transcripts by RNAseq using next generation sequencing

We performed whole transcriptome shotgun sequencing using next generation sequencing to reveal a snapshot of transcripts produced by the *IKBKAP* gene to identify intron-retention events. For this purpose, we isolated polyA+ RNA from nuclear and cytoplasmic fractions of ARPE-19, AST, human bronchial epithelial (BRON), HCN, REN, and THLE-3 cells and constructed cDNA libraries using Illumina's TruSeq Stranded mRNA library Prep Kit. The libraries were pair-end sequenced resulting in 100-nucleotide reads that were mapped to the human genome (Feb. 2009, GRCh37/hg19 assembly). The sequencing results for IKBKAP are shown in Figure 37. Briefly, Figure 37 shows the mapped reads visualized using the UCSC genome browser and the coverage and number of reads can be inferred by the peak signals. The height of the peaks indicates the level of expression given by the density of the reads in a particular region. A schematic representation of all IKBKAP isoforms (drawn to scale) is provided by the UCSC genome browser (below the read signals), so that peaks can be matched to IKBKAP exonic and intronic regions. Based on this display, we identified 2 introns (7 and 8, indicated by arrows) that have high read density in the nuclear fraction of all cells sequenced, but has no reads in the cytoplasmic fraction of these cells (as shown for both introns in the bottom diagram of Figure 37). This indicates that introns 7 and 8 are retained and that the intron-7 and intron-8 containing transcript remain in the nucleus. This suggests that the retained intron-containing (RIC) IKBKAP pre-mRNAs are non-productive, as they are not exported out to the cytoplasm.

### Example 32: Validation of intron retention events identified by RNAseq analysis of IKBKAP

Validation of the intron 7-retention event in the *IKBKAP* (familial dysautonomia) gene was performed using the methods described herein (Figure 38). Briefly, nuclear and cytoplasmic RNA extracts from ARPE-19, HeLa, and U2OS cells were used to perform radioactive reverse transcriptase PCR (RT-PCR) as described in Example 1. In this example, intron retention was assessed using primers positioned in exon 6 and exon 8 leading to the amplification of both intron-7 containing transcript and correctly spliced transcript. The products were run in a 5% polyacrylamide gel and visualized by phosphorimaging. Intron 7 retention levels were calculated as percent intron retention (PIR) of the intensity of the band corresponding to the intron-7 containing product over total transcript (intron-containing plus correctly spliced). Quantification of the bands indicated that approximately 35% of IKBKAP transcripts contain intron 7 and that this product is retained in the nucleus. Moreover, the radioactive RT-PCR results validated the bioinformatic predictions demonstrating that the bioinformatic analysis of the RNAseq results is a powerful tool to identify intron-retention events.

### Example 33: Design of ASO-walk targeting intron 7 and 8 of IKBKAP

An ASO walk was designed to target intron 7 (top panel) or intron 8 (bottom panel) using the method described herein (Figure 39). A region immediately downstream of intron 7 or 8 5' splice site spanning nucleotides +6 to +58 and a region immediately upstream of intron 7 or 8 3' splice site spanning nucleotides -16 to -68 of the intron were targeted with 2'-O-Me RNA, PS backbone, 18-mer ASOs shifted by 5-nucleotide intervals (Figure 39; Table 8, SEQ ID NOS: 329 to 360). These target regions were selected based on the knowledge that intronic regulatory elements concentrate in sequences adjacent to splice sites.

### Example 34: Improved splicing efficiency via ASO-targeting of IKBKAP intron 7 and 8 increases transcript levels

To determine whether we can achieve an increase in *IKBKAP* expression by improving splicing efficiency of *IKBKAP* introns 7 or 8 using ASOs, we used the method described herein (Figure 40). To this end, ARPE-19 cells were mock-transfected, or transfected with each of the targeting ASOs described in Figure 39 and Table 8, SEQ ID NOS: 329 to 360, or a non-targeting SMN-ASO control, independently, using RNAiMAX (RiM) (Invitrogen) delivery reagents. Experiments were performed using 60 nM ASOs (as indicated in Figure 40) for 48 hrs. RT-qPCR results plotted relative to normalized *IKBKAP* PCR product from mock-treated cells show that the IVS7+26 targeting ASO (top graph) and the IVS8+26 and -16 (bottom graph) targeting ASOs increase *IKBKAP* transcript level compared to the mock-transfected or non-targeting ASO (Figure 40). This analysis indicates that these ASOs increase *IKBKAP* transcript level nearly 1.2-1.6 fold (Figure 40). These results indicate that improving the splicing efficiency of rate limiting introns in the *IKBKAP* gene using ASOs leads to an increase in gene expression.

### Example 35: Dose response effect of ASOs targeting IKBKAP introns 7 and 8

To determine a dose-response effect of the IVS7+26 and IVS8-16 ASOs, we used the method described herein (Figure 41). ARPE-19 cells were mock-transfected, or transfected with the IVS7+26 or IVS8-16 ASOs, or a non-targeting SMN-ASO control, independently, at increasing concentrations, or a combination of both ASOs at 45 nM each (total 90 nM) using RNAiMAX (RiM) (Invitrogen) delivery reagents for 72 hrs (Figure 41). Radioactive RT-PCR results show that the IVS7+26 or the IVS8-16 targeting ASOs increase *IKBKAP* transcript level compared to the mock-transfected or non-targeting ASO in a dose-dependent manner (Figure 41). Intensities of the bands corresponding to the *IKBKAP* PCR products from targeting-ASO-transfected cells were normalized to Beta actin and plotted relative to the normalized *IKBKAP* PCR product from mock-treated cells. Results of this analysis indicate the IVS7+26 and the IVS8-16 targeting ASOs, and their combination, increase *IKBKAP* transcript level in a dose-dependent manner 2.0-2.5 fold (Figure 40). These results confirm that improving the splicing efficiency of rate limiting introns in the *IKBKAP* gene using ASOs leads to an increase in gene expression.

### Example 36: Improved splicing efficiency via ASO-targeting of IKBKAP introns 7 or 8 increases protein levels

In order to detect an increase in protein production upon targeting *IKBKAP* intron 7 or 8 with the IVS7+26 ASO or the IVS8-16 ASO, respectively, we used the method described herein (Figure 42). ARPE-19 cells were mock-transfected, or transfected with the IVS7+26 ASO or the IVS8-16 ASO, or a non-targeting SMN-ASO control, independently, at increasing concentrations, or a combination of both ASOs at 45 nM each (total 90 nM) using RNAiMAX (RiM) (Invitrogen) delivery reagents for 72 hrs (Figure 42). Briefly, protein extracts from ARPE-19 treated cells were run on a 4-20% SDS-polyacrylamide gel, and transferred to a nitrocellulose membrane. To evidence an increase in protein production, an anti-IKAP antibody or anti-Beta catenin antibody was used to detect IKAP and Beta catenin as a loading control, respectively. Figure 42 shows western blot results indicating that IKAP is increased in a dose dependent manner upon treatment with the IVS7+26 ASO or the IVS8-16 ASO, or a combination of both ASOs. Intensities of the bands corresponding to the IKAP protein from targeting-ASO-transfected cells were normalized to endogenous Beta catenin and plotted relative to the normalized IKAP protein band from mock-treated cells. Results of this analysis indicate that the targeting ASOs IVS7+26 and IVS8-16 increase IKAP protein level approximately 3 fold (Figure 42). These results demonstrate that promoting splicing efficiency by using ASOs targeted to a region downstream of the 5' splice site of IKBKAP intron 7 or a region upstream of the 3' splice site of IKBKAP intron 8, leads to an increase in target protein production as depicted in Figure 2.

**Table 11: PRPF31 Target Sequences**

| **SEQ ID NO** | **REGION** | **TARGET SEQUENCE** |
|---|---|---|
| 1 | exon 10 | UGGGCUACGAACUGAAGGAUGAGAUCGAGCGCAAAUUCGACAAGUGGCA GGAGCCGCCGCCUGUGAAGCAGGUGAAGCCGCUGCCUGCGCCCCUGGAU GGACAGCGGAAGAAGCGAGGCGGCCG |
| 2 | intron 10 | gggcccuggggguccgguaggcauggggucauggaggggagaagccgg cguccuccucccagccgacucccuggcgccgccca |
| 3 | exon 11 | UACCGCAAGAUGAAGGAGCGGCUGGGGCUGACGGAGAUCCGGAAGCAGG CCAACCGUAUGAGCUUCGGA |
| 4 | exon 12 | UCGAGGAGGACGCCUACCAGGAGGACCUGGGAUUCAGCCUGGGCCACCU GGGCAAGUCGGGCAGUGGGCGUGUGCGGCAGACACAGGUAAACGAGGCC ACCAAGGCCAGGAUCUCCAAGACGCUG |
| 5 | intron 12 | ggccagacccaggugggcugggaccgaggacacaagguggggag cccagaucgcagccucc |
| 6 | exon 13 | GGACCCUGCAGAAGCAGAGCGUCGUAUGGCGGGAAGUCCACCAUCCG CGACCGCUCCUCGGGCACGGCCUCCAGCGUGGCCUUCACCCCACUC |

**Table 12: RB1 Target Sequences**

| **SEQ ID NO** | **REGION** | **TARGET SEQUENCE** |
|---|---|---|
| 7 | exon 24 | AUCUUAGUAUCAAUUGGUGAAUCAUUC |
| 8 | intron 24 | tattttctttctatgaaatataatagtatgcattgtaagtataaaagaa attaaagctttctataatttgaatttccaaatgcagttattcaaacacc tcatccaggcatattgcatagaattttatgagatatatatatctcagat ttactttcaaatcaagtttaatctcaaatcatactcctaattggtgaac ttcaaaactttctaaatatccacttgagattatataatacatatatac atttgtgtatatacatacatatatacgtgagctgtttttgctcacaaca tttctatcaccaaatgtgtgagatttttttctcacccaaatctattctt caactctctggtgttctacaattcaattcaattctgacactaattaccc agagtcagcatcagactccacaggttcaagggctcagtcccacaaaaat ggtctcactgcagacaccagtcacaagtgtcaggtccccaggctacacc acacttccgtctgacttgaatacgaagttgggggttccgatagtgcct cttccttacagtttgatccactgccagaactactcacaaactctggaa aatattctacttactattatcagttcatcataaaagatacaaatgaaca gccagatgaagaaatattatataggtgaggtccagaagagtccctagc acaggggcttctgtccctggggagttggggtgcaccaccttcctagcac ttagacatgtttaccaactccaaagatctcccaaccttattgttgaggg gtttttatgggggtttcattatataggcataattgattaactcaatttc caacccccctcccctccctggatagaggtggggctgaaagttccaagct tctactcaagacttggtctttctggcaaccagcttccatcctaaattag ctaggtacccaccaagtatcacctcattagaacaaaagatggtcccatc acccttatcacacatgaaattcgaagggttttaggagctctgtcccagg aaccagggacaaagaccaaatatctttcaatgataccatgtatgtatgt acataacctcacaggaatctttataaaacaattttgaaattcactcatt atgagtgtgatttgaaatgagatactccaaaatgtaagcccgatatcca aatgtcaccagcctgtccctgcctactggtctccttccatacatatgca ctttttgcttgtccttcctctcagacttctaggatattctttttctggt acactgattaggaattgtttgcatgagatcctgcctcagtgaaagtggc agagcttcattctaggagatccaagggaaagctttgctttgaaacattt |
| | | attctaggctgcaaatccacaaccctagttggccttccattaaagtcac taattcagcagtcccatattcaatatgcattactgttaatatgttgcac catctccattcccctgagagcttatattttaattttaaattttatt tttagagacagtgtctcactctgtcacctacttattataacctcaaact cctcggcccaagcagtcctctcaccttagcctcccaagttgccaggact acaggcatgcaccaccatgtccagctaattttaaatttttgtagaga caggggtttctatgttggccagattggtattgaactcctggcttccacg ataccccgtctcagcctcccaaagaactgggattacagatgtgagccac tgcacctggccagagagcttatattcttataggaatgggaagactgcct atgttatgtgttgctacataatacattacccccaaacttagtgacttaa aacaaacgcttattatctccatttctgtgggtcaataatctaggcatga cttagctgggccagagtttctccaaagtctgtgatcaaggtgtcagttg ggctgggcctgcagtcatctcaaggctccactagaggagcattcactgg cagacttattcaaatggctgttggctgatcctcgatggctattggcccc tctattggtttcttgcccttgggcccctccatagtactgcttgctattc acaacatggcagcttgctttgcccagagcagggactctgaggaggcag ggaaataaagagcaagagagaggtcacagtcttattgtaatctaattct ggaaatgacagcccattacttttggcatattattttggttagaagcaag acaacagtagatctagcccacacacgaggggaggaggatcacacaagga ggtgaataccaggaggtggggtcattgggagccatctgagaggctgccc accacactgcctcaagtaactagggagaggtaaaagtttatatgccaga tgaccaaatattaaaatgtgtgttacaaatagttcacgatgggctcagc tgtcagactttacaaaggagctatgggaccttataaggacagttggaac tggctaggtatcacatagtggtcttcaaacattttgcttgccataacc tctaaaataattgggaaaaagttgaatgtacttccatatcttaaagctg ataatttaaaatattatacatttaatagcagcacggatttagtttttg ttaaattgtatatgtgctccaaatagatttaccatcaaaacctgttttg aatttaatattgggagaattcgctagtttaattttggaaataaagta taattggcaaagctaatcctcactgttgaatctatccgtcaaatcagat ataatttctatcagaaagtctatatgacttgtcaacataataccataa agtgaatcaaaaattattattcattgaacacatcatctcttatcaaatt cttgtgaccttccttctggttgtataatagcctaaaaacaaaaaaagg acaaaagcaagtttccagaaagctgttctgacttgcctacttctgaaaa |
| | | gtagtcctgtatggtgggttctgaaaatgaggaaccaggacttgcagag taggcagttgctggaggaagaatgtgagctgcatgggaaaagacaggag gatttacaaagagtgggtgtttaattggggatggaattaggtagttatt ctgatttttagatttttcatatcttttatttggtccaatgaagcagaaa atttaaatgaagttattacctttgcctgatttttgacacacctcaaact ataacttgaggttgctaactatgaaacactggcatttaatgatttaaag taaagaa |
| 9 | exon 25 | CUUCUGAGAAGUUCCAGAAAAUAAAUCAGAUGGUAUGUAACAGCGACCG UGUGCUCAAAAGAAGUGCUGAAGGAAGCAACCCUCCUAAACCACUGAAA AAACUACGCUUUGAUAUUGAAGGAUCAGAUGAAGCAGAUGG |

**Table 13: HBB Target Sequences**

| **SEQ ID NO** | **REGION** | **TARGET SEQUENCE** |
|---|---|---|
| 10 | exon 1 | AUGGUGCAUCUGACUCCUGAGGAGAAGUCUGCCGUUACUGCCCUGUGGG GCAAGGUGAACGUGGAUGAAGUUGGUGGUGAGGCCCUGGG |
| 11 | intron 1 | tatcaaggttacaagacaggtttaaggagaccaatagaaactgggcatg tggagacagagaagactcttgggtttctgataggcactgactctctctg cctattggtcta |
| 12 | exon 2 | CUGCUGGUGGUCUACCCUUGGACCCAGAGGUUCUUUGAGUCCUUUGGGG AUCUGUCCACUCCUGAUGCUGUUAUGGGCAACCCUAAGGUGAAGGCUCA UGGCAAGAAAGUGCUCGGUGCCUUUAGUGAUGGCCUGGCUCACCUGGAC AACCUCAAGGGCACCUUUGCCACACUGAGUGAGCUGCACUGUGACAAGC UGCACGUGGAUCCUGAGAACUUC |

**Table 14: HBG1/HBG2 Target Sequences**

| **SEQ ID NO** | **REGION** | **TARGET SEQUENCE** |
|---|---|---|
| 13 | exon 1 | ACACUCGCUUCUGGAACGUCUGAGGUUAUCAAUAAGCUCCUAGUCCAGA |
| | | CGCCAUGGGUCAUUUCACAGAGGAGGACAAGGCUACUAUCACAAGCCUG UGGGGCAAGGUGAAUGUGGAAGAUGCUGGAGGAGAAACCCUGGG |
| 14 | intron 1-5' | ctctggtgaccaggacaagggagggaaggaaggaccctgtgcctggcaa aagtccaggtcgcttctcaggatttgtggcaccttctgactgtcaaact gttc |
| 15 | exon 2 | CUCCUGGUUGUCUACCCAUGGACCCAGAGGUUCUUUGACAGCUUUGGCA ACCUGUCCUCUGCCUCUGCCAUCAUGGGCAACCCCAAAGUCAAGGCACA UGGCAAGAAGGUGCUGACUUCCUUGGGAGAUGCCACAAAGCACCUGGAU GAUCUCAAGGGCACCUUUGCCCAGCUGAGUGAACUGCACUGUGACAAGC UGCAUGUGGAUCCUGAGAACUUC |
| 16 | intron 2 | tccaggagatgtttcagccctgttgcctttagtctcgaggcaacttaga caacggagtattgatctgagcacagcagggtgtgagctgtttgaagata ctggggttgggggtgaagaaactgcagaggactaactgggctgagaccc agtggtaatgttttagggcctaaggagtgcctctaaaaatctagatgga caattttgactttgagaaaagagaggtggaaatgaggaaaatgactttt ctttattagattccagtagaaagaactttcatctttccctcatttttgt tgttttaaaacatctatctggaggcaggacaagtatggtcgttaaaaag atgcaggcagaaggcatatattggctcagtcaaagtggggaactttggt ggccaaacatacattgctaaggctattcctatatcagctggacacatat aaaatgctgctaatgcttcattacaaacttatatcctttaattccagat gggggcaaagtatgtccaggggtgaggaacaattgaaacatttggctg gagtagattttgaaagtcagctctgtgtgtgtgtgtgtgtgcgcgcg cgcgtgtgtgtgtgtgtgtcagcgtgtgtttcttttaacgtcttcagcc tacaacatacagggttcatggtggcaagaagatagcaagatttaaatta tggccagtgactagtgcttgaaggggaacaactacctgcatttaatggg aaggcaaaatctcaggctttgagggaagttaacataggcttgattctgg gtggaagcttggtgtgtagttatctggaggccaggctggagctctcagc tcactatgggttcatctttattgtctc |
| 17 | exon 3 | UCCUGGGAAAUGUGCUGGUGACCGUUUUGGCAAUCCAUUUCGGCAAAGA AUUCACCCCUGAGGUGCAGGCUUCCUGGCAGAAGAUGGUGACUGCAGUG GCCAGUGCCCUGUCCUCCAGAUACCAC |

**Table 15: CFTR Target Sequences**

| **SEQ ID NO** | **REGION** | **TARGET SEQUENCE** |
|---|---|---|
| 18 | exon 1 | AAUUGGAAGCAAAUGACAUCACAGCAGGUCAGAGAAAAAGGUUGAGCG GCAGGCACCCAGAGUAGUAGGUCUUUGGCAUUAGGAGCUUGAGCCCAGA CGGCCCUAGCAGGGACCCCAGCGCCCGAGAGACCAUGCAGAGGUCGCCU CUGGAAAAGGCCAGCGUUGUCUCCAAACUUUUUUU |
| 19 | intron 1 | aaggtggccaaccgagcttcggaaagacacgtgcccacgaaagaggagg gcgtgtgtatgggttgggtttggggtaaaggaataagcagtttttaaaa agatgcgctatcattcattgttttgaaagaaaatgtgggtattgtagaa taaaacagaaagcattaagaagagatggaagaatgaactgaagctgatt gaatagagagccacatctacttgcaactgaaaagttagaatctcaagac tcaagtacgctactatgcacttgttttatttcatttttctaagaaacta aaaatacttgttaataagtacctaagtatggtttattggtttcccccct tcatgccttggacacttgattgtcttcttggcacatacaggtgccatgc ctgcatatagtaagtgctcagaaaacatttcttgactgaattcagccaa caaaaattttggggtaggtagaaaatatatgcttaaagtatttattgtt atgagactggatatatctagtatttgtcacaggtaaatgattcttcaaa aattgaaagcaaatttgttgaaatatttattttgaaaaaagttacttca caagctataaattttaaaagccataggaatagataccgaagttatatcc aactgacatttaataaattgtattcatagcctaatgtgatgagccacag aagcttgcaaactttaatgagatttttttaaaatagcatctaagttcgga atcttaggcaaagtgttgttagatgtagcacttcatatttgaagtgttc tttggatattgcatctactttgttcctgttattatactggtgtgaatga atgaataggtactgctctctcttgggacattacttgacacataattacc caatgaataagcatactgaggtatcaaaaaagtcaaatatgttataaat agctcatatatgtgtgtagggggaaggaatttagctttcacatctctc ttatgtttagttctctgcat........ccaaataaggtctgaatgaca caaattttagaactctccagagaaaagaaagatgctgaggaaaaagca taggtttgggactcactaaatcccagttcaattcctttctttaataaat atattcaattttacctgagaaagctctcgtgctctcgaatttatttag aaatttctctttgtacatgattgatttcacaatccttcttctgcctcct cttctactttcttctttctagattttcctatctttatgaagattattct |
| | | gccttatcctcaacagttagaaacaatatttttgaaaatcactacggta tcctgcatagtgatttcccatgccaactttactaatttccattataaat tattatttattgatgcctagagggcagatgagtgtagctgctatggagt gaggagacaaaacataagaaagttatgatcctaccctcaggtaatgatt cagacatgataattaagtcaacaaattgatagaaactaatcactaactc tctggctatagtcattctttcaatgaatagctcattactgagtatgcat gctacagtaacaaaattatataaggctgttgattaaatgttgattaagt gcatgtcttattcagagtttttttatatttgaaatggaagaggctggac ttcagtaatttgctataaactgctagtatatgattatttgggggcagtt atttttaaagaataatttaaatatggaatgtttagcagtttgtttttt ccctgggaaaaaccatactattattccctcccaatccctttgacaaagt gacagtcacattagttcagagatattgatgttttatacaggtgtagcct gtaagagatgaagcctggtatttatagaaattgacttattttattctca tatttacatgtgcataattttccatatgccagaaaagttgaatagtatc agattccaaatctgtatggagaccaaatcaagtgaatatctgttcctc |
| 20 | exon 2 | UGGACCAGACCAAUUUUGAGGAAAGGAUACAGACAGCGCCUGGAAUUGU CAGACAUAUACCAAAUCCCUUCUGUUGAUUCUGCUGACAAUCUAUCUGA AAAAUUGGA |
| 21 | intron 2 | ttcatgtacattgtttagttgaagagagaaattcatattattaattatt tagagaagagaaagcaaacatattataagtttaattcttatatttaaaa ataggagccaagtatggtggctaatgcctgtaatcccaactatttggga ggccaagatgagaggattgcttgagaccaggagtttgataccagcctgg gcaacatagcaagatgttatctctacacaaaataaaaaagttagctggg aatggtagtgcatgcttgtattcccagctactcaggaggctgaagcagg agggttacttgagcccaggagtttgaggttgcagtgagctatgattgtg ccactgcactccagcttgggtgacacagcaaaaccctctctctctaaaa aaaaaaaaaaaaaggaacatctcattttcacactgaaatgttgactgaa atcattaaacaataaaatcataaaagaaaaataatcagtttcctaagaa atgatttttttcctgaaaatacacatttggtttcagagaatttgtct tattagagaccatgagatggattttgtgaaaactaaagtaacaccatta tgaagtaaatcgtgtatatttgctttcaaaacctttatatttgaataca aatgtactccctgggaagtcttaaggtaatggctactggttatcaaaca aatgtaaaaattgtatatttttgagtacctgttacatgccaggtagaat |
| | | atctcctctcagccactctgagtggaaagcatcattatctctattttac agaaaagcaaactgaggctcagagagataatatactttgccagttaatg aatgatggagccatgattccagctgaggtctgtattgccttgctctcta ggaatggtagtccccccataaagaatctctcagtttcctttccaatca aaaggttaggatccttttgattgccagtgacagaaacccaatttactag cttaagtaaataaaaggaac......gcccgccttggcctcccaaagtg ttgggattagtggcgtgagccactgccccggcctattactcctttagag tgatttagagccatgtttacttatggtaacttgacagtaatgggaataa ccactgatgaaacgtaaagcctttgtctaattgtttacctagttcttcc ttgtggttcatgaatttttcatctctgtacagtttgaaattaagatg ataatatttagagatattttattcctttgtgaagagaaaaaggctttc attaacagaaatcagtggcaataacttaataaatacaatcagctggtgt tcctatagtatttaaaagaaaacagaaagtttactagatttcagccagt tttcagactatttaatgtctattcttactataatagaaatatataatt tgatcttgttctcatttttcaaagacctttaatacatgatttagtagt tgaaaatgaagtttaatgatagtttatgcctctacttttaaaaacaaag tctaacagatttttctcatgttaaatcacagaaaaagccacctgacatt ttaacttgtttttgatttgacagtgaaatcttataaatctgccacagtt ctaaaccaataaagatcaaggtataagggaaaaatgtagaatgtttgtg tgtttatttttccaccttgttctaagcacagcaatgagcattcgtaaa agccttactttatttgtccacccttttcattgtttttagaagcccaac acttttctttaacacatacaatgtggccttttcatgaaatcaattccct gcacagtgatatatggcagagcattgaattctgccaaatatctggctga gtgtttggtgttgtatggtctccatgagatttgtctctataatacttg ggttaatctccttggatatacttgtgtgaatcaaactatgttaagggaa ataggacaactaaaatatttgcacatgcaacttattggtcccactt |
| 22 | exon 3 | GAAUGGGAUAGAGAGCUGGCUUCAAAGAAAAAUCCUAAACUCAUUAAUG CCCUUCGGCGAUGUUUUUCUGGAGAUUUAUGUUCUAUGGAAUCUUUUU AUAUUUA |
| 23 | intron 3 | gatctcatttgtacattcattatgtatcacataactatattcattttg tgattatgaaaagactacgaaatctggtgaataggtgtaaaatataaa ggatgaatccaactccaaacactaagaaaccacctaaaactctagtaag gataagtaaaaatcctttggaactaaaatgtcctggaacacgggtggca |
| | | atttacaatctcaatgggctcagcaaaataaattgcttgcttaaaaaat tattttctgttatgattccaaatcacattatcttactagtacatgagat tactggtgcctttatttgctgtattcaacaggagagtgtcaggagaca atgtcagcagaattaggtcaaatgcagctaattacatatatgaatgttt gtaatattttgaaatcatatctgcatggtgaattgtttcaaagaaaaac actaaaaatttaaagtatagcagctttaaatactaaataaataatacta aaaatttaaagttctcttgcaatatatttcttaatatcttacatctca tcagtgtgaaaagttgcacatctgaaaatccaggctttgtggtgtttaa gtgccttgtatgttccccagttgctgtccaatgtgactctgatttatta ttttctacatcatgaaagcattatttgaatccttggttgtaacctataa aaggagacagattcaagacttgtttaatcttcttgttaaagctgtgcac aatatttgctttggggcgtttacttatcatatggattgacttgtgttta tattggtctttatgcctcagggagttaaacagtgtctcccagagaaatg ccatttgtgttacattgcttgaaaaatttcagttcatacaccccatga aaaatacatttaaaacttatcttaacaaagatgagtacacttaggccca gaatgttctctaatgctcttgataatttcctagaagaaatttttctgac ttttgaaataatagatccat.....atttcctctcagggttaccctctg atccctattttactaaatcgttataaaacaaaatgaggaattatgtgtc cttcccttttgaagccaatgtaacaagatgggtaagaattagacctcct gagttcaaaatccctggattcagatctattcctgtatattcaggagaag tggtaataaattcgatggacaatttggtttagtagtcgattgaggaccc tgatgaggtatatttgggaaacataacttccgctctctctcattgact cacgggcctttgaggagtccaggagtcattggaatctggcctgaggttg aggctgctggcaaactccttccccaaagtccattcctattgctgactg agaagggactagcattggaagtggctgattttaaataccgctagtgctg gtgtgctcctccctcccattcccagctctgctttgtgtagttgccttga gaagctaagttcattctgaaaataatgccattgcacaaaacacttttga aagttctagtttgaaattacatcaggtcacttggtctgtgtggcctcag tttcttcatctgccatgtgaaaataataatgcctactctgtagcaaaga aagtctctatagtaaacaaaaaaaaagcctactctgatactgaaagttg ttatgaaaaataaaaaagggaaatgctttagaaactgttaagtgctatg tagatgttactaattaacaaaccatttcagaaactatactttttatttt atggccactattcactgtttaacttaaaatacctcatatgtaaacttgt |
| | | ctcccactgttgctataacaaatcccaagtcttatttcaaagtaccaag atattgaaaatagtgctaagagtttcacatatggtatgaccctctatat aaactcattttaagtctcctctaaagatgaaaagtcttgtgttgaaatt ctcagggtattttatgagaaataaatgaaatttaatttctctgtt |
| 24 | exon 4 | AAGUCACCAAAGCAGUACAGCCUCUCUUACUGGGAAGAAUCAUAGCUUC CUAUGACCCGGAUAACAAGGAGGAACGCUCUAUCGCGAUUUAUCUAGGC AUAGGCUUAUGCCUUCUCUUUAUUGUGAGGACACUGCUCCUACACCCAG CCAUUUUUGGCCUUCAUCACAUUGGAAUGCAGAUGAGAAUAGCUAUGUU UAGUUUGAUUUAUAAG |
| 25 | intron 4-5' | acttccttgcacaggccccatggcacatatattctgtatcgtacatgtt ttaatgtcataaattaggtagtgagctggtacaagtaaggataaatgc tgaaattaatttaatatgcctattaaataaatggcaggaataattaatg ctcttaattatccttgataatttaattgacttaaactgataattattga gtatcttctgtaaactgcctctgttgtagtttttttttctcctaatca tgttatcatttttttggaatccatggtttcctgttaagatgactcacac agcctacataaaagtaattgacaaaatatcatcttatagtaaaatgcca catatctttatgttcagcaagaagagtataatatatgattgttaatgat aacccaaacaacaaaagatttcaccttaactggttgtcataagtagtag tatccaccgccttattttgagttggattttatcatcctatgagcccta caaatttaaagtttttggaacagcacgtgcattgaacccataagaacct actctgcttttctgcatgtattgtccagacaagagaccaaattgccgag gcatcatttaggtgaattctaattaacatttagctaccttacaaccaca attcaaggttgtttcaaaggcatgtgcttgcatcatcctgattcactac catgtgttactaacttggatctgcaaagtcattataaaaagctgttttg atggacttatttggatattgctttacccttcttctcttttcttttat caatgtaaaaacattatatgttaaatacttggcttttaagagcatagat ctgaaatctgcctctagcaaataacccataacacttctaagatatacct gcaaggtcaattgtgttgtaaaaccttgataaccatactttattgttca aaaaagccttttatgaaggcagaagttaaaaaaaaaaacaaaaaaaac agagtccacagttatcacctcagctacaatctcatcagttcacaagtac cagcaaaacatgtgataagtcaacaaatgttttatttcaatctgaacat tttacgtaagtgaagactttgttagatatcatttggaatgtggaatcta cacagttggcatatcagagaaggttgaattcagtttaataaatgtttat |
| | | agaaagtgcttgttatcataatgataatagctcaggatgtgcatgacaa gcttttaagcgattgggtacactatctcatttgatcttctgcacaacta ttaatggtaggtactattatccctatcttatggataagtaaactaagat ttaaaaagtacagaacatggtgtgaacactgcttcaaaatttctaaaat aggtaaatcacgatctctaaactggagggttgtccaaccactagggaca atagagtactgatatttagtggtcagactgtaatgcgggaagagacagg catgggctaaacgggtgtagagatcaaataaggggcaggttagtttgta aacatgtccatatgtaacatttagcacaaatacaggatataggtgcttt cagacccagctgcattgataaaaagttaggtggtattgtatctgtcttc ctttctcaatgttgcatatctgtgttcttgcccagtttgcttcatctct ctagccacacttattggcctacaatggcatcatcaccaaagaaggcaat cccatctccgtgtggctttggtttgctccctaaagtaaaccttgtgttt acttttcccaggtctcatgctttcccatatctgacctgttttgtcctca tggccaggatatgtgggacctttcctacaatgttccaaagtttgtaata gagctcttctctgctttgttccaaattctgcaacattttactttaaata atgaatttaaatacaaacaaacttgagctttgcctatacttttcaagaa tgcagagataactaaattaataaaaatattcattgagtccttactgtgc acacagctctatgttaagccttgtgcagaactcaaagtcactcgagatt aagcctgttactaagttatgtgcaatttagctcagtggatttcccccac ttcatattgctctgataatgttttggaattaactgccttgattccttct tttctctgcttgtctatacactatttattattctacaccatctcaaatt ctaactcctcaagaaaatccttccagatgattttctaaccaggagttt taacttccttttaactaccctattactttctacttccttaactcatcta tcatattatatttagttatttatatactaggtcgccttgaagaagggat tgtgttttcataaatcttaataatccctgaggcatcaagtacagtgatt tgcatttactaaatgctcaacaaatatgtgaggattcacttgaaacta atattagataattcccagtcaaagtgatctaatagcaaatcaattcttc agttttataggcaaagtatgactctggttttccataatcataattaatt tgtcaactttataattttaattaagtaaatttaattggtagataaataa gtagataaaaaataatttacctgcttaactacgtttcatatagcattgc atttttctttgtaaaatttaagaattttgtattaataaactttttaca aaagtattaattattcagttattcatcatatactttattgacttaaaa gtaattttattcaaaagagttagtataggactacatgaaaaattcaagg |
| | | ccaaggcttaatttcaaatttcactgcctttggctctatcttttaaaac aaaacaaaaaactcccgcacaatatcaatgggtatttaagtataatatc attctcattgtgaggagaaaaaataattatttctgcctagatgctggga aataaaacaactagaagcatgccagtataatattgactgttgaaagaaa catttatgaacctgagaagatagtaagctagatgaatagaatataattt tcattacctttacttaataatgaatgcataataactgaattagtcatat tataattttacttataatatatttgtattttgtttgttgaaattatcta acttt |
| 26 | exon 5 | CUUUAAAGCUGUCAAGCCGUGUUCUAGAUAAAAUAAGUAUUGGACAACU UGUUAGUCUCCUUUCCAACAACCUGAACAAAUUUGAU |
| 27 | intron 5 | tacctattgatttaatcttttaggcactattgttataaattatacaact ggaaaggcggagttttcctgggtcagataatagtaattagtggttaagt cttgctcagctctagcttccctattctggaaactaagaaaggtcaattg tatagcagagcaccattctggggtctggtagaaccacccaactcaaagg caccttagcctgttgttaataagattttcaaaacttaattcttatcag accttgcttctttttaaaactttaaatctgttatgtactttggccagat atgatacctgagcaattcttgttctgggttgtcttatgtgaaaaataaa ttcaaggtccttgggacagataatgtgttttatttatctttgcatatcc attacttaaaacagcattggacccacagctggtacaaattaattactg ttgaattgagcaaatatttattctaaatgtctctgtcaaatgacagagt gtggttgtgtggattaagtccctggagagagttctttgttctctcatgt tctatgctgtggttcttgctttatgcaaaaagaagtaagttacttaaaa cctggacatgatacttaagatgtccaatcttgattccactgaataaaaa tatgcttaaaaatgcactgacttgaaatttgtttttgggaaaaccgat tctatgtgtagaatgtttaagcacattgctatgtgctccatgtaatgat tacctagattttagtgtgctcagaaccacgaagtgtttgatcatataag ctccttttacttgctttctttcatatatgattgttagtttctaggggtg gaagatacaatgacacctgtttttgctgt |
| 28 | exon 6 | GACUUGCAUUGGCACAUUUCGUGUGGAUCGCUCCUUUGCAAGUGGCACU CCUCAUGGGGCUAAUCUGGGAGUUGUUACAGGCGUCUGCCUUCUGUGGA CUUGGUUUCCUGAUAGUCCUUGCCCUUUUUCAGGCUGGGCUAGGAGAA UGAUGAUGAAGUA |
| 29 | intron 6 | aacctattttcataacttgaaagttttaaaattatgttttcaaaagc |
| | | ccactttagtaaaaccaggactgctctatgcatagaacagtgatcttca gtgtcattaaattttttttttttttttttttgagacagagtctagat ctgtcacccaggctggagtgcagtggcacgatcttggctcactgcactg caacttctgcctcccaggctcaagcaattctcctgcctcagcctccgga gtagctgggattagaggcgcatgccaccacacccagctaattttttgtat tttagtagagacagggtttcaccaggttgcccaggctggtctcgaatgc ctgacctcaggtgatccgcccacctcggcctcccaaagtactgatatta caggcatgagctaccgcgcccggcctaaaaatactttttaagatggtg taaatattactttctgtatcaatggtacatttttacttgtcagtctct agaatttctttataaatatgttgattcagttcattttgtagattataa aacaggtaaaaaggataaaacatttatgtgaattaaaggaatacctta atttttgtgtagagtttattagcttttactactctggtttatggatcat cacaccagagccttagttactttgtgttacagaataactaatatgagtg aatgaatgacttacacaagtcactgcttaggataaagggcttgagtttg tcagctagagtatgacagaaagtatctaagttttggagtcaaatagcac tttgtttgaatcccagattgcatgcttactagttatgtgaccttagtca agccacttcacctcactgagtctttgctttttcatctctaaaatagag atacccaccgctcataggctgtcataaggatagagatagcatatggaa tgagtctgtacagcgtctggcacataggaggcatttaccaaacagtagt tattattttgttaccatctatttgataataaaataatgcccatctgtt gaataaaagaaatatgacttaaaaccttgagcagttcttaatagataat ttgacttgtttttactattagattgattgattgattga |
| 30 | exon 7 | GAUCAGAGAGCUGGGAAGAUCAGUGAAAGACUUGUGAUUACCUCAGAAA UGAUUGAAAAUAUCCAAUCUGUUAAGGCAUACUGCUGGAAGAAGCAAU GGAAAAAAUGAUUGAAAACUUAAG |
| 31 | intron 7 | ttgttccaataatttcaatattgttagtaattctgtccttaattttta aaaatatgtttatcatggtagacttccacctcatatttgatgtttgtga caatcaaatgattgcatttaagttctgtcaatattcatgcattagttgc acaaattcactttcatgggctgtagttttatgtagttggtccagggtgt tattttatgctgcaagtatattatactgatacgttattaaagaatttcc tacatatgttcactgctgctcaatacatttatttcgttaaaacaattat caagatactgaaggctgattggtaactcacatggaactgggagagtata caattctgaaccaaatagatgattctctattattatatcttaatttatg |
| | | tgttatggtatattaaacatgaaaaaattgtatttggttagaatatgt ttgctcttccttaactcggaatgacataggtaatattcacagattgg gttcctataaatcctccacttgaagtgaagtcagttcaagtaatgaaag ctacctcctgagatagaatcagtacttggcacctatctctagtgttctt tcacctcatataacctttcactgattagtaaagattatatccaacaaag aaagtacagcacagactgagatatgattactgagataaatttgggcaaa atataaactacagcatttctgtagcaatgagaccatttttcttcagttg agctccatgttctacaaacttcaatcaaaaaggttctaggagactcag tgaaagttgatacactgttcaaggaacaaataatttcagcacatgggaa tttcacagggaaaaatatactaaaagagaggtaccattttggatggtg tcaatatgggttatgaggaattcaggctgctgagtccagtgtacaatgg aaactgagctgcaggtgtgtgattgtaacaacaaaagaaatgctgaaat attaagtcctttgccatgtaaatagaaaaagagtatttatttcccaaac attattgctcacctgtttttgttatgcctttcaagataaatccaggaaa ggaattgcattttctttccagaaaacaagttcttgggggaattgttcaa ttggtagatgttgtttttctcattaacaagtgagtgctccatcacactt gctgagtgctccatcacacttgctctctgcattactcctctgcctgcaa acacatatatagcaagggtgatgacaaggatatcagagggtctggtttt ctcaaactcatgataaactcatggctgggtcattcttggtgctgatttt actttgtttttgttgttattgttccctcttcctcaaaagatgaaatct atccctcttacttggaatttctctttgatatatagcgaatgtttggttg taacctgtataatctggcatgaaattgtcactcgaaaaggctagaagtg ttgacataaatatgggacagcaagagttgctcctactcaagagagcaaa tataatgttctggaagagattggcagaattcacatcaaaggagtgatta cttcagcctgggccactgttgtactggtcaaaaggctgtgcaaagctct ctgaaaatccactcttttattgctctttagtaataaagtcactttcaat tttaaaaataacaaactgatatattttatgactcataaaatgttagca attatattatggagaatctactttctgggtgattcttacaaatgttctt ggatctatttttttttcttatagtacctattcttcccatttttctcagc tctagttaatatatttcaacaacagttcaacaatttaacattttata aaaagtgtttcctatcattttataaataccagcctagtccatgttattc cttttcttgttgaggagaaaggacacacattgtaaattcaaatatagac ctctactgtgctatttaatcttggtaacaactccacaaaggagatgaca |
| | | tgttttccttctatagaggtagattctgtaaagttagagggaagagtga cttgcttaagatggcataagctgtaactggcagaaccaggattcaaagc caggtgggatgccaaaatcataatctgtcttcagtgtcaagttactgaa attggtaaacattagacctaaatagacggaattgcaatccgggttgggc acattaaactccatttcttcatcaatgtgctcagattacattttactt ttcaggctaaaaatggaaaaaagagtccctcttagttctgcacttgag aatgagaatagcttttctgaattatacaaggaagaagaactaatgccca aatgccaggtacccacatgcactatgccatggcacagctgttgcccccct ttcaccagagccctctctctgtatcctggttgacctttccttgggcaag agctgggtggggaggatcacaagtgactccaatttggatggcttcggga agactgggaccgagctgaaggcagtgttgtcctctgcactccctgtttt ctgtctgctggagcactgaagcctcacatatgtattaaaaaataattt ccatttgcatttcagactagaagattgaacgtatagtgtaatgtgattg caaataattatattgaaatgagacagagaggatgtagtatctactgtca taatttttcaaaacccacctgcaacttgaattaaaagaaccacttgggt tttttttttgtttcaaacgcaaatcctggaaacctactgagactcatt cagtcagtatctctaagaggcaagcttgagactgtatatttaaaaagca tctcaggtgatttttacacatgctaaggcttaagaaccacttctctgta gcttatatgttattttcaatgttcctcaaagccaagttagaatttccaa agtgttaagaatccattagacaatcacagaattgtctttttcctttata aatcttgcaatgttgttctcatttccatacttaattacttaaaacacca accaaccaacaagcaaaaaatgattagtctaactaatattacaagttaa taatgaagtaaaggtttaaaaataatgtcataataatgttaataacaaa ttattaattataatttaaaaataatatttataatttaaaaataatattt acaagtactacaagcaaaacactggtactttcattgttatcttttcata taaggtaactgaggcccagagagattaaataacatgcccaaggtcacac aggtcatatgatgtggagccaggttaaaaatataggcagaaagactcta gagaccatgctcagatcttccattccaagatccctgatatttgaaaaat aaaataacatcctgaattttattgt |
| 32 | exon 8 | ACAGAACUGAAACUGACUCGGAAGGCAGCCUAUGUGAGAUACUUCAAUA GCUCAGCCUUCUUCUUCUCAGGGUUCUUUGUGGUGUUUUAUCUGUGCU UCCCUAUGCACUAAUCAAAGGAAUCAUCCUCCGGAAAAUAUUCACCACC AUCUCAUUCUGCAUUGUUCUGCGCAUGGCGGUCACUCGGCAAUUUCCCU |
| | | GGGCUGUACAAACAUGGUAUGACUCUCUUGGAGCAAUAAACAAAAUA |
| 33 | intron 8 | gtaccataatgctgcattatatactatgatttaaataatcagtcaatag atcagttctaatgaactttgcaaaaatgtgcgaaaagatagaaaaagaa atttccttcactaggaagttataaaagttgccagctaatactaggaatg ttcaccttaaactttcctagcatttctctggacagtatgatggatgag agtggcattttatgccaaattaccttaaaatcccaataatactgatgta gctagcagctttgagaaattctaaagttttcaagtgataagactcaatt tatacaaagctaattggataaacttgtatatgattaagaagcaaataaa tacttattatgctttttttgctgtttatttaaatatttaacccagaaaat aagtcactgtgacagaaataaaaatgagagagaagggtgagccactctt aggtagttctggcattatttaatctaggccagaggttgcaaatggtgtc ccatagaactaatttggctcctagacctgtcttatttaacctttcatt taaaaaatttgtattggttgccagcaattaaaaattggagatgtctca cacacacacacacataaacacacacactcatgtgtgcagcctcttttga agaattggaataactagtcaactgcgtcctccttttccacaagctgtga cagctccctgctcacagagcacctgccctctcctgttcatcatgctctc ttctcagtcccattccttcattatatcacctatttggtcctgagactaa gtgagtttgagatctgtgatttagacaaagtggtgaatctagctctgaa tcatagtaagtagctctgggaatcatcttgtcttctgttagccattga gagagaaatagagagagagagagagagaaagaaagaagaaacagat ctggggagagtcactgaatgggagcatagagacagagaaacagatctag aaaaccaaactgggagaaaatgagagaaaccaaaagagaggtagagagg agcagagaagaaaatgaagaagcaaggcaaggaccaggcttttcatta tttcttatggccaagacttcagtatgcgtggacttaattcttccttatg ctcctaccttccctagggaaactgatttggagtctctaatagagccctt cttttagaatcacagtttgatgccttaaaactagttatataccttcaca tgcttccttaacccacagaagtgatgctaatgaggcccttaataaggag cgtgctattaagatgaagacattcatttttttctccgtccaatgttgg attaaggcacattagtgggtaattcagggttgctttgtaaattcatcac taaggttagcatgtaatagtacaaggaagaatcagttgtatgttaaatc taatgtataaaaagttttataaaatatcatatgtttagagagtatattt caaatatgatgaatcctagtgcttggcaaattaactttagaacactaat aaaattattttattaagaaataattactatttcattattaaaattcata |
| | | tataagatgtagcacaatgagagtataaagtagatgtaataatgcatta atgctattctgattctataatatgtttttgct |
| 34 | exon 9 | AUUUCUUACAAAAGCAAGAAUAUAAGACAUUGGAAUAUAACUUAACGAC UACAGAAGUAGUGAUGGAGAAUGUAACAGCCUUCUGGGAG |
| 35 | intron 9-5' | aatttttaaaaaattgtttgctctaaacacctaactgttttcttctttg tgaatatggatttcatcctaatggcgaataaaattagaatgatgatata actggtagaactggaaggaggatcactcacttatttctagattaagaa gtagaggaatggccaggtgctcatggttgtaatcccagcactttgggag accaaggcgggtggatcacctgaggtcaggagttcaagaccagcctggc caacatggtaaaacccggtctctactaaaaatacaaaaattaactggg catggtggcagatgctgtagtcccagctgctcgggaggctgaggcagga gaatcacttgaacctgggaggcggaggttgcagtgagctaagatcacgc cactgcactccagcctgggcaacaaggcgagactctgtctgaaaaagaa aaaaaaataaaaataaaaataaaaagaagtggaggaatattaaatgcaa tataaaagctttttttatttttaagtcatacaatttgtttcacataaca gatcaggaaataatacagagatcataagttttggagctgggtttgaatc ctggctctgccatttactttctgtgtaatctaagtcaagttactgaact ttgtgggccctctggctctccatgtgtaaaatggagaatattaatattt accttgcaagtttgttgtgaagactgaaggagagaatttaggtaaaaca ttcatcagagtaccatgcacacagttgttcctcaataaacattagcttc tctgattgcaagttccagtctaaagtgctttatatataccagccaataa aaggatgcgagagagatataccagtgtattgttttctaccattttaaac ctattttcatccactgttacaaattctatcatactgctccacataaaaa atattatcaatgatttttagtctctgaagtgcaatatttgattattgag cacacctgttgaagttttagtttcttctcacttacatgggttgtgtaaa ggtaggaggtataaaaccagtgtcctaggtctaaatctttcttaatgtc atactttggattcattgatataagtaacttgagcaccagcgcttcattt tacttcatttttttaaagatatagtaagagtaattcccatctgcctagca aaattgttttgtagaaaagtttgtggatcagatttattttactttgatt ttaggaatttcaagtgtcttcgtcggcatgaaggaaaaatatgcagttt gacattttctactactttcaggtcattattttcctactctggtgcaaaa accctcaattcctgtctcactccatctaatcaaataggtagcatgcttg |
| | | agcccttactatgtgccaggcactaggataagcactttatatgttttgt cccaattaattctcacagcatttctatgacctaaataaaattaatattt tcatttcaccaataataaaatggaggcttcaaaagttaggacttgg ctcagctcacacaactggcaaggactgaaaatggatttagtcccaaat gtcataggctagagccctttcactaaactgttgtcttccatctggtggc atcctcttcctccagtctttgtcacctaaactctgggcaccccttgatg gcatttacttatgatggtgatgcttgttaaacttcctgtttgcgacttc aacgtccatataaatgagtcttccaatactgtacttagaacttatattt tgtagtgacttctttaaaagctttctctcttagtcatatcctgagtttt gttagcacctggacttaccttactttggaaatgttgcactctgaaatct ctttctcagcttggaatttcctaatcttccaactgtttgagtcttttaa ttctacatttactgcctttccatttcatcaggatttctagtctctttaa ttcttccttttgaactcctcctgatttaacctctgcttattcgaagaac aataattttattctctcagctgcactctcaattccctttttcctttggt gatttttctttttcctacagaacacttactttatcagttttggagaagg aagtgctatctgggtaacagtagtgctatctgttgactctagtcaactg taagttttatacatttattgtttaaaccttatatgggtctataatcctt cttgggaaatcctttcatttgtctttaatttcctttaccatttccctaa aggctattccagattttatcacattcacaaaattcccgtcttttctca ggatctgttcacccccagtagatagccttgtctcccacaatacatggag aaaatagaggccaccgtcatatttgaatgtttccaacttctctcttcac ctttggaattatctttttcttcttttgtgtctaagagaaagatgtatac ttcttcttacccttgtctgaactactctatttgcttcatcttctcaga acaggggaccagcaattattcttcctccagaagcttcaacatcttttgt caactgactccttctcatgtttaaatattttcaagttaaacaatttctt tcctgactttcgctcacgcaacctcatgcccaaaaccttatcactcttc ttccctttgctgtcaaggctgttctcacttcttcacttttgtggactt ctccccactacaacatagattctgctatcaccaatctattaaaactgtt atactcttgtggaatttatcatttaatttagcttcagtgaaccgttctt tccagattattttggcctcagaccatgacttctaagtctgccgtgcttg ccacttaagtgatgatgggccagtgggtccccacctaggcctctgtgtt agtctgttttcatgttgctgataaagacatacccaagaatgggcaattt acagaagaaaggggtttgagggactcacagttccatgtgactggggagg |
| | | cctcacaatcatggtggatgatgaaaggcatgtctcacatggaggcaga taagagcatagaacttgtgcagggaaacttccctttattaaaccaccag gtcttgtgagacttcttcactatcacgagaataggatgggcaagaccct cccccatgattcaattatctcccactgggtccctcccacaacacatggg aattatgggagctataattcaagatgagatttgggtgaggacatagcca aaccatatcagcctccttctggcttttatgttctccgtgggtgacctc tctcaggctcaagtgataaccaatgtgctgatgactctcaaatgcgcat ctctggcttcagtttcttccttgaacttcatacatatgtttccaaattt cctgcgtgtacctcaaggttcttgttcatcacttcccaagcttcataaa cgcactcatttagtgtattctctgtctcctttgatagcatccctgaga ggcaagtccctggtgagttatatacaactcctcccttgctccaaacctg agagtaagtaacattcctattaacatattaggaagctgaggcttagaca gtttaagtaactcaagcatggttacacaactagctagggcagagctaaa atgtcaggctaggcttctgtgactccaaagccctttctcacttagcata tcatcacttattttttttttaatcacatatatgatttttttttcttta agagatagaatcttgctctatcacgtgggctggagtgcagtggcacaat catagctcactgtaaccttgaacttgggctcaagtgatcctcctgcctt agcctactgagtagctagggctacagacacacaccaccatgcctagcta attttattttattttatttatttttgagacagagtctcactctgtca cccaggctggagtgcagtggtgcgatcttggctcactggaacctctgct gcccgggttcaagcgattctcctgcctcagcctcctgagtagctgggat tacaggtgcctgccactgtgcccagctaattttgtatttttagtagag acggggtttcaccatcttggccaggcttgtcttgaactcctgacctcgt gatccactcgcctcggcctcccaaagtgctgggattacaggtgtgagcc accacgcctggccacctacctaattttaatttttttgtagagacaggg tctcactacgttgcccaggctggtcttgaactcctgttctcaaacaatc ctcctgcctcggacaccccaagtgcagggattacaggcatgagtcattg cagctgacctgtatatatgattttagtatatgtaaatatacatattta ttaaatgtaaatataaatataaatgtgtggagtgatatccattgaaatg ttaaacatagttctcagtggtacaactacaggtgatttctcttttctta tttctggttttctgtgttttccaaatttcttgaaatgtgtcttctgtaa tcagaaataaaagttattagtaacaacagtcttccactggtacaagtgc ttattggataaaagtcccacttctaagcatgatactcacaacttttagg |
| | | ttaatagcctttgtcaccttgccatatacatctgatccagccactcaca ccattcctgagatatatttgttcctttgtgcctaaatcattgtgcatg cagatccatcttcctggaacacctataaccatttcttagtcctgtgaaa tcctacttacatccttcatagcctagcatgtatgtcatttatttggtca agggtgagttggttgttctcttgaatgtactgccatatgacgtggtgtg atttcaattgtagcaccaagctcattgcaatattaattcgtttgtcatt ctcccatgtaggatgtttgaagtagtttctaacacagagattatactca ataaatatttattagataaataaatgaataaggaataacaaatgcctt tgtctcattttaaaatactttcattgttagctacccatataataaaaaa ctaaaagcagtagttttcaagcatgattgtttatgtatgccttaaaaga attttgaaaacctatgtacccctgacacacttttaagttaacttataaa tttttcaacatagttttaagtggtggcaaatgatgtagtttcttgtgta ttttaaactgcttaagtatgctatacatggatttcttcaaaccctgaa gctgcagtttcagtgcattcaatttatggaaaagaaattaatttataaa attggttcttattgtcaagtcaatcagctaaatataacttgctttctgt caggaaaagtctgactttaaaatacagataagtaataactattattaat taattaaattattaaaattaaaataattaaataatttgttaattaaaat gccttattcccctacttatttctgcaatttgactctaagaatagatagg acatgtagattgccttaggtttgaaatctgggtgaaataagatactgcc tccttcagtatttctgcctttgcttttatgggagcctctttcaagaaaa agtcattctctcatggtccctttgtttgagtcccagaggttttcctact ccagaaagtgcaacgtagtgagactagtactatactcccttgcatggta agtgagaaggctgtctgtataaaatgagggaaggactcatgagagggaa gtaggtcaggagaaatgataggttctcaggcaggttaattttaggaaag agtgaatagagtcccttaaaacaaggtgcatctgcttcctcctgatcaa tctttaggactgtttactttgatttgaagaccactatgctaaagcttcc cacgggggcaatagtgaggcaaggaatttttaaaagggaattacttctt cgtagctacttttgtgaaatgaattcatttgaattatctggcaatctct tcatatttatattcaacaataattacttaaagaaatgctttgagcttct cagaggagggtgctaccagtgtgatggagtagaattcagatttgggtag tgactttaaagctgtgtgactttagtcatttaactgctgagtcacagtc tacagctttgaaagaggaggattataaaatctatctcatgttaatgctg aagattaaataatagtgtttatgtaccccgcttataggagaagagggtg |
| | | tgtgtgtgtgtgtgtgtgtgtgtgtgtatgtgtatgtatacatgtat gtattcagtctttactgaaattaaaaatctttaacttgataatgggca aatatcttagttttagatcatgtcctctagaaaccgtatgctatataat tatgtactataaagtaataatgtatacagtgtaatggatcatgggccat gtgcttttcaaactaattgtacataaaacaagcatctattgaaaatatc tgacaaactcatcttttatttttgatgtgtgtgtgtgtgtgt |
| 36 | exon 10 | GAUUUGGGGAAUUAUUUGAGAAAGCAAAACAAAACAAUAACAAUAGAAA AACUUCUAAUGGUGAUGACAGCCUCUUCUUCAGUAAUUUCUCACUUCUU GGUACUCCUGUCCUGAAAGAUAUUAAUUUCAAGAUAGAAAGAGGACAGU UGUUGGCGGUUGCUGGAUCCACUGGAGCAGGC |
| 37 | intron 10 | tcttttgttcttcactattaagaacttaatttggtgtccatgtctcttt ttttttctagtttgtagtgctggaaggtattttggagaaattcttaca tgagcattaggagaatgtatgggtgtagtgtcttgtataatagaaattg ttccactgataatttactctagtttttatttcctcatattattttcag tggctttttcttccacatctttatattttgcaccacattcaacactgta tcttgcacatggcgagcattcaataactttattgaataaacaaatcatc cattttatccattcttaaccagaacagacatttttcagagctggtcca ggaaaatcatgacttacattttgccttagtaaccacataaacaaaaggt ctccattttgttaacattacaattttcagaatagatttagatttgctt atgatatattataaggaaaaattatttagtgggatagtttttgaggaa atacataggaatgttaatttattcagtggtcatcctcttctccatatcc caccctaagaacaacttaacctggcatatttggagatacatctgaaaaa atagtagattagaaagaaaaaacagcaaaaggaccaaaactttattgtc aggagaagactttgtagtgatcttcaagaatataacccattgtgtagat aatggtaaaaacttgctctcttttaactattgaggaaataaatttaaag acatgaaagaatcaaattagagatgagaaagagctttctagtattagaa tgggctaaagggcaataggtatttgcttcagaagtctataaaatggttc cttgttcccatttgattgtcattttagctgtggtactttgtagaaatgt gagaaaaagtttagtggtctcttgaagcttttcaaatactttctagaa ttataccgaataatctaagacaaacagaaaaagaaagagaggaaggaag aaagaaggaaatgaggaaga.....gaggctgaggcaggagaatggcgt gaacccaggaggcagaacttgcagtgagccgagatcgcgccactgcact ctagcctgggtgacagagtgagactctgtctctaaataaataaataaat |
| | | aaataaataaataaataaaatcagtgctttttcttcctctgctacctcc tttccttctactcagttttagtcagtagtattatctttttttcagattta tctttgtattgttaaatctgcttatgcttctattactttatttattagc tttaaatgataccttttgactttcagcttttcttaataaagcaatcagc aaatttcctttacactccacacttataccccatttcctttgtttgttta tttggtttttacttctaacttttcttattgtcaggacatataacatatt taaactttgtttttcaactcgaattctgccattagttttaatttttgtt cacagttatataaatctttgttcactgatagtccttttgtactatcatc tcttaaatgactttatactccaagaaaggctcatgggaacaatattacc tgaatatgtctctattacttaatctgtacctaataatatgaaggtaatc tactttgtaggatttctgtgaagattaaataaattaatatagttaaagc acatagaacagcactcgacacagagtgagcacttggcaactgttagctg ttactaacctttcccattcttcctccaaacctattccaactatctgaat catgtgccccttctctgtgaacctctatcataatacttgtcacactgta ttgtaattgtctcttttactttcccttgtatcttttgtgcatagcagag tacctgaaacaggaagtattttaaatattttgaatcaaatgagttaata gaatctttacaaataagaatatacacttctgcttaggatgataattgga ggcaagtgaatcctgagcgtgatttgataatgacctaataatgat |
| 38 | exon 11 | CUUCACUUCUAAUGGUGAUUAUGGGAGAACUGGAGCCUUCAGAGGUAA AAUUAAGCACAGUGGAAGAAUUUCAUUCUGUUCUCAGUUUUCCUGGAUU AUGCCUGGCACCAUUAAAGAAAAUAUCAUCUUUGGUGUUUCCUAUGAUG AAUAUAGAUACAGAAGCGUCAUCAAAGCAUGCCAACUAGAA |
| 39 | intron 11 | aaactatgtgaaaactttttgattatgcatatgaacccttcacactacc caaattatatatttggctccatattcaatcggttagtctacatatattt atgtttcctctatgggtaagctactgtgaatggatcaattaataaaaca catgacctatgctttaagaagcttgcaaacacatgaaataaatgcaatt tattttttaaataatgggttcatttgatcacaataaatgcattttatga aatggtgagaattttgttcactcattagtgagacaaacgtcctcaatgg ttatttatatggcatgcatataagtgatatgtggtatctttttaaaaga taccacaaaatatgcatctttaaaaatatactccaaaaattattaagat tattttaataattttaataatactatagcctaatggaatgagcattgat ctgccagcagagaattagagggtaaaattgtgaagatattgtatccct ggctttgaacaaataccatataacttctagtgactgcaattctttgatg |
| | | cagaggcaaaatgaagatgatgtcattactcatttcacaacaatattgg agaatgagctaattatctgaaaattacatgaagtattccaagagaaacc agtatatggatcttgtgctgttcactatgtaaattgtgtgatggtgggt tcagtagttattgctgtaaatgttagggcagggaatatgttactatgaa gtttattgacagtatactccaaatagtgtttgtgattcaaaagcaatat ctttgatagttggcatttgcaattcctttatataatcttttatgaaaaa aattgcagagaaagtaaaatgtagcttaaaatacagtatccaaaaaaat ggaaaagggcaaaccgtggattagatagaaatggcaattcttataaaaa gggttgcatgcttacatgaatggctttccatgtatatactcagtcattc aacagtttttttttagagc.....gaggaggtggaaacgaatgtacaa ggatgggaggagaaaggagagagactttttttttttaaggcgagag tttactacctatctaactcttcgcattcttgaagtctcagaccaaatcc catcggtttgaaagcctctagggtattctatctattgtatacttctgtt atgtacaaaattaatttgccaattaattgtgaactgttttataaactat cttaaaatggttagttaaatctttgggatagtatttagctttctccagg attatgacttaccttctaaattagacatacaatgcctaggagtcaagga ctattttgcataaattccagtcttcttttacaatgcctagaatgattgt taccacagaaatattcattacctgggagaaaggatgacaggaggggcag aatgaatggagagaggtcgtgagaatgaggtgctgaggatggacgagga agaaagctgttttagttgggaggataggtgacagaagcatggaaaggaa ttgccttggacccatggaagcccagtgaagatacttagatcctgcaggg gtgtgaataatgttcttttagtttctcttcttaggaggtttgttcattt tgggagatttcttttgaaaagagtgaacttaaattggagaaaagtacat tttagtatgttgataacatttgaatttgtaaaatggacctatggatgat ctacacatatttatatacccataaatatacacatattttaatttttggt attttataattattatttaatgatcattcatgacattttaaaaattaca gaaaaatttacatctaaaatttcagcaatgttgtttttgaccaactaaa taaattgcatttgaaataatggagatgcaatgttcaaaatttcaactgt ggttaaagcaatagtgtgatatatgattacattagaaggaagatgtgcc tttcaaattcagattgagcatactaaaagtgactctctaattttc |
| 40 | exon 12 | ACAUCUCCAAGUUUGCAGAGAAAGACAAUAUAGUUCUUGGAGAAGGUGG AAUCACACUGAGUGGAGGUCAACGAGCAAGAAUUUCUUUAGC |
| 41 | intron 12-5' | taactaattattggtctagcaagcatttgctgtaaatgtcattcatgta |
| | | aaaaaattacagacatttctctattgctttatattctgtttctggaatt gaaaaaatcctggggtttatggctagtgggttaagaatcacatttaag aactataaataatggtatagtatccagatttggtagagattatggttac tcagaatctgtgcccgtatcttggtgtcagtgtatttgtttgcctcata gtatagtttactacaaatggaaaactctaggattctgcataatactgga cagagaagatgtaaatatctgttagttccatcatagaccctgccactcc aatgtacacaccagctttaggcttcttggtatagataaacatacatttt caaaattttcatcataattttcataacaaataggaaggcaaatgatg tcacttggcttaaaatctataatatttaaaataaacaggacaaatgcat taacattgttgggggaggaggtcccttagtagaaacactcttggtccaa gcattttaaagctgtcaaagagatgtaaatatagataatgtatgtcaag gagagagctttgtggttaaactgtaactttcagtttaaacaattattgg tgactctgatgtcaaatgtttctcaagctttatctgaacaaattcttc tcactttgttgccaaagtcgttaacaagaaatcacattgactcattgat gttttggctcctttcccttactttctgttgctttccaaaagctgagaca ggaaactaaccctaactgagcacctgcaattgcctggtagtattctagt catgtgtgtactttgtgtgtatgtaatccccttacagctctgcaaagt aagaattgttctccctgctttacagaagagatcataagataattgaggc tgttagatgttaacttgccaaaagccatacaggaaaatggtagagtcac agtttgaaccaggtccttttgattctttacattaaaccatgctttgatc ttggaaatacactgtaaggcaataaatcaatagatacggataattcaca ggcttctaaataaatggaagttgattgtttttatctgtgagccaaagta agacttattctaagaattccacaaatttagataagatagagtatatggc ttctagacatccaacatagaactgagtttgtgttatcagtttaagattt ggttttgctgtaaggtgcacacactttgaggaactaaaaataattgtct gttcttattctgatcagaatgtgtaatgtgttgtccagttttggatgat gaatttcttatttctaatctcataagaaacttgtcatagatgtgaggga gagaattaagaacagagtgtgggaagaaactgtgtacattttgatggg atccattatgtagctcttgcatactgtcttcaaaaataagttacactat aaaggttgttttagacttttaaagttttgccattggtttttaaaaaaat ttttaaattggctttaaaaatttcttaattgtgtgctgaatacaatttt ctttattacagaagtaccaacaattacatgtataaacagagaatcctat gtacttgagatataagtaaggttactatcaatcacacctgaaaattta |
| | | aatgttatgaagaaattatctcatttctattaatatgggaactgtgtct tcatctttattactgttctaaggtcaactcaatgtagattttacttgct tatggtttcatattttagctaaatagtaaaataatatggatatacattt tgttgtgacttactcatactttccttatttggaacttttatgaatatga tatagagactgaaactacaaggaacaaaatgcaatatcaattatacagt tgtggcagcactgctatcaatttgttgatagtggttaacacttagaaaa acattttaaaaataatttcacataagtaatgtaatttattagctgtctc tgacattttacagtttggaatagtttattttctttttggtgtcctcacc aaaacccaacatcttcaagggcaggaactgtataatttttgccattgta ttttgagcacatagcatggtacttgcctctaaatagatactattgttaa aatatttttaaggtaatattttaaagtgtatgctatggtacagttcag tttgtgacttttgctagtttatgccacttacagttagcaaaatcacttc agcagttcttggaatgttgtgaaaagtgataaaaatcttctgcaactta ttcctttattcctcatttaaaataatctaccatagtaaaaacatgtata aaagtgctacttctgcaccacttttgagaatagtgttatttcagtgaat cgatgtggtgaccatattgtaatgcatgtagtgaactgtttaaggcaaa tcatctacactagatgaccaggaaatagagaggaaatgtaatttaattt |
| 42 | exon 13 | GCAGUAUACAAAGAUGCUGAUUUGUAUUUAUUAGACUCUCCUUUUGGAU ACCUAGAUGUUUAACAGAAAAGAAAUAUUUGA |
| 43 | intron 13 | ttctttgaataccttacttataatgctcatgctaaaataaaagaaagac agactgtcccatcatagattgcattttacctcttgagaaatatgttcac cattgttggtatggcagaatgtagcatggtattaactcaaatctgatct gccctactgggccaggattcaagattacttccattaaaacctttctca ccgcctcatgctaaaccagtttctctcattgctatactgttatagcaat tgctatctatgtagtttttgcagtatcattgccttgtgatatatattac tttaattattattatacttaacattttatttactttttgtgttagtat tttattctgtcttctccttagatagtaaccttcttaagaaaatatatat gctaagtgttttactggtttaatatgcttagactactcatctacctcaa tacttccttggagatctcctcctcagtcacacagagctcaggacttata tttccttggaactcctgttagggtccaatgtacatgaaattccctagac agacagacagtcagttatatggcttgatttcaaagtttcaaaatgattt aatggactatcaagtagtttattaggagaacagttattatactcttcta aaaataaagactttaagcaataaagatgtatatgtatataaaatggctg |
| | | ggttattcctagaagtacctttcttagaatttagttaaatttaatatcc aagatactatcttttcaaccctgagattgtgaaaagtaacttctatcaa tataaactttactacatttgtattgtgttagtgtgttacagtataatct agaacaatgtgtctttctatatgatatatgacattttaatgcctaaaaa aactgatatgtcttagatgattctagtcaggatttacttctagaataga ttaaaattctatttgaggagagtcaaattaattatcgaattctcagttg ttattattgctgttttatttttagtgaaacagattagtcttaatgtaaa cacttgagaaataaattgatggtcaacctaaaatgtaaaaagaaatta atagaaaatttaaagagcaacaaagctctgacatttaaaagaaatgaag tacaaatctctagggaccttaaagatcatctaataatttcctcatttc tagataaataaactgagagaccccgaggataaatgatttgctcaaagtc aaatatctacttaatataggaaatttaatttcattctcagtctgttaac atgcaacttttcaatatagcatgttatttcatgctatcagaattcacaa ggtaccaatttaattactacagagtacttatagaatcatttaaaatata ataaaattgtatgatagagattatatgcaataaaacattaacaaaatgc taaaatacgagacatattgcaataaagtatttataaaattgatatttat atgt |
| 44 | exon 14 | UGUGUCUGUAAACUGAUGGCUAACAAAACUAGGAUUUGGUCACUUCUA AAAUGGAACAUUUAAAGAAAGCUGACAAAAUAUUAAUUUUGCAUGAAGG UAGCAGCUAUUUUAUGGGACAUUUCAGAACUCCAAAAUCUACAGCCA GACUUUAGCUCAAAACUCAUGGGAUGUGAUUCUUUCGACCAAUUUAGUG CAGAAAGAAGAAAUUCAAUCCUAACUGAGACCUUACACCGUUUCUCAUU AGAAGGAGAUGCUCCUGUCUCCUGGACAGAAACAAAAAAACAAUCUUUU AAACAGACUGGAGAGUUUGGGGAAAAAGGAAGAAUUCUAUUCUCAAUC CAAUCAACUCUAUACGAAAAUUUUCCAUUGUGCAAAAGACUCCCUUACA AAUGAAUGGCAUCGAAGAGGAUUCUGAUGAGCCUUUAGAGAGAAGGCUG UCCUUAGUACCAGAUUCUGAGCAGGGAGAGGCGAUACUGCCUCGCAUCA GCGUGAUCAGCACUGGCCCCACGCUUCAGGCACGAAGGAGGCAGUCUGU CCUGAACCUGAUGACACACUCAGUUAACCAAGGUCAGAACAUUCACCGA AAGACAACAGCAUCCACACGAAAAGUGUCACUGGCCCCUCAGGCAAACU UGACUGAACUGGAUAUAUAUUCAAGAAGGUUAUCUCAAGAAACUGGCUU GGAAAUAAGUGAAGAAAUUAACGAAGAAGACUUA |
| 45 | intron 14 | tatacatcgcttgggggtatttcaccccacagaatgcaattgagtagaa |
| | | tgcaatatgtagcatgtaacaaatttactaaatcataggattaggat aaggtgtatcttaaaactcagaaagtatgaagttcattaattatacaag caacgttaaaatgtaaaataacaaatgatttcttttgcaatggacata tctcttcccataaaatgggaaaggatttagttttggtcctctactaag ccagtgataactgtgactataagttagaaagcatttgctttattaccat cttgaaccctctgtgggaagaggtgcagtataaataactgtataaataa atagtagctttcattatttatagctcgcaaaataatctgtatggaagta gcatatataaggtatataaacatttagcctcttgataggactaactcac attctggtttgtatatcagtcttgcctgaatttagctagtgtgggcttt tttttatcttgtgagtttgctttatacattgggtttctgaaaagatttc ttttagagaatgtatataagcttaacatgtactagtgccaatcttcaga cagaaattttgttctattaggttttaagaataaaagcattttattttta aaacaggaaataatataaaaaggagagtttttgttgttttagtagaaaa cttaatgccttggatgaaatgagccatgggcaggttgtaatgaattga tatgtttaatagtatagatcatttgtgaataatatgacctttgacaaga cacaagccattaacatctgtaggcagaagtttccttctttgtaaaatga gggaataaaatagatccctaaagtgtgtaatttagtatttctaaactt tatgaaggtttcctaaatgataattcatctatatagtgtttttttgtgt gtttgtttgtttgtttgtttgagatggagtctcgctctgtcacctaggc tggagtgcaatggtgcaacctcggctcactgcaacctctgcctcctggg ttcaagctaatctcctgcctcagcctcctgagtagctgagattacaggc atgcaccaccatgccgagctaattttgtatttttagtagagaaggggt ttcatcatgttgaccaggctggtcttgaactcctgaccttgtgatccac ccacctcagcctcccaaagtgctggtattacaggcgtgtgccaccacgt ccagcctgagccactgcgcccagcccatctatatagtttaatatcaatc taaatgaatttctcagtcctgagcctaaaaatttagttgtaaagaatga tatccttgactaataatagtttctattaatggattgcatctagtgctag gtggcatatatttagtccccacaactaccctggaaggtatttaaaattt ttcacatttgcagataaggaaactaaagttcagagttcggcaacatgct tgaattcaagcagctcctaggatgttaatggtggaggttgggttcaaat ccagatctgtctgactcaaaaaatgcatactcctaaccagtgcactata tcccaattccataggagcccttctttgtgattcatagcactttcccatg agttttgttgattttgtgagaaacaaaactctttttcctttggactgtc |
| | | tggaatctctctttttcaaatttttgaaatgtatttctatgccaaaaga caaagatttctagaggaatatgcctaggatgagaattatgtaatttaaa tcacagctggaaagagagaaagtcctaagttactaagaaatgttcaaac acaaatgagctttcagtctattggaagacctttatagctagaagtatac tgaactgtacttgtccatggacccctgaagaaacaggttaaatcaaaga gagttctgggaaacttcatttagatggtatcattcatttgataaaaggt atgccactgttaagcctttaatggtaaaattgtccaataataatacagt tatataatcagtgatacatttttagaattttgaaaaattacgatgtttc tcatttttaataaagctgtgttgctccagtagacattattctggctata gaatgacatcatacatggcatttataatgatttatatttgttaaaatac acttagattcaagtaatactattcttttattttcatatattaaaaataa aaccacaatggtggcatgaaactgtactgtcttattgtaatagccat |
| 46 | exon 15 | AGUGCUUUUUGAUGAUAUGGAGAGCAUACCAGCAGUGACUACAUGGAA CACAUACCUUCGAUAUAUUACUGUCCACAAGAGCUUAAUUUUUGUGCUA AUUUGGUGCUUAGUAAUUUUUCUGGCA |
| 47 | intron 15 | aatgttctattgtaaagtattactggatttaaagttaaattaagatagt ttggggatgtatacatatatatgcacacacataaatatgtatatataca catgtatacatgtataagtatgcatatatacacacatatatcactatat gtatatatgtatatattacatatatttgtgattttacagtatataatgg tatagattcatatagttcttagcttctgaaaaatcaacaagtagaacca ctactgatattttattatttcatattacatataaaatatatttaaatac aaatataagaagagtttttaatagatttttaataataaaggttaagaga ttcgaaagctcaaagtagaaggcttttatttggattgaaattaaacaat tagaatcactgttgatattttattatttcatattacatataaaatatat ttaaatataaagataagagtttttaatagattttataataaatgttaag agattaaaaaactgaaaatagaaggcttttatttggattgaaattaaag gccaggcatggtggttcatgcctgtaatcccagaattttaggagactga gtggggaggattgcttgagcccaggggtcaagaccagcctgggcaacac agtgagacaccgtatctacaaaataattaaaaaattagctgggcatggt ggtgtgtgcctgtatgctaccattaactaaggaggctgaggtgggagaa tcgcttgagcctgggaggtcaaggctgccctgaactgtgattgtgccat tgcattccagcctgggtgccagagagagaccctatctctaaataaataa ataagtaaataaataaacagcaacaacaaaaacactcaagcaaatctg |
| | | tactaaattttgaattcattctgagaggtgacagcatgctggcagtcct ggcagccctcgctcactctcagggcctccttgaccttgacgcccactct ggctgtgcgtgaggagccct.....tagaacagagcacagatgatctaa atataaaaagaactacaaaatcacagttgtttaaaaggtttttgtt tgtttatatatggtgcagaacatttgttccttagccaaatgtttccacc ttgagaaagctatagagattctatgtagtcctagtaccaataatatgtt ttaacctgaatgtaccttatctttattcataaactgtgactttttacac tgctgaaacttttttttttaagacaatctcactctgtcgtccagtctgg agtgcagcagtggtgtgatcttggctcactgcaacctctaccttctgtg ttcaagcaattctggtgcctcggccacctgagtagttgggatcacaggt gtacaccaccaggcctggctaatagtttttgatatttctagtagagatg agttttgccacattggccaggctggcctgaaactcctggcctcaagtga tctgcctgccttggcctcccaaagtgttggtattacaagtgtgagccac tgtgcctggcctgaaactcataattcatttccattaatattaatctcac cttttccaataattaattgatttcacaagtattagtcccctataatcat tgaatggctaataaaattatttatagcaaacagattaattatctgccag cagtctgagattagtttctttaaaaaatgtttattatttaaacattca gctgtgatcttggctttcttgtgaggttcaatagtttctattgagtaaa ggagagaaatggcagagaatttacttcagtgaaatttgaattccattaa cttaatgtggtctcatcacaaataatagtacttagaacacctagtacag ctgctggacccaggaacacaaagcaaaggaagatgaaattgtgtgtacc ttgatattggtacacacatcaaatggtgtgatgtgaatttagatgtggg catgggaggaataggtgaagatgttagaaaaaaaatcaactgtgt |
| 48 | exon 16 | UGGCUGCUUCUUUGGUUGUGCUGUGGCUCCUUGG |
| 49 | intron 16 | tattccatgtcctattgtgtagattgtgttttatttctgttgattaaat attgtaatccactatgtttgtatgtattgtaatccactttgtttcattt ctcccaagcattatggtagtggaaagataaggtttttttgtttaaatgat gaccattagttgggtgaggtgacacattcctgtagtcctagctcctcca caggctgacgcaggaggatcacttgagcccaggagttcagggctgtagt gttgtatcattgtgagtagccaccgcactccagcctggacaatatagtg agatcctatatctaaaataaaataaaatgaataaattgtgagca tgtgcagctcctgcagtttctaaagaatatagttctgttcagtttctgt gaaacacaataaaaatatttgaaataacattacatatttagggttttct |
| | | tcaaatttttaatttaataaagaacaactcaatctctatcaatagtga gaaaacatatctattttcttgcaataatagtatgattttgaggttaagg gtgcatgctcttctaatgcaaaatattgtatttatttagactcaagttt agttccatttacatgtattggaaattcagtaagtaactttggctgccaa ataacgatttc |
| 50 | exon 17 | ACUCCUCUUCAAGACAAAGGAAUAGUACUCAUAGUAGAAAUAACAGCU AUGCAGUGAUUAUCACCAGCACCAGUUCGUAUUAUGUGUUUUACAUUUA CGUGGGAGUAGCCGACACUUUGCUUGCUAUGGGAUUCUUCAGAGGUCUA CCACUGGUGCAUACUCUAAUCACAGUGUCGAAAAUUUUACACCACAAAA UGUUACAUUCUGUUCUUCAAGCACCUAUGUCAACCCUCAACACGUUGAA AG |
| 51 | intron 17 | ttactaggtctaagaaatgaaactgctgatccaccatcaatagggcctg tggttttgttggttttctaatggcagtgctggcttttgcacagaggcat gtgccctttgttgaacctccatttgactggcatgcacatgtctcagata ttataggttatcatatattgttgctcctaatatttctgtgttagataat tagagtagcttggtttgtaagaatgtgatgttggtgggactgtagcaga acaagaaggcccttatgggtcagtcatacctctcttttcaaatatttgg tctagctctcttctgggcatcttgttgccaatatatagtattgctcaaa agggcaggagatttgaagtgatcaaggaaaatatatttttctattgat taagtcttttgatggggtagaataatctaatttcatgtaactgctcaaa gttatatggtagggggatcccaaatgtattttaaaactattttatatc atcatatttgaagtaatagaaagtcagagtagcagaataaaggtactaa aaattttaaaactaataaggtactttgaaagaaatcaattatgttgat tcctcattaaacaaatttgcacttaaagactgaggttaataaggatttc cccaagtttttcatagcaacctgtgagcactttctctgttgaggcatt tatggtatgaaaagatgagtaaggcacagttcttgccctggagaaggtc acaggtgagaggaggagttgacacagaaacatttgatataaagcaagga ataaattccaagactaaaattttcagaaatctaaaaactcaagataag aaaaacccattatattttctgggtaacaaaatttcagtgttattaacat gtaggaagatcttgatatttattctgaagcccatgtgtgttgctgaaat attgccgcatttgcatatactcatcaccatcctctgttttggagctaag aattttagactcaagatgtctaattaagttgatccattgattttatttt ttatggaaatctgagacccacagaaggcaggggatttgcccacatttct |
| | | agaagagtcagacatgagcgatgaggcacagtggaaagaacatgagcat tgcctgagctctgagttggcgctataagagcagtgatcatgggcaagtg actcttctgagccttggcctcctcacctgttaagtgaagaaagaatat ttcagaagatctttgtgagaatgaaacaaggcaatttacttgcctgcta catagccaatgggaaatcaatataagttccccgtggttcccttctgtgg ggttttgttcccacagaggtgcactggccattccacttcttcttttcc aagctcctcattccctttaacgctgttcatagttggttccaaaccattt gaaatataataagcaccaggatggtttttctttccaccaaagcaaatt tcattttctaaacactgtttataaatatcaatggctatttttttcaattt ttgattatcatgaaaatatacaaatatgtttaattaaatatgctaaaga atgtattaataaatatgtattaaataattcctacatataaggcctttttt gcttggggtatgggtgatacaaaataaatgtggcatgaacccactgacc tctagcaatttataacctagaaaaagagttatgatatgtttataagttc ctgtgatataagacatgcatatagtcattataacagaggtgcaaacaag atgtatcaagtatgtccagaggaggaagagattaatcccagctggagga aacactgatgctttcttgcagcaggggcatttgagttgagaaaggagg aaacatagattttgacaatgagagctgaggggaaaggggtttcaggtgg agggaaccgcatgtggaaagcagggaggtaggaaagtgtagagtgtgtt taaagaatagaccagtttggctgaaacaggatatttgagcagaggaagc ttgtactaggtaggtgggttgaggccaaattatgcaaggcattaaatat taaactaggaattttggactttatcctgcagtttatgggggtaaatga taagattcaatatcactttatttgtacagtattatgttacattttatct aattgtttgtttaattcctgtctagacaatgaattcctcaagggcaagg agcatggcttattcacctcagtaatttcagtgcctagcattgtgcctgg tacaaagtggacacttgtatataacctttttttaattgaagcaacaagtt gtcaaccttacaaatgtgaatccgtgattcagatgacaggttgaaatgt agattgtctgcgaagagggcagaaagagagtatgacaaaggaggacaag acagtggggcaggcagggagagagagcagccagggtttcggtagaggta tgtcaaaaaggtatggaagtcagaggagaaggagacccctatgttatag aatacaaatggaagggaaatgatgacaacagtaagttgtcattaaatgc aaggttgcaaaagtaagattgtaaagcaggatgagtacccacctattcc tgacataatttatagtaaaagctatttcagagaaattggtcgttacttg aatcttacaagaatctgaaacttttaaaaaggtttaaaagtaaaagaca |
| | | ataacttgaacacataattatttagaatgtttggaaagaaacaaaaatt tctaagtctatctgattctatttgctaattcttatttgggttctgaatg cgtctactgtgatccaaacttagtattgaatatattgatatatctttaa aaaattagtgtttttttgaggaatttgtca |
| 52 | exon 18 | UGGGAUUCUUAAUAGAUUCUCCAAAGAUAUAGCAAUUUUGGAUGACCUU CUGCCUCUUACCAUAUUUGACUUCAUC |
| 53 | intron 18 | taaaaataagtaccgttaagtatgtctgtattattaaaaaaacaataac aaaagcaaatgtgattttgttttcatttttatttgattgagggttgaa gtcctgtctattgcattaattttgtaattatccaaagccttcaaaatag acataagtttagtaaattcaataataagtcagaactgcttacctggccc aaacctgaggcaatcccacatttagatgtaatagctgtctacttgggag tgatttgagaggcacaaaggaccatctttcccaaaatcactggccacaa agtgtgacattttggcattggcatcactatttgatggaagccaacctcc ccccaaaaggcctgtattagaatgaagatggattccctgggtgggttac acttgaaactagcctcacccatgaacactttggcacagattagctagcc cattcccccacagtaaggaccataaggaagggacagaagcaaagataag ttttagaacaaaagagagggaaagaaaaaatctagggttttatgaggg ctgtccctgagtgatagatgtgaataggcctccagggcaggctggctca gaggctgactctttgggttggggtgactgattggtggtgaggatggaga agaaaaggggagtggaggaggtgaaagtgaccttgggacattaggtctc cataagtgacaggatttaaggagtgttgtaagctgtggttgttggacca ggtttaagcacagcttcctgagcttcctgactggtttaggtcaagctcc agagagcaaatgccacagtctcagtgatctccttggagaaacagttgga ataggatgttgcccatgttgggatgagtcattgtccgctcttgctcttt ccctacccctgcaaataataatactgtatttgattgaacatataaaac aaaagaaggattatcacataagtatgtatatataaccaacattggcagg tgcagaaaaaccagactgtcagtttgcctcatctgaaatgattgacaca aacaaatatatttactgtcccaagtgaactttggcattttggatatcct tcagttgttctgtttaaagatataacttagaagcagctgatggaatatt taaatccatgcgttgaattcatgcattcaaagaaacatgtcctgagtca ctaaatgctgacatttgtttttcatgttaagagtgtaaataactggtcc caaatataatattattacatcagataaaaactggaatgtgaacctctta acttgattgtgaaagtatttgccaatggtgcctcttgataattatttga |
| | | ggctcacttcagaactcctctggaagggttaattttaaatagtcattt tataaattaacattttgacatatgtgatggctctcaaatttttcttt tatgccagtttgaatcatttctgctcaatttttttttaattgggatg gagtctcactctgttgcccaggctggagtgcagtgatgcaatcttggct gactgcaacctccacctcctcggttcaagcgattctctcgcatcagcct ccagagtagctgggattacaggcgcaccaccatgcctggataatttt tgtattattactagagatggggtttcaccacgttggccaggctggtctt gaactcctgaactcctgacctcaagtgatccacctgcctcagcctctta aagagctggaattataggtgtgagccactgcaccaggccctgttcaact tttaatgctaagattcatttgttgtttcacaagtgattaggcagag gtcttttatattaatttacccattttatttgtaagagagtctcatatta aggaagcataatatatgacaatccaaatacagtacaaatttggttaatt ttgattttgttaaataattaatcacaggggtccttcaaattgtgagctc ctctggttatacttatgttttacctctggttatacttaatttcaaacaa atgaaatttcattctattcatgatatttcagaagcagatctgttgcaca aaataaagcatacctataaatttcttttttaaaaaaagtctctgtt cactctatttctattattttctcttttaaatttgaattttattgt ggcaagtccacttaacatgagatttaccctcttaacagattttatgtg taaaatacaatattgttcaccatgggtaaatgttgcacagcagatctct ggaacttattcattttgcactactgaaattttatacctgttgattagta tctccccatttccctctctcccctgtcctgttacccatggttctgttct ttgcttctttgagtttgagtattttgatacctcatgtaatcttcattct attttctaactttgacaatgttctgacaaatttgctttccggattggag cactgtatagtgaaaattgaaaatcttggttattttctacagattccca ctattttaccttgagcagacacttatcttgaagggtctcagatttgtca cttgtagaatggggaatataaacctgataatggtccctttcagttctaa agttatatcagttgaaaatacatgtgtcacttatggtaacgggtagaga actggctcactgaacagcatatggatattataaagtggtttttttaat cctttctgcagacagttactttatactttattcaaatggattattgtga agtacatgttagcggactttgtaccttttaaaatgtatgtatttggtg taatgtagaaatatagaaatttattaagtatgatttatttcaatgttaa gcatgagaaaatatgctccgaaaggttagatagcttgcctaaatgacaa gcttgtatttcaagcagaactttctgaatcaaaagactccaagacgaat |
| | | gcccagctttcaaaaactgtctaaccaaaataaatcctaagattcacct tcatactaaaattatttaaaaatagtttattttaaattaatattcactt aaaatgtatttatcatgcaatactttaaagtgtctgggaaatgaaaata tccaaagatcaaagaacaccatgttttcaaacttcaaaaatgttatcag tgacctaaacaattttaaaattttcatagagcctatgaaaaatgtact tgcaaatggctactttctgactaggaatagaatggggagagtatttagt ccaacaatgatagactggattaagaaaatgtggcacatatacaccatgg aacactatgcagccataaaaaatgatgagttcatgtcctttgtagggac atggatgaaattggaaaacatcattctcagtaaactatcgcaagaacaa aaaaccaaacaccgcatattctcactcataggtgggaattgaacaatga gatcacatggacacaggaaggggaatatcacactctggggactgttgtg gggtgggggaggggggagggatagcactgggagatatacctaatgcta gatgacgagttagtgggtgcagtgcaccagcatggcacatgtatacata tgtaactaacctgcacaatgtgcacatgtaccctaaaacttaaagtata ataaaaaaaataaaaaaaagtttgaggtgtttaaagtatgcaaaaaaa aaaaagaaataaatcactgacacactttgtccactttgcaatgtgaaaa tgtttactcaccaacatgttt |
| 54 | exon 19 | UGUUAUUAAUUGUGAUUGGAGCUAUAGCAGUUGUCGCAGUUUUACAACC CUACAUCUUUGUUGCAACAGUGCCAGUGAUAGUGGCUUUUAUUAUGUUG AGAGCAUAUUUCCUCCAAACCUCACAGCAACUCAAACAACUGGAAUCUG |
| 55 | intron 19 | acagtgaatgtgcgatactcatcttgtaaaaagctataagagctattt gagattctttattgttaatctacttaaaaaaattctgcttttaaactt ttacatcatataacaataatttttttctacatgcatgtgtatataaaag gaaactatattacaaagtacacatggatttttttcttaattaatgacc atgtgacttcattttggttttaaaataggtatatagaatcttaccacag ttggtgtacaggacattcatttataataaacttatatcagtcaaattaa acaaggatagtgctgctattactaaaggtttctctgggttcccaaatga tacttgaccaaatttgtccctttggcttgttgtcttcagacaccctttc ttcatgtgttggagctgccatttcgtgtgcccccaaactctacttgagc tgttagggaatcacattttgcagtgacagccttagtgtgggtgcatttt caggcaatactttttcagtatatttctgctttgtagattattagctaaa tcaagtcacataaacttccttaatttagatacttgaaaaattgtctta aaagaaaatttttttagtaagaattaatttagaattagccagaaaactc |
| | | ccagtggtagccaagaaagaggaataaatattggtggtaatttttttaag ttcccatctctggtagccaagtaaaaaaagagggtaactcattaataaa ataacaaatcatatctattcaaagaatggcaccagtgtgaaaaaagct ttttaaccaatgacatttgtgatatgattattctaatttagtctttttc aggtacaagatattatgaaattacattttgtgtttatgttatttgcaat gttttctat |
| 56 | exon 20 | CAGGAGUCCAAUUUCACUCAUCUUGUUACAAGCUUAAAGGACUAUGG ACACUUCGUGCCUUCGGACGGCAGCCUUACUUUGAAACUCUGUUCCACA AAGCUCUGAAUUUACAUACUGCCAACUGGUUCUUGUACCUGUCAACACU GCGCUGGUUCCAAAUGAGAAUAGAAAUGAUUUUGUCAUCUUCUUCAUU GCUGUUACCUUCAUUUCCAUUUUAACAA |
| 57 | intron 20 | atgaactcattaactttagctaagcatttaagtaaaaattttcaatga ataaaatgctgcattctataggttatcaattttgatatctttagagtt tagtaattaacaaatttgttggtttattattgaacaagtgatttctttg aatttccattgttttattgttaaacaaataatttccttgaaatcggata tatatatatatatgtatatatatatatatatatatatatatatacatat atatatatagtattatccctgttttcacagttttaaaaaccgatgcaca cagattgtcagatagcaattctgtgattgaaggggaaatatgtcacctc ttcatactcatattggtgaagggtcctagcttcaaaattaatagattcc taaagaggggaaatgaaacatccgcatttacacacacacacacacacac acacacagagttcctcttgtcggtaagttttgtttttttaaatctcta ctagataaaatttgttatctaattgtgagttttacacaaagaaaaactg tcacagaaaagaaagacagtgtcacattttcaaaagaaaaagaagaaa agaaagtgccatgtttttcaaatacaaatgttctggattgattttagga tctttagtgaaaaacaaagtatttcataataagtaaaataaaaatctat gtaggtaaatttgtttctctaatttaagaatttgaatttctgagtattt atgataagtgttgaaataacttcttatatgtgacagtgaatactggcag agcaaatgccaaatcaatgccaaatctgtaggatcatttgattgtagga acagaattctactcaaaccgaaagcaggcatttgctggagttacagaaa ggcctcatggaacaccgagaaggtggtgccattcgactcttaaagaagc tgcaacaggcacaagagagtcagctgcagctcttcttcttgagtctata tctgtcctgggtccattcctttttgtggttgcttcattcctttctctct ctgaagactggtttttctggtctaccagggctatgccacattgacttta |
| | | tgtagtgtctccattctggcctcctgaatttacaggagagttcctctgt acaaactcaaagtcctggagagaacagaaaacagcttccttttggctca ggggtccaactgcagtctactctgctgctatgaggatagtgggttcacc acctttgttgttctctcagctagggcagtgggaaatgactctatgaaag gaatatacatgggcaggcaaatgtactaatcctcatcagtactgtaatt ttaagcaactttaaaaattcttttaagttatttgaaaataagatcaaa gaaggctgaattacataaatgaagatttgttaacaattaattcaaacca atataacacatgctataacatggttgagtgtgattgagtcttgatttat taggggcaataatcaaaacatttaacaatcattatagtacagaacttac caatcaaatcagatgctcagccggagtggatgttggccacccagctatt attatccctggctcaattggtcttcagctgtgttaacttgcaaacatta attaactatctaagcccctcattttcctcaagtgtaaatagacacaata atattacctattccataggtgtggggtgaatagtaaatgtaataatttg tccaaaacacttagtatagtgcctggtccatggtaaatactaaataaat gttatctgacttattattaaattttatcttctcagcttaaccttcaga acagtaatatattggggtctagataaatcttgcctatatgaaaataatt taatactacatgcagatatatgctgtgtatattatgccttctgttagag gaattgcagaaacaaaaatttcaattaataataagatgaattatttctc ccaattgtagaatcttttgacaattttatcatgcattacagatgtaaga actcttgattgggacttgatagtctaactttataataatttaagaacat tcctcttagagaatttctatggccataatactgaacacatgaattttaa ttagctgtcctctttagccctaaaaaaaaattactgtaatttaacact taagtgttgttcttcccaggtacagtaatcttttttttttttttttt tttttgcatagagggtaatcttttctctttccaatggcagaactgtt agttttctgactgtccggtgaaattctaagtccacttacttcccaatag catgcaattagcaaaggtcctccttgcaaaggcacagaacacacctaaa catcttgcagatgctgtttggacactcttcccctgcttttggtctcttt gtaaagcagctcatctggatacaggatctcttttccccattgcccattc taatatatgttaccgttattacttatagaataatagtagaagagacaaa tatggtacctacccattaccaacaacacctccaataccagtaacatttt ttaaaaagggcaacactttcctaatattcaatcgctctttgatttaaaa tcctggttgaatacttactatatgcagagcattattctattagtagatg ctgtgatgaactgagatttaaaaattgttaaaattagcataaaattgaa |
| | | atgtaaatttaatgtgatatgtgccctaggagaagtgtgaataaagtcg ttcacagaagagagaaataacatgaggttcatttacgtct |
| 58 | exon 21 | AGAAGGAGAAGGAAGAGUUGGUAUUAUCCUGACUUUAGCCAUGAAUAUC AUGAGUACAUUGCAGUGGGCUGUAAACUCCAGCAUAGAUGUGGAUAGC |
| 59 | intron 21 | tcttatcatctttttaacttttatgaaaaaattcagacaagtaacaaa gtatgagtaatagcatgaggaagaactatataccgtatattgagcttaa gaaataaaacattacagataaattgagggtcactgtgtatctgtcatta aatccttatctcttctttccttctcatagatagccactatgaagatcta atactgcagtgagcattctttcacctgtttccttattcaggattttcta ggagaaatacctaggggttgtattgctgggtcataggattcacccatgc ttaactgagtggtgccaaattgtcctcaagtctgttgtactgatatata tccccatcaagagagtacaagaattctcatagctatgtatcttcaacaa cacttggtgtctggtagatgtgaagtgattactaaaaatatagggaagc tgcatacataattattggcttttgctgttctcttacattaatttcttat tcatgttgattactcatttgtcacctagtttttcttccttaattaaat tgtaggaatttatgaattatggattgatcatcagctctatacatttcaa acataatccctcagtcagtggcttggcttatagagtcttttgatgaaaa gaagctttttaagtttaataaagttcaatttattgtcttttcctttatgt tttgtgcttttggtatcttgattaagaactccttccttatattgggttc tcaaatttagcagcataacattttcatactattatttaaatttttttca cattatttagtgatagcacctttcttattcctaaagtgtttatcattgc cttctgtctttctgcttgataaatattgccacacatttgtatactttat tagtgtgtacaaagaccacatttagttgtgttatttctcttgttttgg ttttctagaatgcagagccattaatattatagtaatgcttatgtgctaa taccatatcaggggcacaaa.....aaataagagcagtaaaattgtgtc taatcagctactaatatctgggaaggattgagccacaggatcaaagatg gtatcttttaaaaatagaagttgagtgaattcggtcttcaaattctttc tttttattcatttatatttatttactcattagtatattcattcctttat tcatgtattgttcaaatatatattgggtacttattatatgccaagttgt ttttaaaatcacattccaaattcccgtaagtcataattattcagagatg tatgtttttttttaaaaaaaattgaacacctttaaaaattatcaagtcct tttatttctgtatgcattaaagataaactttactaaatgttacatgaat agatttataaagcagataaatatttaatttcaaatataacccttatatg |
| | | caattatattttccttagcactaaaaatgaatatttaagtaatttatat taaaagtgtaattatttaactgcagatgtatgccaatgacttaaattgt ttaaagattatagcaaagttgtttaaaattgtctaatcatgaagagttc acttaaccacctggttgacacataaaattatagttagttactaaggtag ttcgagagaaagagaagaatcttcagtagtggttttgaggtgtggtaca ttttattataatataccggttatacagcattgtgcagtgctgctcatag tagaaataaatttctctttgatgtcatctattcccttgtgtggcttac ataactgagaattaggtgatcacaaaaataaacaggcctatacagagcc catttatataagtcctggttatttctcttcagttaaacttttaattata tccaattatttcctgttagttcattgaaaagcccgacaaataaccaagt gacaaatagcaagtgttgcatttacaagttatttttaggaagcatca aactaattgtgaaattgtctgccattcttaaaaacaaaaatgttg |
| 60 | exon 22 | UGCGAUCUGUGAGCCGAGUCUUUAAGUUCAUUGACAUGCCAACAGAAGG UAAACCUACCAAGUCAACCAAACCAUACAAGAAUGGCCAACUCUCGAAA GUUAUGAUUAUUGAGAAUUCACACGUGAAGAAAGAUGACAUCUGGCCCU CAGGGGGCCAAAUGACUGUCAAAGAUCUCACAGCAAAUACACAGAAGG UGGAAAUGCCAUAUUAGAGAACAUUUCCUUCUCAAUAAGUCCUGGCCAG |
| 61 | intron 22 | atttgaacactgcttgctttgttagactgtgttcagtaagtgaatccca gtagcctgaagcaatgtgttagcagaatctatttgtaacattattattg tacagtagaatcaatattaaacacacatgttttattatatggagtcatt atttttaatatgaaatttaatttgcagagtcctgaacctatataatggg tttattttaaatgtgattgtacttgcagaatatctaattaattgctagg ttaataactaaagaagccattaaataaatcaaaattgtaacatgtttta gatttcccatcttgaaaatgtcttccaaaaatatcttattgctgactcc atctattgtcttaaattttatctaagttccattctgccaaacaagtgat actttttttctagctttttcagtttgtttgtttttgtttttctttgaag ttttaattcagacatagattatttttcccagttatttactatatttat taagcatgagtaattgacattattttgaaatccttcttatggatcccag cactgggctgaacacatagaaggaacttaatatatactgatttctggaa ttgattcttggagacaggatggtcattatccatatacttcaggctcca taaacatatttcttaattgccttcaaatccctattctggactgctctat aaatctagacaagagtattatatattttgattgatatttttttagataaa ataaaagggagctgaaaactgaattgcaaactgaattttaaaactttat |
| | | ctctctgtggttaattgcaaacacagatacaaaaatatagagagagata cagttagtaaagatgttaggtcaccgttactaacactgacatagaaaca gttttgctcatgagtttcagaatatatgagtttgattttgcccatggat tttagaatatttgataaacatttaatgcattgtacaaattctgtgaaaa catatatataggatgtgcga.....aacaaaactgtccttcactacaga ttgaaaagcattatactaaaagaccatttgctcagttatagtatataaa ggccaaatgacttaaaaacaaattatgtaaggagaaggaaacaaccatt tattcagtgccactaactgtcagccagtttttcagtggtcagttaatg actgcagtagtgttctaccttgctcaaagcaccctcctcaagttctggc atctaagctgacatcagaacacagagttggggctctctgtgggtcacct ctagcacttgatctcctcatgcagtgcatggtgctctcacgtctatgct atgttcttatggtctttaggtaacaagaataatttctttcttttcctt actatacattttgctttctgaaattcccttctcgccaatccaggtgaat gtcagaatgtgatttgacaactgtccaaagtactcattcactgaggagt ggtaaggccttcgcccaacctgccttctctgggaatatactgctgcctg aacatatcattgtttattgccaggcttgaacttcaccaaattaatttat tagggtcaacatctaaatattagaactatttcagattaattttaagtc gtatccactttgggtactagatcaaattgcaggtctctgcttctggctt gagcctatgtttagagatgatgtgcatgaagacactctttgcttttcct ttatgcaaaatgggcattttcaatctttttgtcattagtaaaggtcagt gataaaggaagtctgcatcaggggtccaattccttatggccagtttctc tattctgttccaaggttgtttgtctccatatatcaacattggtcaggat tgaaagtgtgcaacaaggtttgaatgaataagtgaaaatcttccactgg tgacaggataaaatattccaatggtttttattgaagtacaatactgaat tatgtttatggcatggtacctatatgtcacagaagtgatcccatc |
| 62 | exon 23 | UGGGCCUCUUGGGAAGAACUGGAUCAGGGAAGAGUACUUUGUUAUCAGC UUUUUGAGACUACUGAACACUGAAGGAGAAAUCCAGAUCGAUGGUGUG UCUUGGGAUUCAAUAACUUUGCAACAGUGGAGGAAAGCCUUUGGAGUGA UACCA |
| 63 | intron 23 | caaaaggacttagccagaaaaaaggcaactaaattatattttttactgc tatttgatacttgtactcaagaaattcatattactctgcaaaatatatt tgttatgcattgctgtctttttttctccagtgcagttttctcataggcag aaaagatgtctctaaaagtttggaattctcaaattctggttattgaaat |
| | | gttcatagctttgatagtgtttttcagaagaccaatttacagtgggag ccttgggcttttgtttttaacagctctttttgttcctgcttcagtgg cctgacctccaagttagcaatcgccaggttgagaaatgctttgcgagac ataacagatgctcctgaaataacaaacacttggaatcatgaggtagtgg aattgaaaatagaaagtgtagtgattgtttttgttatttggatgggat gaacaatgtcagattagtctgtaactatttttttaatgtcactctga tttggtcacaaaggatctctagtctcattgccttagtatcattctacga attagaatgtgttactgtgtaagagcacttcttgtatatgagagaaata gcaacagttccagtttaaagtgatataaatggaaaccaagaatgtctt tactgggaccaaatctggacagcatttactgtatttttgctggtatttt ctctagtctttccgggtatattcacatttaatgatcacttttctccctt tgtgctaatggacactgaatccattccactaccatagttcttgctaata ctactctacttttacacaaattaaaatgccaggagcacctccaggta gactgactataaatctagactgaaaaaaagcttgtatttcttaacaga ttaccttgtggaacatttgctcctttcaactaatgaggcactaaatatt gtaactgctcaactggtgcttttaatttatttgtctagactttgtcatg ttgccagaagctttatcctg.....ttgacttgacttgtgtggttcctt gtggaccagatggccactaaatattctcatttcaaggcaattggtaaaa actacacttcaagaaatttcattcttaattccccttagtggatgttatt aaccaaaggcaaaagaaaaaaggtaaaaaaatattctaaatgttaa tatcaaaaatattattttcaattcaccccaggcacagagaactaagtat tattattgctattgcaccggcattccccaatgagacagtgatttcttt taagacattttaaataatataggcagaattaagtagacggtgatctgg taagtagatgtttcagggtaacagctgtgcaatgctccatgcagggaat tagattgtcattttattccttaccaggaacatacattcagttaaacaat tatttgacttctgctcttccactgatttctaagttgaggctctctcttg tgcctgtctgatcagataagtagagttgtgccttggtttatagatgaga taaatgtgtatttgaataagcataagttaaagaattttaaatccctt aggaagctaggcttatcagagaaatccaaggaaatacattaacaaacta ggaatttgttctaacaggttaattataactcataaacttattgggtttt tttaccttttaattttatattacatttgcttataataaggaatattgct aggaataaaatttttaatattctacaattaacaattatctcaatttct ttattctaaagacattgggattagaaaaatgttcacaagggactccaaa |
| | | tattgctgtagtatttgtttcttaaaagaatgatacaaagcagacatga taaaatattaaaatttgagagaacttgatggtaagtacatgggtgtttc ttattttaaaataatttttctacttgaaatattttacaatacaataagg gaaaaataaaaagttatttaagttattcatactttcttcttcttt |
| 64 | exon 24 | AAGUAUUUAUUUUUUCUGGAACAUUUAGAAAAAACUUGGAUCCCUAUGA ACAGUGGAGUGAUCAAGAAAUAUGGAAAGUUGCAGAU |
| 65 | intron 24 | gctgctaactgaaatgattttgaaaggggtaactcataccaacacaaat ggctgatatagctgacatcattctacacactttgtgtgcatgtatgtgt gtgcacaactttaaaatggagtaccctaacatacctggagcaacaggta cttttgactggacctacccctaactgaaatgattttgaaagaggtaact cataccaacacaaatggttgatatggctaagatcattctacacactttg tgtgcatgtatttctgtgcacaacttcaaaatggagtaccctaaaatac ctggcgcgacaagtacttttgactgagcctacttctctcctcactggta tggctccaaccatcaggccctatcttggtccatttaggctgctaaaata aaataccaaagactgagctgcttataagcaatctttggaggctgagaag tcaaagatcaaggtgccagcaggtttgctgtctcgtgagagcatacttc ctggttcattgatggtgctttcttgctgtgtcctcacataatggaaagg gcaagacctctctggtgtctcttttacaatggcactaatcccatcatga gggctttgttctcatgacctaatcacctcccacatgtcctacattctaa tactatcaccttgggggttaggattttaacatatgaatttgaggaggtg gcgggggggacacaaatatttagaccatagcatttcactcctgacctcc aaagttcatgtcttcttcacatgcaaaatacattcattccatcccaata gcccccaaagtcttaacttgttccagcatcaacttacaaggctaaagtc caaggtttcatctaaatatcagctaaatcagcacaaacagctaaatcag gtagagtgggacttaaggtgtgattcctctttaggcagattgctctcca actatgaaattgtgaaatcaaacctattatgtactttcaaataaaatg gtgaaacaggcacaggctag.....ataagattctttctgagccattat ctcattctatattacagtcaggtggagcccatcttacctcctcatacta aattctagacttctcaagggcaggagacaatcatctgtatatctctttg gccttcatacactcaggagtacttgccaaaaataaacatttaatgcaca tttatttgaataattgataagatccaatacttcaataactttgtcatat ttttatagaatgggtttctatatctcatttgcattttcaaactttactt ttactgtctagctttaaaaaaaaagcctttgactctaatacagccctca |
| | | tattctaccccaatatctaagaggctttatatctcctagtgttgtacca ctattttaactccagtatttttacttcatagttttacctatttgttac agttagttttatgaattcaagagatgaatagcaattttccatatgtaa tttaaaaaccccacagttgactattttatgctatcttttgtcctcagt catgacagagtagaagatggaggtagcaccaaggatgatgtcatacct ccatcctttatgctacattctatcttctgtctacataagatgtcatact agagggcatatctgcaatgtatacatattatcttttccagcatgcattc agttgtgttggaataatttatgtacacctttataaacgctgagcctcac aagagccatgtgccacgtattgttttcttactactttttggatacctg gcacgtaatagacactcattgaaagtttcctaatgaatgaagtacaaag ataaaacaagttatagactgattcttttgagctgtcaaggttgtaaata gacttttgctcaatcaattcaaatggtggcaggtagtgggggtagaggg attggtatgaaaaacataagctttcagaactcctgtgtttatttttaga atgtcaactgcttgagtgtttttaactctgtggtatctgaactat |
| 66 | exon 25 | UUGGGCUCAGAUCUGUGAUAGAACAGUUUCCUGGGAAGCUUGACUUUGU CCUUGUGGAUGGGGGCUGUGUCCUAAGCCAUGGCCACAAGCAGUUGAUG UGCUUGGCUAGAUCUGUUCUCAGUAAGGCGAAGAUCUUGCUGCUUGAUG AACCCAGUGCUCAUUUGGAUCC |
| 67 | intron 25 | tttcagatgttctgttacttaatagcacagtgggaacagaatcattatg cctgcttcatggtgacacatatttctattaggctgtcatgtctgcgtgt gggggtctcccccaagatatgaaataattgcccagtggaaatgagcata aatgcatatttccttgctaagagtcttgtgttttcttccgaagatagtt tttagtttcatacaaactcttccccttgtcaacacatgatgaagcttt taaatacatgggcctaatctgatccttatgatttgcctttgtatcccat ttataccataagcatgtttatagccccaaataaagaagtactggtgatt ctacataatgaaaaatgtactcatttattaaagtttctttgaaatattt gtcctgtttatttatggatacttagagtctaccccatggttgaaaagct gattgtggctaacgctatatcaacattatgtgaaaagaacttaaagaaa taagtaatttaaagagataatagaacaatagacatattatcaaggtaaa tacagatcattactgttctgtgatattatgtgtggtatt |
| 68 | exon 26 | ACAUACCAAAUAAUUAGAAGAACUCUAAAACAAGCAUUUGCUGAUUGCA CAGUAAUUCUCUGUGAACACAGGAUAGAAGCAAUGCUGGAAUGCCAACA AUUU |
| 69 | intron 26 | tctttataactttacttaagatctcattgcccttgtaattcttgataac aatctcacatgtgatagttcctgcaaattgcaacaatgtacaagttctt ttcaaaaatatgtatcatacagccatccagctttactcaaaatagctgc acaagtttttcactttgatctgagccatgtggtgaggttgaaatatagt aaatctaaaatggcagcatattactaagttatgtttataaataggatat atatacttttttgagccctttatttggggaccaagtcatacaaaatactc tactgtttaagattttaaaaaggtccctgtgattctttcaataactaa atgtcccatggatgtggtctgggacaggcctagttgtcttacagtctga tttatggtattaatgacaaagttgagaggcacatttcatttttctagcc atgatttgggttcaggtagtacctttctcaaccaccttctcactgttct taaaaaaactgtcacatggccaggcacagtggcttacatctgtaatccc aatactttgggaggctgaggtgggggattacttgaggccaggaattca agaccagcccaggcaacatagtgaggccccatctgtctttattaaaaca aaacaaaactgtcacagcttctttcaagtgatgtttacaaattccctat ggtttagtcacaaggaagttctgaggatgatgtatcacgtcatttctgt tcaggcttttgagcctcctggaggtaaatggtttccttactgaaggctt gttattaccatgattatcactaagcttgaagtaacaaattagggggca gactcacaacctcttgccctgccatggacaagttcaagaatctaagtaa agtcctctattgtctgatcttggatttgctcaacctgaacaagccaagg aggtgtattaaactcaggcacatcctgaccaatttggaattcttaagct tcagatcactgtggaagaggctcaactctttatggtgctgtagacttac gctcattttctaggtaatttataagggacctaatattttgttttcaaag caacttcagttctactaaacctccctgaagaatcttccagctgctgagt agaaaatcacaactaatttcacagatggtagaacctccttagagcaaaa ggacacagcagttaaatgtgacatacctgattgttcaaaatgcaaggct ctggacattgcattctttgacttttatttcctttgagcctgtgccagt ttctgtccctgctctggtctgacctgccttctgtcccagatctcactaa |
| 70 | exon 27 | UCAUAGAAGAGAACAAAGUGCGGCAGUACGAUUCCAUCCAGAAACUGCU GAACGAGAGGAGCCUCUUCCGGCAAGCCAUCAGCCCCUCCGACAGGGUG AAGCUCUUUCCCCACCGGAACUCAAGCAAGUGCAAGUCUAAGCCCAGA UUGCUGCUCUGAAAGAGGAGACAGAAGAAGAGGUGCAAGAUACAAGGCU U |

**Table 16: ADAMTS13 Target Sequences**

| **SEQ ID NO** | **REGION** | **TARGET SEQUENCE** |
|---|---|---|
| 71 | exon 25 | GCUCUGUUUCCUGUGGGGAUGGCAUCCAGCGCCGGCGUGACACCUGCCU CGGACCCCAGGCCCAGGCGCCUGUGCCAGCUGAUUUCUGCCAGCACUUG CCCAAGCCGGUGACUGUGCGUGGCUGCUGGGCUGGGCCCUGUGUGGGAC AGGGUACGCCCAGCCUGGUGCCCCACGAAGAAGCCGCUGCUCCAGGACG GACCACAGCCACCCCUGCUGGUGCCUCCCUGGAGUGGUCCCAGGCCCGG GGCCUGCUCUUCUCCCCGGCUCCCCAGCCUCGGCGGCUCCUGCCCGGGC CCCAGGAAAACUCAGUGCAGU |
| 72 | intron 25 | guccuguccuccuuccugucaggcagcugcugcaggaggguggcaaa ggcaucuuccucugggaaggacuggcacaagcacuugguccugguug ugugccugggaggccgggaucagggcuggcccucuuucuccuggcaaa gcaaaaccucccuuuuacuacuaucaaggggaaguaacuugaagguagg aacccagcuugugagccccuagccucugggcugcucugcaugugcccc cucuugcuggaucaucugguagcagcccugugcccugagggugaugcuc ugaccuaugcagcccccucccuguccugagaaggcuuccagcugggcc uuggaggacaggguccaccccuaccuccuggucuccuuccucagcuugg aagccccggagccugcccugcugggaaucggggaagcacugcuuaccug ucuc |
| 73 | exon 26 | UGCCUGUGGCAGGCAGCACCUUGAGCCAACAGGAACCAUUGACAUGCGA GGCCCAGGGCAGGCAGACUGUGCAGUGGCCAUUGGGCGGCCCCUCGGGG AGGUGGUGACCCUCCGCGUCCUUGAGAGUUCUCUCAACUGCAGUG |
| 74 | exon 27 | GGACAUGUUGCUGCUUUGGGCCGGCUCACCUGGAGGAAGAUGUGCAGG AAGCUGUUGGACAUGACUUUCAGCUCCAAGACCAACACGCUGGUGGUGA GGCAGCGCUGCGGGCGGCCAGGAGGUGGGGUGCUGCUGCGGUAUGGAG CCAGCUUGCUCCUGAAACCUUCUACA |
| 75 | intron 27 | gccaggccuucuccaccucccuugggugcuccaguccuggcagggaggc uggguggugcugcuggggauggggccagucccaguggggcaguggaa gauacggagggaacugacugagauggaaggaacuggguuggccagugu cagucugcacgugccagggagggucacaggaugaaugcuaucccuc cuuuuugggaccgugcagcaagauggacggaugugggacaugguccaca |
| | | uccucagucagucccucaggccucugccccacacccaccugccccgccc ccaccccuccagccuuucaagggcuuuuaggguuuuguggaagccacug ucccucagcccuguuucagugcacugguguaagcagacaugcuuguaca ugcaugugcacccacaagcacaccucaggcagaggaugccaccucaggg acuccagccuugcccguggccccucgauauccucugauagcccucucg guuguccugggggcuugcccucucccaacagcccgagcuggccgaagu uggcuucccuagcugguuccagagguuccucggcucccccaggugucug gggcuuaguggcaacaggggcuuagccucugcagagaccuagugcgccg ccuccuugccccagaccugcccgggcagagagccguguaugugucccag ugcacaggcgcugcugggcccugccaaaggccacaagcccacugucac cguucacauugcuucucgcuucccggcccagccccgcccacacaggcau cugccuugaaagaggugcaggagguacaggcagguggggcuccaguga gcucugaggaacagcaguggccgccaugguggagccuaucuuuguugc caguuucaguguuaaacacucuugcacgugugacaucauugaguccuaa agaccacucugcucagugcaugccauuguuuccuucaguuacagaggag ggaaccagagcccagaacauuuagccuuugccuaaagucacuggccag gaagugguagagguggguucagcaggauuugccuggaaccccaauau ugaccacagugccaugcugcccugcacggcucccuggcugugaguuguc cuggccucuggcaccaccggucugucugguuccuaugucccu |
| 76 | exon 28 | AUGUGACAUGCAGCUCUUUGGGCCCUGGGGUGAAAUCGUGAGCCCCUCG CUGAGUCCAGCCACGAGUAAUGCAGGGGGCUGCCGGCUCUUCAUUAAUG UGGCUCCGCACGCACGGAUUGCCAUCCAUGCCCUGGCCACCAACAUGGG CGCUGGGACCGAGGGAGCCAAUGCCAGCUACAUC |

**Table 17: TSC1 Target Sequences**

| **SEQ ID NO** | **REGION** | **TARGET SEQUENCE** |
|---|---|---|
| 77 | exon 5 | ACCUCUUGGACAGGAUUAACGAAUAUGUGGGCAAAGCCGCCACUCGUUU AUCCAUCCUCUCGUUACUGGGUCAUGUCAUAAGACUGCAGCCAUCUUGG AAGCAUAAGCUCUCUCAAGCACCUCUUUGCCUUCUUUACUAAAAUGUC UC |
| 78 | intron 5 | auguuuguaaggauuugaaugaaaugguuuuaugaguauaguuucugaa |
| | | auuuuaggcaacuuaaagcaaggaagcuagauuuaacuuuuagaguuu aaaaccuucuaggcauuuggcuuuucucaaauagaauguuguccagagu ugguacuuaguaaguucucaaauacaucacuaugacuauugaauaccuu guccaugcaaguauggaaaaauuucgaucagaugguucaauguuacau uauuccaaaccucuugauuucgucaucguuuagccuucccucauuuaaa aacauccuggauuaucuuuugggaaucccuguuucuaaauuaucuuuua gcuaauagaaaaauggcuuaaaguuucuguuaaccauuuaggaguaugg ucugguugcagcuauaauuaagacuuuguugauguaaauucuacuaagu ugcauucuauuuuuugcacuaaauuuagugcauuuuucuauauagggag ucaaaaucuaaauagaacuuuaugguuuuaguuuuaacaguggcgugca gccauacucaggguuauuuguuuaaucuguuuuaguuccuggacuuguu uucuaucuauaaaauaagaaaaugugguuaauauuaacugccuguaccu cacagagacaugaaaauauccaauaguauuuguuccaggauggcaguac cauuggauucaucugcuacagcaccaugcaaauugauuuuugugucugc caagaaggguaacucuuuuauuaucccuagagguggucccaaggaguc acauuggcaggguauuauaaaaacaugcauuuaauucagaaaaaauagg aacaguuuuaacaacuuaauguuuuuuaaacaaauggauugaugagaau auaaucuaauuaauggauuggugagaauauaaucuaaauggauugauga gaauauaaucuaaauggauuggugagaauauaaucuaaauggauugaug agaauauaaucuaauuuugaggcacaucauuuaguucagauugcaaaac acuuaucuuuuccaaaagaguacguuuuguuaaucauggauaagucuuc aguuagacuguuaggaaaaugaaaucagggcuaguucuuucugcugaga aucauuauauagucucauauauucucaauucuccuaccaauauauuauu cuuacuggauaucuuccguaaugaaggcuugaugcuugauguaaaaau caaaauauauuuaaaacuuuauucccagacucauagauuccuauucuaa uaggaauaauggaugucuuaaccuacauaguagucuuuugauuaauauc uuguuucauaaaucugaauuucaucuaccuggcaaacauucaugauuua auuaugggucaggugagcugcuguagcuagcuagucagagcugauugag uauccauuggguguuaagugucuucaguuagccugaaguuauuuauuug acuuaauauuuaaacuguaggcgugcugaaagguuuccauauauauaua uuuuaauuuacuggucucuaaauacugcuuugaagugagccuuuaaguu gacuuguuagugcuauaugaauuucuccuucaauuauacuucuguugua guucuuuaaaaaauaguaaguuacuugucaaugugcaguuuuuuuuuuu |
| | | uuuaauuaacaaaaguaaguaucuuaggauuugguugaaugaaugaaa cagagcagugcuccuguguuuuguugaaaagcagcuccuuuuguuuuca uccaacugcuaucaauagggcauccuaaggcugcaggacuuggugucc ccaagucaaguugaacucgucucccggaugccuuugcauaggugugu u guaaaugguccucacugacucauuacaguagaguugggcucaguguuc uguugagucuguuugaauguuaucccuucaguaauccuuaggauaggg aaaugaguacgugagucaacuugugauuugugauucucucaguguuuag agccucuucauguacuguacaaugccgauccuggugccagugccugaca gacguuuccuguuuga |
| 79 | exon 6 | UGGACACUGACGUCGUUGUCCUCACAACAGGCGUCUUGGUGUUGAUAAC CAUGCUACCAAUGAUUCCACAGUCUGGGAAACAGCAUCUUCUUGAUUUC UUUGACAUUUUGGCCGUCUGUCAUCAUGGUGCCUGAAGAAAC |
| 80 | exon 10 | GUGUGCUACUUCUACCCCUUACUCCACGUCUCGGCUGAUGUUGUUAAAU AUGCCAGGGCAGCUACCUCAGACUCUGAGUUCCCCAUCGACACGGCUGA UAACUGAACCACCA |
| 81 | intron 10 | gugucaacuagugugccugcucucucccucugcuuucuggugaagcugac ccuuugggucagauuuaguaugugguuggaaaauuucacacugcucau uucaggagucacuuuuaaggauccaugauauuagcaaagaaaguuacug uugccucuuagauucaucuugaagucuugauuuacaaaaugcaacuugu uucuugauacgcuuuuaauaagaugccuuuuucuagaugaaaagcuaa auuuaagcugaacacuggccauggauauaaaccucguggaugacuuagc auuccuuugccacugcugauguacu |
| 82 | exon 11 | CUACUCUUUGGAGCCCAUCUAUGGUUUGUGGUAUGACCACUCCUCCAAC UUCUCCUGGAAAUGUCCCACCUGAUCUGUCACACCCUUACAGUAAAGUC UUUGGUACAA |
| 83 | intron 11 | uaugucuuagguuggauuugauuaguugguuuuggccugccuuuaaugg caggaggagcucucuuuuagaucuaagggaccacuugcuguuguaaacu uguuuuugacacuuauugcaaaucccugggcuuucagaauguguaaag ugaaccuaaaaacaaaaagagagagacugaucuagauccccagaaagu uaacucuagcagcuuuauuuauaguaauaguuauaggcugaaaaaaau cggcaguuuucuaauaguugggcucaguguucauauauguucu |
| 84 | exon 12 | AGGUGGAAAAGGAACUCCUCUGGGAACCCCAGCAACCUCUCCUCCUCCA GCCCCACUCUGUCAUUCGGAUGACUACGUGCACAUUUCACUCCCCCAGG |
| | | CCACAGUCACACCCCCCAGG |

**Table 18: IMPDH1 Target Sequences**

| **SEQ ID NO** | **REGION** | **TARGET SEQUENCE** |
|---|---|---|
| 85 | exon 14 | GAUGAUGGGCUCCCUGCUGGCCGCCACUACGGAGGCCCCUGGCGAGUAC UUCUUCUCAGACGGGGUGCGGCUCAAGAAGUACCGGGCAUGGGCUCAC UGGAUGCCAUGGAGAAGAGCAGCAGCAGCCAGAAACGAUACUU |
| 86 | intron 14 | cugacccugggccccaccugggcagaucagcccacaacccuucagggcc cgcucaugccaccgacuuccccagauggcagccaguccccauauggugg uucuggaaacugaggcacagggcuuaaguagcagacccaggaucugucc cugggccaucugacucagcccagugaggguggccuggggaccuuccu gggcgguaucccguuuugcccuuaagaggugggugggguccucugag cuucaagcugcugggcucagucuu |
| 87 | exon 15 | GAGGGGAUAAAGUGAAGAUCGCGCAGGGUGUCUCGGGCUCCAUCCAGG ACAAAGGAUCCAUUCAGAAGUUCGUGCCCUACCUCAUAGCAGGCAUCCA ACACGGCUGCCAGGAUAUCGGGGCCCGCAGCCUGUCUGUCCU |

**Table 19: PKD1 Target Sequences**

| **SEQ ID NO** | **REGION** | **TARGET SEQUENCE** |
|---|---|---|
| 88 | exon 32 | AGGCCUUUGUUGGACAGAUGAAGAGUGACUUGUUUCUGGAUGAUUCUAA |
| 89 | intron 32 | uucccuagagaaaccucgagcccuggugcaggucacugugucuggggug ccggggugugcgggcugcguguccuugcuggugucuguggcuccaug uggucacaccacccgggagcagguuugcucggaagcccagguguccgu gcgugacuggacgggggugggcugugugugugacacauccccugguacc uugcugac |
| 90 | exon 33 | CUGGUGUGCUGGCCCUCCGGCGAGGGAACGCUCAGUUGGCCGGACCUGC UCAGUGACCCGUCCAUUGUGGGUAGCAAUCUGCGGCAGCUGGCACGGGG CCAGGCGGGCCAUGGGCUGGGCCCAGAGGAGGACGGCUUCUCCCUGGCC AGCCCCUACUCGCCUGCCAAAUCCUUCUCAGCAU |
| 91 | intron 33 | cugggugagaggagggggcucugaagcucacccuugcagcugggccca cccuaugc |
| 92 | exon 34 | UGAAGACCUGAUCCAGCAGGUCCUUGCCGAGGGGGUCAGCAGCCCAGCC CCUACCCAAGACACCCACAUGGAAACGGACCUGCUCAGCAG |
| 93 | exon 37 | UCUUGCUGGAAGCCCUGUACUUCUCACUGGUGGCCAAGCGGCUGCACCC GGAUGAAGAUGACACCCUGGUAGAGAGCCCGGCUGUGACGCCUGUGAGC GCACGUGUGCCCCGCGUACGGCCACCCCACGGCUUUGCACUCUUCCUGG CCAAGGAAGAAGCCCGCAAGGUCAAGAGGCUACAUGGCAUGCUG |
| 94 | intron 37 | ccugggugcggccugugccccugccaccuccgucucuugucucccaccu cccacccaugcacgcaggacacuccugucccccuuccucaccucagaa ggcccuuagggguucaaugcucugcagccuuugcccggucucccuccua ccccacgcccccacuugcugccccagucccugccagggcccagcucca augcccacuccugccuggcccugaaggccccuaagcaccacugcagugg ccugugugucugcccccaggugggguuccgggcagggugugugcugcca uuacccuggccagguagagucuugggcgccccugccagcucaccuuc cugcagccacaccugccgcagccauggcuccagccguugccaaagcccu gcugucacugugggcuggggccaggcugaccacagggc |
| 95 | exon 38 | GCCUCCUGGUGUACAUGCUUUUUCUGCUGGUGACCCUGCUGGCCAGCUA UGGGGAUGCCUCAUGCCAUGGGCACGCCUACCGUCUGCAAAGCGCCAUC AAGCAGGAGCUGCACAGCCGGGCCUUCCUGGCCAUCAC |
| 96 | intron 38 | ggcauccggugcacuggucugucuucugggcuuuaguuuugccuuuagu ccagccagacccuaggggacauguggacauguguagauaccuuuguggc ugcuagaacuggagguaggugcugcuggcaucaguaggcagagggagg gacacagguccgugucuugcagugcacaggacgggcccaugacagacaa cugucugccccagaacauccccaggauaaggcugagaagccaggucua gccguggccagcagggcaguggagccauguucccuggucucuggugg ccgcucacucgaggcgggcaugggcaguagggcuggagcguguga |
| 97 | exon 39 | UCUGAGGAGCUCUGGCCAUGGAUGGCCCACGUGCUGCUGCCCUACGUCC ACGGGAACCAGUCCAGCCCAGAGCUGGGGCCCCCACGGCUGCGGCAGGU GCGGCUGCAGG |

**Table 20: IKBKAP Target Sequences**

| **SEQ ID NO** | **REGION** | **TARGET SEQUENCE** |
|---|---|---|
| 98 | exon 7 | AUGAGUCUGCUUUGCCCUGGGAUGACCAUAGACCACAAGUUACCUGGCG GGGGGAUGGACAGUUUUUGCUGUGAGUGUUGUUUGCCCAGAAA |
| 99 | intron 7 | gaaauauauugcaguuaaacaacaauaaaaaauuuuuaucuuauuaaaa uuaaggaaaauuucuuucuuuugcuuugaguagguauuaauuauaca uaugaggcaaggaugugcugcuuuaaaugugaaaugagguuagaguuaa gaauuagaagaguccuuugaggccauuugguccauccuccuaccuggug gacacaaauuuguaacaaaauuaaucuaauuggcuauguaaaccaugg caguuuuuauuuguaaggaagguguuugaauaguucugaauugacaacu uuuaucauaauguuuuaagugugaugugguuugac |
| 100 | exon 8 | GGCUCGGAAGGUCAGAGUGUGGAACCGAGAGUUUGCUUUGCAGUCAACC AGUGAGCCUGUGGCAGGACUGGACCAGCCCUGGCUUG |
| 101 | intron 8 | ugggagaagaaaccuuagagaaauucuuggaaccagaguagaggugug guacacauggauacagaugauacagauguuuguguaacacaaaggauu uuuacguuucuucauuugguuauaaggcuguaucuaucuuuguuucuuc uuuuuuuuuucuuauucccugaagucugaauucaacucgaauaguag auuuuacgcuucuucacagauuucauuguuccaaggccgcauauauuuu gcauuccuaacucuuaaaggcugugguuuuaaggcagguauauauga agccauuguacagagcagaaaaugguguuuagaagggaaggcccaguuu gcaaggcucuguggggcaaauggugcuuuuguggaaauuagggaaagag ccuccuuccuuggcacaaaauuccuacagcagaggaucugcuugccaag gagcaugcaggcuggauucagacccugcucuuuccuuccauucuccucc uuggcccaguacccuugugcagguuacaauuugccugucauauguggcu gccugauuuuagauagaagauguaucuccucuguuucggugauaucugu uguauguagaccucuuguuucccaccaguaucugaaugguauuauauga uagagcagaagagaaauguauuugaauuaaaacccuagagacaaauaug aauaagaugaggcaauuaagauguuucaacauuggugaagucuuaaa aaagaccuacuggagcauagaauauuugcugaaguuguauaauggaagg agaaauagauuuugauuuuuaggacauuauaccuggaaugguuuagaua acuuauuauuuuuaaagucauccaaaugcaauguaaauauguaagguuu ugugggcaaauggagccucuguguaaaacaggaaaaggcacucuuuccu cugggcaaguacagucccacaguggaugaaccgcucgccgagagacaa gggacacauggauuuaaaacuuccuuggauaaagauauucauuaauuc guucauucauucauucauguuugcuggaaaaaaacucuucuggauuuu aucuauucuuuaguuaggugagcuuucgauauuguaacacuc |
| 102 | exon 9 | CCCUCAGGCAGUUUGAUUGCAUCUACACAAGAUAAACCCAACCAGCAGG AUAUUGUGUUUUUGAGAAAAAUGGACUCCUUCAUGGACACUUUACACU UCCCUUCCUUAAAGAUGAGGUU |

Certain embodiments of the invention are set out in the following numbered paragraphs:
1. A method of treating a subject to increase the expression of a target protein or functional RNA by cells of the subject, wherein the cells have a retained-intron-containing pre-mRNA (RIC pre-mRNA), the RIC pre-mRNA comprising a retained intron, an exon flanking the 5' splice site, an exon flanking the 3' splice site, and wherein the RIC pre-mRNA encodes the target protein or functional RNA, the method comprising contacting the cells of the subject with an antisense oligomer (ASO) complementary to a targeted portion of the RIC pre-mRNA encoding the target protein or functional RNA, whereby the retained intron is constitutively spliced from the RIC pre-mRNA encoding the target protein or functional RNA, thereby increasing the level of mRNA encoding the target protein or functional RNA, and increasing the expression of the target protein or functional RNA in the cells of the subject.
2. A method of increasing expression of a target protein or functional RNA by cells having a retained-intron-containing pre-mRNA (RIC pre-mRNA), the RIC pre-mRNA comprising a retained intron, an exon flanking the 5' splice site of the retained intron, an exon flanking the 3' splice site of the retained intron, and wherein the RIC pre-mRNA encodes the target protein or functional RNA, the method comprising contacting the cells with an antisense oligomer (ASO) complementary to a targeted portion of the RIC pre-mRNA encoding the target protein or functional RNA, whereby the retained intron is constitutively spliced from the RIC pre-mRNA encoding the target protein or functional RNA, thereby increasing the level of mRNA encoding the target protein or functional RNA, and increasing the expression of target protein or functional RNA in the cells.
3. The method of paragraph 1, wherein the subject has a condition caused by a deficient amount or activity of the target protein or a deficient amount or activity of the functional RNA.
4. The method of paragraph 1, wherein the target protein or the functional RNA is a compensating protein or a compensating functional RNA that functionally augments or replaces a target protein or functional RNA that is deficient in amount or activity in the subject.
5. The method of paragraph 2, wherein the cells are in or from a subject having a condition caused by a deficient amount or activity of the target protein or a deficient amount or activity of the functional RNA.
6. The method of any of paragraphs 3 to 5, wherein the deficient amount of the target protein is caused by haploinsufficiency of the target protein, wherein the subject has a first allele encoding a functional target protein, and a second allele from which the target protein is not produced, or a second allele encoding a nonfunctional target protein, and wherein the antisense oligomer binds to a targeted portion of a RIC pre-mRNA transcribed from the first allele.
7. The method of any of paragraphs 3 to 5, wherein the subject has a condition caused by an autosomal recessive disorder resulting from a deficiency in the amount or function of the target protein, wherein the subject has
   a. a first mutant allele from which
      i. the target protein is produced at a reduced level compared to production from a wild-type allele,
      ii. the target protein is produced in a form having reduced function compared to an equivalent wild-type protein, or
      iii. the target protein is not produced, and
   b.a second mutant allele from which
      i. the target protein is produced at a reduced level compared to production from a wild-type allele,
      ii. the target protein is produced in a form having reduced function compared to an equivalent wild-type protein, or
      iii. the target protein is not produced, and
      wherein the RIC pre-mRNA is transcribed from the first allele and/or the second allele.
8. The method of paragraph 7, wherein the target protein is produced in a form having reduced function compared to the equivalent wild-type protein.
9. The method of paragraph 7, wherein the target protein is produced in a form that is fully-functional compared to the equivalent wild-type protein.
10. The method of any of paragraphs 1 to 9, wherein the targeted portion of the RIC pre-mRNA is in the retained intron within the region +6 relative to the 5' splice site of the retained intron to -16 relative to the 3' splice site of the retained intron.
11. The method of any of paragraphs 1 to 9, wherein the targeted portion of the RIC pre-mRNA is in the retained intron within:
   (a) the region +6 to +100 relative to the 5' splice site of the retained intron; or
   (b) the region -16 to -100 relative to the 3' splice site of the retained intron.
12. The method of any of paragraphs 1 to 9, wherein the targeted portion of the RIC pre-mRNA is within:
   (a) the region +2e to -4e in the exon flanking the 5' splice site of the retained intron; or
   (b) the region +2e to -4e in the exon flanking the 3' splice site of the retained intron.
13. The method of any of paragraphs 1 to 12, wherein the antisense oligomer does not increase the amount of the target protein or the functional RNA by modulating alternative splicing of pre-mRNA transcribed from a gene encoding the functional RNA or target protein.
14. The method of any of paragraphs 1 to 13, wherein the antisense oligomer does not increase the amount of the target protein or the functional RNA by modulating aberrant splicing resulting from mutation of the gene encoding the target protein or the functional RNA.
15. The method of any of paragraphs 1 to 14, wherein the RIC pre-mRNA was produced by partial splicing of a full-length pre-mRNA or partial splicing of a wild-type pre-mRNA.
16. The method of any of paragraphs 1 to 15, wherein the mRNA encoding the target protein or functional RNA is a full-length mature mRNA, or a wild-type mature mRNA.
17. The method of any of paragraphs 1 to 16, wherein the target protein produced is full-length protein, or wild-type protein.
18. The method of any of paragraphs 1 to 17, wherein the total amount of the mRNA encoding the target protein or functional RNA produced in the cell contacted with the antisense oligomer is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to the total amount of the mRNA encoding the target protein or functional RNA produced in a control cell.
19. The method of any of paragraphs 1 to 18, wherein the total amount of target protein produced by the cell contacted with the antisense oligomer is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to the total amount of target protein produced by a control cell.
20. The method of any of paragraphs 1 to 19, wherein the antisense oligomer comprises a backbone modification comprising a phosphorothioate linkage or a phosphorodiamidate linkage.
21. The method of any of paragraphs 1 to 20, wherein the antisense oligomer comprises a phosphorodiamidate morpholino, a locked nucleic acid, a peptide nucleic acid, a 2'-O-methyl, a 2'-Fluoro, or a 2'-O-methoxyethyl moiety.
22. The method of any of paragraphs 1 to 21, wherein the antisense oligomer comprises at least one modified sugar moiety.
23. The method of paragraph 22, wherein each sugar moiety is a modified sugar moiety.
24. The method of any of paragraphs 1 to 23, wherein the antisense oligomer consists of from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, or 12 to 15 nucleobases.
25. The method of any one of paragraphs 1 to 24, wherein the antisense oligomer is at least 80%, at least 85%, at least 90%, at least 95%, at least 98%, at least 99%, or 100%, complementary to the targeted portion of the RIC pre-mRNA encoding the protein.
26. The method of any one of paragraphs 1 to 25, wherein the cell comprises a population of RIC pre-mRNAs transcribed from the gene encoding the target protein or functional RNA, wherein the population of RIC pre-mRNAs comprises two or more retained introns, and wherein the antisense oligomer binds to the most abundant retained intron in the population of RIC pre-mRNAs.
27. The method of paragraph 26, whereby the binding of the antisense oligomer to the most abundant retained intron induces splicing out of the two or more retained introns from the population of RIC pre-mRNAs to produce mRNA encoding the target protein or functional RNA.
28. The method of any one of paragraphs 1 to 25, wherein the cell comprises a population of RIC pre-mRNAs transcribed from the gene encoding the target protein or functional RNA, wherein the population of RIC pre-mRNAs comprises two or more retained introns, and wherein the antisense oligomer binds to the second most abundant retained intron in the population of RIC pre-mRNAs.
29. The method of paragraph 28, whereby the binding of the antisense oligomer to the second most abundant retained intron induces splicing out of the two or more retained introns from the population of RIC pre-mRNAs to produce mRNA encoding the target protein or functional RNA.
30. The method of any of paragraphs 3 to 29, wherein the condition is a disease or disorder.
31. The method of paragraph 30, wherein the disease or disorder is selected from: thrombotic thrombocytopenic purpura, tuberous sclerosis complex, polycystic kidney disease, familial dysautonomia, retinitis pigmentosa type 10, retinitis pigmentosa type 11, cystic fibrosis, retinoblastoma, familial adenomatous polyposis, protein S deficiency, beta thalassemia, and sickle cell disease.
32. The method of paragraph 31, wherein the target protein and the RIC pre-mRNA are encoded by a gene selected from: *ADAMTS13, TSC1, PKD1, IKBKAP, IMPDH1, PRPF31, CFTR, RB1, APC, PROS1, NEDD4L, HBG1, HBG2,* and *HBB.*
33. The method of any of paragraphs 1 to 32, wherein the method further comprises assessing protein expression.
34. The method of any of paragraphs 1 to 33, wherein the antisense oligomer binds to a portion of an RIC pre-mRNA selected from SEQ ID NOS: 1-102.
35. The method of any of paragraphs 1 to 34, wherein the subject is a human.
36. The method of any of paragraphs 1 to 34, wherein the subject is a non-human animal.
37. The method of any of paragraphs 1 to 36, wherein the cells are *ex vivo.*
38. The method of any of paragraphs 1 to 36, wherein the antisense oligomer is administered by intravitreal injection, intrathecal injection, intraperitoneal injection, subcutaneous injection, or intravenous injection of the subject.
39. The method of any of paragraphs 1 to 38, wherein the 9 nucleotides at -3e to -1e of the exon flanking the 5' splice site and +1 to +6 of the retained intron, are identical to the corresponding wild-type sequence.
40. The method of any of paragraphs 1 to 39, wherein the 16 nucleotides at -15 to -1 of the retained intron and +1e of the exon flanking the 3' splice site are identical to the corresponding wild-type sequence.
41. An antisense oligomer as used in a method of any of paragraphs 1 to 39.
42. A pharmaceutical composition comprising the antisense oligomer of paragraph 41 and an excipient.
43. A composition comprising an antisense oligomer for use in a method of increasing expression of a target protein or a functional RNA by cells to treat a condition in a subject associated with a deficient protein or deficient functional RNA, wherein the deficient protein or deficient functional RNA is deficient in amount or activity in the subject, wherein the antisense oligomer enhances constitutive splicing of a retained intron-containing pre-mRNA (RIC pre-mRNA) encoding the target protein or the functional RNA, wherein the target protein is:
   (a) the deficient protein; or
   (b) a compensating protein which functionally augments or replaces the deficient protein or in the subject;
   and wherein the functional RNA is:
   (a) the deficient RNA; or
   (b) a compensating functional RNA which functionally augments or replaces the deficient functional RNA in the subject;
   wherein the RIC pre-mRNA comprises a retained intron, an exon flanking the 5' splice site and an exon flanking the 3' splice site, and wherein the retained intron is spliced from the RIC pre-mRNA encoding the target protein or the functional RNA, thereby increasing production or activity of the target protein or the functional RNA in the subject.
44. A composition comprising an antisense oligomer for use in a method of treating a disease or disorder associated with a target protein or functional RNA in a subject, the method comprising the step of increasing expression of the target protein or functional RNA by cells of the subject, wherein the cells have a retained-intron-containing pre-mRNA (RIC pre-mRNA) comprising a retained intron, an exon flanking the 5' splice site of the retained intron, an exon flanking the 3' splice site of the retained intron, and wherein the RIC pre-mRNA encodes the target protein or functional RNA, the method comprising contacting the cells with the antisense oligomer, whereby the retained intron is constitutively spliced from the RIC pre-mRNA transcripts encoding the target protein or functional RNA, thereby increasing the level of mRNA encoding the target protein or functional RNA, and increasing the expression of the target protein or functional RNA, in the cells of the subject.
45. The composition of paragraph 44, for use in a method of treating a condition in the subject resulting from a deficiency in the amount or activity of the target protein or the functional RNA.
46. The composition of paragraph 43 or 45, wherein the condition is a disease or disorder.
47. The composition of paragraph 46, wherein the disease or disorder is selected from: thrombotic thrombocytopenic purpura, tuberous sclerosis complex, polycystic kidney disease, familial dysautonomia, retinitis pigmentosa type 10, retinitis pigmentosa type 11, cystic fibrosis, retinoblastoma, familial adenomatous polyposis, protein S deficiency, beta thalassemia, and sickle cell disease.
48. The composition of paragraph 47, wherein the target protein and RIC pre-mRNA are encoded by a gene selected from: *ADAMTS13, TSC1, PKD1, IKBKAP, IMPDH1, PRPF31, CFTR, RB1, APC, PROS1, NEDD4L, HBG1, HBG2,* and *HBB.*
49. The method of any of paragraphs 43 to 48, wherein the antisense oligomer targets a portion of the RIC pre-mRNA that is in the retained intron within the region +6 relative to the 5' splice site of the retained intron to -16 relative to the 3' splice site of the retained intron.
50. The composition of any of paragraphs 43 to 49, wherein the antisense oligomer targets a portion of the RIC pre-mRNA that is in the retained intron within:
   (a) the region +6 to +100 relative to the 5' splice site of the retained intron; or
   (b) the region -16 to -100 relative to the 3' splice site of the retained intron.
51. The composition of any of paragraphs 43 to 48, wherein the antisense oligomer targets a portion of the RIC pre-mRNA that is within the region about 100 nucleotides downstream of the 5' splice site of the at least one retained intron, to about 100 nucleotides upstream of the 3' splice site of the at least one retained intron.
52. The composition of any of paragraphs 43 to 48, wherein the targeted portion of the RIC pre-mRNA is within:
   (a) the region +2e to -4e in the exon flanking the 5' splice site of the retained intron; or
   (b) the region +2e to -4e in the exon flanking the 3' splice site of the retained intron.
53. The composition of any of paragraphs 43 to 52, wherein the antisense oligomer does not increase the amount of target protein or functional RNA by modulating alternative splicing of the pre-mRNA transcribed from a gene encoding the target protein or functional RNA.
54. The composition of any of paragraphs 43 to 53, wherein the antisense oligomer does not increase the amount of the functional RNA or functional protein by modulating aberrant splicing resulting from mutation of the gene encoding the target protein or functional RNA.
55. The composition of any of paragraphs 43 to 54, wherein the RIC pre-mRNA was produced by partial splicing from a full-length pre-mRNA or a wild-type pre-mRNA.
56. The composition of any of paragraphs 43 to 55, wherein the mRNA encoding the target protein or functional RNA is a full-length mature mRNA, or a wild-type mature mRNA.
57. The composition of any of paragraphs 43 to 56, wherein the target protein produced is full-length protein, or wild-type protein.
58. The composition of any of paragraphs 43 to 57, wherein the retained intron is a rate-limiting intron.
59. The composition of any of paragraphs 43 to 58 wherein said retained intron is the most abundant intron in said RIC pre-mRNA.
60. The composition of any of paragraphs 43 to 58, wherein the retained intron is the second most abundant intron in said RIC pre-mRNA.
61. The composition of any of paragraphs 43 to 60, wherein the antisense oligomer comprises a backbone modification comprising a phosphorothioate linkage or a phosphorodiamidate linkage.
62. The composition of any of paragraphs 43 to 61 wherein said antisense oligomer is an antisense oligonucleotide.
63. The composition of any of paragraphs 43 to 62, wherein the antisense oligomer comprises a phosphorodiamidate morpholino, a locked nucleic acid, a peptide nucleic acid, a 2'-O-methyl, a 2'-Fluoro, or a 2'-O-methoxyethyl moiety.
64. The composition of any of paragraphs 43 to 63, wherein the antisense oligomer comprises at least one modified sugar moiety.
65. The composition of paragraph 64, wherein each sugar moiety is a modified sugar moiety.
66. The composition of any of paragraphs 43 to 65, wherein the antisense oligomer consists of from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, or 12 to 15 nucleobases.
67. The composition of any of paragraphs 43 to 66, wherein the antisense oligomer is at least 80%, at least 85%, at least 90%, at least 95%, at least 98%, at least 99%, or is 100% complementary to the targeted portion of the RIC pre-mRNA encoding the protein.
68. The composition of any of paragraphs 43 to 67, wherein the antisense oligomer binds to a portion of an RIC pre-mRNA selected from SEQ ID NOS: 1-102.
69. A pharmaceutical composition comprising the antisense oligomer of any of the compositions of paragraphs 41 to 68, and an excipient.
70. A method for identifying an antisense oligomer that increases the amount of mRNA encoding a target protein or functional RNA by inducing constitutive splicing of a retained intron from a RIC pre-mRNA encoding the target protein or functional RNA, from among a set of antisense oligomers that each hybridize to a target region of the RIC pre-mRNA, wherein the RIC pre-mRNA comprises at least one retained intron, wherein the antisense oligomers in the set are tiled every 1 to 5 nucleotides, and wherein the antisense oligomers in the set hybridize to the RIC pre-mRNA within the sequence that is:
   about 100 nucleotides upstream of the 5' splice site of the at least one retained intron, to about 100 nucleotides downstream of the 5' splice site of the at least one retained intron; or
   about 100 nucleotides upstream of the 3' splice site of the at least one retained intron, to about 100 nucleotides downstream of the 3' splice site of the at least one retained intron;

   the method comprising:
      a. delivering a first antisense oligomer in the set to a cell comprising the RIC pre-mRNA;
      b. measuring the amount of the RIC pre-mRNA and measuring the amount of mRNA encoding the target protein or functional RNA in the cell to which the first antisense oligomer was delivered;
      c. measuring the amount of the RIC pre-mRNA and measuring the amount of mRNA encoding a target protein or functional RNA in a control cell; and
      d. comparing the amounts of RIC pre-mRNA and mRNA encoding a target protein or functional RNA measured in b and c;
   wherein the first antisense oligomer is identified as an antisense oligomer that increases the amount of mRNA encoding the target protein or functional RNA by inducing constitutive splicing of the at least one retained intron from the RIC pre-mRNA based on an observed decrease in the amount of the RIC pre-mRNA and an observed increase in the amount of mRNA encoding the target protein or functional RNA in the cell to which the first antisense oligomer was delivered compared to a control cell; and
   repeating steps a through d with additional antisense oligomers in the set of antisense oligomers as needed to identify an antisense oligomer that increases the amount of mRNA from a gene in a cell by inducing constitutive splicing of a retained intron from the RIC pre-mRNA.
71. A method for identifying an antisense oligomer (ASO) for treating a condition, wherein the condition results from insufficient production of a gene product, the method comprising:
   identifying the presence of at least one RIC pre-mRNA in the nucleus of a cell from a subject having the condition, wherein the RIC pre-mRNA comprises at least one retained intron and is transcribed from a gene encoding the gene product, and wherein the identified RIC pre-mRNA when fully spliced to mature mRNA encodes the gene product in a form that is fully-functional or partially-functional;
      a. preparing a set of ASOs that each hybridize to a target region of the at least one RIC pre-mRNA, wherein the antisense oligomers in the set are tiled every 1 to 5 nucleotides, and wherein the antisense oligomers in the set hybridize to the at least one RIC pre-mRNA within the sequence that is:
         i. about 100 nucleotides upstream of the 5' splice site of the at least one retained intron, to about 100 nucleotides downstream of the 5' splice site of the at least one retained intron; or
         ii. about 100 nucleotides upstream of the 3' splice site of the at least one retained intron, to about 100 nucleotides downstream of the 3' splice site of the at least one retained intron;
      b. delivering a first ASO in the set of ASOs to a cell comprising the at least one RIC pre-mRNA;
      c. measuring the amount of RIC pre-mRNA and measuring the amount of mRNA encoding the gene product in the cell to which the first antisense oligomer was delivered;
      d. measuring the amount of RIC pre-mRNA and measuring the amount of mRNA encoding the gene product in a control cell; and
      e. comparing the values obtained in steps c and d;
   wherein the first antisense oligomer is identified as an antisense oligomer that increases the amount of mRNA encoding the gene product by inducing constitutive splicing of the at least one retained intron from the RIC pre-mRNA based on an observed decrease in the amount of RIC pre-mRNA and an observed increase in the amount of mRNA encoding the gene product in the cell to which the first antisense oligomer was delivered compared to a control cell; and
   repeating steps a through e with additional antisense oligomers in the set of antisense oligomers as needed to identify an antisense oligomer that increases the amount of a mRNA encoding the gene product from a gene in a cell by inducing constitutive splicing of a retained intron from a RIC pre-mRNA; and
   further testing such antisense oligomers that increase the amount of a mRNA encoding the gene productin a cell by inducing constitutive splicing of a retained intron from a RIC pre-mRNA for the ability to increase the amount of the gene product produced by a cell.

## Claims

1. A composition comprising an antisense oligomer (ASO) for use in a method of treating a condition, disease or disorder in a human subject caused by a deficient amount or activity of a target protein or a target functional RNA resulting from haploinsufficiency, wherein the ASO binds to a retained-intron-containing pre-mRNA (RIC pre-mRNA) encoding the target protein or target functional RNA by targeting a portion of the RIC pre-mRNA that is in the retained intron within the region +6 relative to the 5' splice site of the retained intron to -16 relative to the 3' splice site of the retained intron, thereby increasing splicing efficiency and so increasing the expression of the target protein or target functional RNA.

2. The composition for use according to claim 1, wherein the RIC pre-mRNA is transcribed from a wild-type gene or allele, or a polymorphic gene or allele, that encodes a fully functional target protein or functional RNA, and wherein the gene or allele does not have a mutation that causes alternative splicing or aberrant splicing of the retained intron.

3. The composition for use according to claim 1 or claim 2, wherein the human subject has a first allele encoding a functional target protein, and a second allele from which the target protein is not produced, or a second allele encoding a nonfunctional target protein, and wherein the RIC pre-mRNA is transcribed from the first allele.

4. The composition for use according to any one of the preceding claims, wherein the ASO does not increase the amount of mRNA encoding the target protein or target functional RNA, the amount of the target protein, or the amount of the target functional RNA, by modulating alternative splicing or aberrant splicing of a pre-mRNA transcribed from a gene encoding the functional RNA or target protein.

5. The composition for use according to any one of the preceding claims, wherein the composition is a pharmaceutical composition comprising: (i) the ASO, and (ii) one or more pharmaceutically acceptable excipients.

6. The composition for use according to any one of the preceding claims, wherein the ASO comprises a backbone modification comprising a phosphorothioate linkage or a phosphorodiamidate linkage.

7. The composition for use according to claim 6, wherein the ASO comprises a phosphorodiamidate morpholino, a locked nucleic acid, a peptide nucleic acid, a 2' -O-methyl moiety, a 2'-Fluoro moiety, or a 2'-O-methoxyethyl moiety.

8. The composition for use according to any one of the preceding claims, wherein the ASO comprises a modified sugar moiety.

9. The composition for use according to any one of the preceding claims, wherein the ASO consists of from 8 to 50 nucleobases.

10. The composition for use according to any one of the preceding claims, wherein the composition is administered by intravitreal injection, intrathecal injection, intraperitoneal injection, subcutaneous injection, or intravenous injection.
